# EUROPEAN PATENT APPLICATION

(11) **EP 4 715 043 A1**
(43) Date of publication of application: **25.03.2026**
(21) Application number: 24811076.9
(22) Date of filing: 17.05.2024
(51) Int. Cl.: C12N 5/0797, A61L 27/36, A61L 27/38, C12N 5/0775, C12N 15/12, C12Q 1/04

(54) **METHOD FOR PRODUCING NEURAL CREST CELLS**

(30) Priority: 19.05.2023 JP 2023082964
(71) Applicant: Kyoto University, Kyoto-shi, Kyoto 606-8501 (JP)
(72) Inventor: IKEYA, Makoto, Kyoto-shi, Kyoto 606-8501 (JP); MOTOIKE, Souta, Kyoto-shi, Kyoto 606-8501 (JP)
(74) Representative: J A Kemp LLP
(86) International application number: PCT/JP2024/018417
(87) International publication number: WO 2024/242069

(57) **Abstract**

The present invention provides a method for producing a neural crest cell from a pluripotent stem cell, the method comprising: (1) a step of suspension-culturing the pluripotent stem cell in a culture medium containing an ALK inhibitor and a bone morphogenetic protein; and (2) a step of suspension-culturing the cell in a culture medium containing the ALK inhibitor and a GSK-3β inhibitor.

## Description

### TECHNICAL FIELD

The present invention relates to a method for producing neural crest cells. More specifically, the present invention relates to a method for producing neural crest cells including a step of suspension-culturing cells derived from pluripotent stem cells under specific conditions, uses of the neural crest cells obtained by the method, and the like.

### BACKGROUND ART

Neural crest cells (NCCs) develop between a neuroectoderm and a surface ectoderm during the formation of a neural tube from neural plates in early development, and have multipotency, which is a capacity to differentiate into a large variety of cells such as nerve cells, glial cells, mesenchymal interstitial cells, osteocytes, chondrocytes, corneal cells, and pigment cells, and self-proliferation capacity. Such multipotency and self-proliferation capacity show the usefulness of NCCs as a cell pharmaceutical for regenerative medicine, and there is a demand for a technology for efficiently inducing NCCs.

Non-Patent Literature 1 states that NCCs can be induced by conducting neural priming on human pluripotent stem cells seeded on Matrigel for 3 days and then conducting a neural crest (NC) induction step for 5 days. Also, Non-Patent Literature 2 states that NCCs can be induced by conducting adherent culture of human pluripotent stem cells in a culture medium containing BMP4 and SB431542 for 7 days, and ectodermal chondrogenic cells (ECCs) can be induced by culturing the thus induced NCCs in a culture medium containing CHIR99021, SB431542, SAG, EGF, FGF2, and DMH1 for 23 days.

The inventors of the present invention previously reported a method in which NCCs are obtained by conducting adherent culture of pluripotent stem cells in a culture medium containing SB431542 and CHIR99021 under xeno-free conditions for 10 days, and then NCCs with loss of a capacity to differentiate into nerve cells are induced by conducting adherent culture of the obtained NCCs in a culture medium containing SB431542, EGF, and bFGF (Non-Patent Literature 3). Meanwhile, the inventors of the present invention also succeeded in expansion culture of NCCs retaining pluripotency by culturing NCCs obtained through induction of differentiation of pluripotent stem cells in a culture medium containing a GSK-3β inhibitor at such a concentration that enables effects equal to those of CHIR99021 at a concentration of more than 1 µM to be exhibited (Patent Literature 1).

### Citation List

### Patent Literature

Patent Literature 1: WO 2019/107485

### Non-Patent Literature

Non-Patent Literature 1: Kreitzer F.R. et al., Am J Stem Cells, 2(2): 119-131 (2013)
Non-Patent Literature 2: Shen P. et al., NPJ Regen Med, 7(1): 69 (2022)
Non-Patent Literature 3: Kamiya D. et al., NPJ Regen Med, 7(1): 47 (2022)

### SUMMARY OF INVENTION

### Technical Problem

However, with the conventional methods above, it took at least 7 days to induce differentiation of pluripotent stem cells into neural crest cells, and it took 10 days to induce neural crest cells under xeno-free conditions. Accordingly, it is an object of the present invention to provide a method with which neural crest cells can be induced from pluripotent stem cells in a shorter time preferably under xeno-free conditions.

### Solution to Problem

The inventors of the present invention took note of suspension culture while advancing the research on a method for inducing a bone organoid from pluripotent stem cells. Accordingly, they searched for a method with which in a process of inducing neural crest cells (NCCs) from pluripotent stem cells that is conducted as the first step, NCCs can be induced from pluripotent stem cells through suspension culture instead of adherent culture, which has been conducted hitherto. As a result of extensive research, it was found that NCCs can be stably induced under xeno-free conditions with high efficiency by forming a circular cell aggregate through short-term suspension culture of pluripotent stem cells in a culture medium containing an ALK inhibitor and a bone morphogenetic protein and subsequently culturing it in a culture medium containing an ALK inhibitor and a GSK-3β inhibitor. In addition, surprisingly, they succeeded in inducing NCCs only 4 days (5 days even when the step of forming a pluripotent stem cell aggregate is included) after the start of the culture in the culture medium containing the ALK inhibitor and the bone morphogenetic protein. Moreover, they also succeeded in improving the efficiency of the NCC induction and shortening the culture period by conducting shaking culture in the middle of the culture step.

The inventors of the present invention verified the differentiation capacity of the NCCs above obtained through suspension culture. As a result, it was found that similarly to conventional NCCs, the differentiation into peripheral nerve cells, melanoblasts, and mesenchymal stem cells can be induced, and the differentiation into these types of cells is induced with very high efficiency. Furthermore, it was very surprising that the differentiation of the NCCs obtained through suspension culture into first pharyngeal arch ectomesenchyme (PA1-EM) cells can be induced because it has not been reported that the differentiation of NCCs into PA1-EM cells are successfully induced in vitro. It was also possible to induce a bone organoid from the PA1-EM cells, and it was also possible to efficiently restore a bone defect by transplanting the bone organoid into a living organism. As a result of further conducting a study based on these findings, the inventors of the present invention achieved the present invention.

That is to say, the present invention is as follows.
[1] A method for producing a neural crest cell from a pluripotent stem cell, comprising:
   (1) a step of suspension-culturing the pluripotent stem cell in a culture medium containing an ALK inhibitor and a bone morphogenetic protein; and
   (2) a step of suspension-culturing the cell in a culture medium containing the ALK inhibitor and a GSK-3β inhibitor.
[2-1] The method according to [1], wherein step (2) comprises a step of conducting shaking culture and/or stirred culture.
[2-2] The method according to [1] or [2-1], wherein step (1) comprises a step of conducting shaking culture and/or stirred culture.
[3-1] The method according to any one of [1] to [2-2], wherein a culture period in step (1) is 6 hours to 5 days.
[3-2] The method according to any one of [1] to [3-1], wherein a culture period in step (1) is 6 hours to 3 days.
[3-3] The method according to any one of [1] to [3-2], wherein a culture period in step (1) is 1 day to 3 days.
[4-1] The method according to any one of [1] to [3-3], wherein a culture period in step (2) is 1 day to 6 days.
[4-2] The method according to any one of [1] to [4-1], wherein a culture period in step (2) is 1 day to 4 days.
[4-3] The method according to any one of [1] to [4-2], wherein a culture period in step (2) is 1 day to 3 days.
[4-4] The method according to any one of [1] to [4-3], wherein a culture period in steps (1) and (2) is 2 days to 5 days.
[4-5] The method according to any one of [1] to [4-4], wherein a culture period in step (1) is 1 day and a culture period in step (2) is 3 days.
[5] The method according to any one of [1] to [4-5], comprising a step of suspension-culturing the pluripotent stem cell in a culture medium containing a ROCK inhibitor prior to step (1).
[6-1] The method according to any one of [1] to [5], wherein at least one of the bone morphogenetic proteins used in step (1) is selected from the group consisting of BMP4, BMP2, and BMP7.
[6-2] The method according to any one of [1] to [6-1], wherein at least one of the ALK inhibitors used in step (2) is SB431542 or A-83-01.
[6-3] The method according to any one of [1] to [6-2], wherein the pluripotent stem cell is derived from a patient with a disease (e.g., osteogenesis imperfecta).
[7] The method according to any one of [1] to [6-3], wherein at least one of steps (1) and (2) is a step of culturing the cell under xeno-free conditions and/or feeder-free conditions.
[8-1] The method according to any one of [1] to [7], wherein the pluripotent stem cell is an induced pluripotent stem cell.
[8-2] The method according to any one of [1] to [8-1], wherein the pluripotent stem cell is derived from a human.
[8-3] The method according to [8-2], wherein the pluripotent stem cell is derived from a patient with a disease (e.g., osteogenesis imperfecta).
[9] A neural crest cell obtainable by the method according to any one of [1] to [8-3].
[10] A neural crest cell having all the features (A) to (C) below:
   (A) derived from a pluripotent stem cell,
   (B) expressing one or more genes selected from CD271, SOX10, TFAP2A, and PAX3,
      and
   (C) having a capacity to differentiate into a first pharyngeal arch ectomesenchyme cell.
[11-1] The neural crest cell according to [10], having at least one of features (D) to (F) below:
   (D) having a capacity to differentiate into a melanoblast,
   (E) having a capacity to differentiate into a nerve cell, and
   (F) having a capacity to differentiate into a mesenchymal stem cell.
[11-2] The cell according to [10] or [11-1], wherein the pluripotent stem cell is an induced pluripotent stem cell.
[11-3] The cell according to any one of [10] to [11-2], wherein the pluripotent stem cell is derived from a human.
[11-4] The cell according to [11-3], wherein the pluripotent stem cell is derived from a patient with a disease (e.g., osteogenesis imperfecta).
[12] A method for producing a differentiated cell or an organoid, comprising a step of inducing differentiation of the neural crest cell according to any one of [9] to [11-4].
[13] The method according to [12], wherein the differentiated cell is selected from the group consisting of a mesenchymal cell, a nerve cell, and a melanoblast.
[14] The method according to [13], wherein the mesenchymal cell is selected from the group consisting of a mesenchymal stem cell, a first pharyngeal arch ectomesenchyme cell, an osteoprogenitor cell, an osteoblast, an osteocyte, a chondrocyte, a myoblast, an odontogenic mesenchymal cell, and a smooth muscle cell.
[15] The method according to [12], wherein the organoid is a bone organoid.
[16-1] The method according to [15], wherein the bone organoid is a jawbone organoid.
[16-2] The method according to [15] or [16-1], wherein the bone organoid represents a phenotype of a bone disease (e.g., osteogenesis imperfecta).
[17-1] The method according to [12], wherein the step of inducing differentiation is a step of culturing the neural crest cell in a culture medium containing endothelin and a fibroblast growth factor, and the differentiated cell obtainable through this culture is a first pharyngeal arch ectomesenchyme cell.
[17-2] The method according to [17-1], wherein at least one of the endothelins is endothelin-1.
[17-3] The method according to [17-1] or [17-2], wherein at least one of the fibroblast growth factors is FGF-8.
[18-1] The method according to any one of [17-1] to [17-3], wherein the culture medium further contains a bone morphogenetic protein.
[18-2] The method according to [18-1], wherein at least one of the bone morphogenetic proteins is BMP4.
[19] A differentiated cell or an organoid obtainable by the method according to any one of [12] to [18-2].
[20-1] A first pharyngeal arch ectomesenchyme cell having all of features (a) to (c) below:
   (a) derived from a neural crest cell that does not express HOX,
   (b) expressing DLX5, PRRX1, and TWIST1, and
   (c) responding to the epithelial signal and enabling induction of patterning.
[20-2] The cell according to [20-1], wherein the neural crest cell that does not express HOX is derived from a pluripotent stem cell.
[20-3] The cell according to [20-1] or [20-2], wherein the pluripotent stem cell is an induced pluripotent stem cell.
[20-4] The cell according to any one of [20-1] to [20-3], wherein the pluripotent stem cell is derived from a human.
[20-5] The cell according to [20-4], wherein the pluripotent stem cell is derived from a patient with a disease (e.g., osteogenesis imperfecta).
[21] An agent for transplantation therapy comprising the differentiated cell or the organoid according to any one of [19] to [20-5].
[22] A method for screening a disease therapeutic or preventive medicament, comprising a step of culturing the differentiated cell or the organoid according to any one of [19] to [20-5] in the presence or absence of a test substance.
[23] The agent for transplantation therapy according to [21], which is for treating or preventing a tissue damage or a disease.
[24] A method for treating or preventing a tissue damage or a disease, comprising administrating or transplanting an effective amount of the differentiated cell or the organoid according to any one of [19] to [20-5].
[25] The differentiated cell or the organoid according to any one of [19] to [20-5] for use in the treatment or prevention of a tissue damage or a disease.
[26] Use of the differentiated cell or the organoid according to any one of [19] to [20-5] in the manufacture of a therapeutic or preventive medicament for a tissue damage or a disease.

### Advantageous Effects of Invention

With the present invention, it is possible to produce neural crest cells (NCCs) from pluripotent stem cells in a short period of time with high efficiency. Since NCCs can be produced with high efficiency, cell sorting becomes unessential, which contributes to a reduction in the development expense and thus a reduction in the cost for clinical applications. Furthermore, it is possible to use the NCCs obtained through the present invention to not only produce differentiated cells such as nerve cells, melanoblasts, and mesenchymal stem cells with high efficiency but also produce first pharyngeal arch ectomesenchyme cells, which have not been previously reported, which contributes significantly to medical treatment and particularly to regenerative medicine in the field of oral surgery.

### Brief Description of Drawings

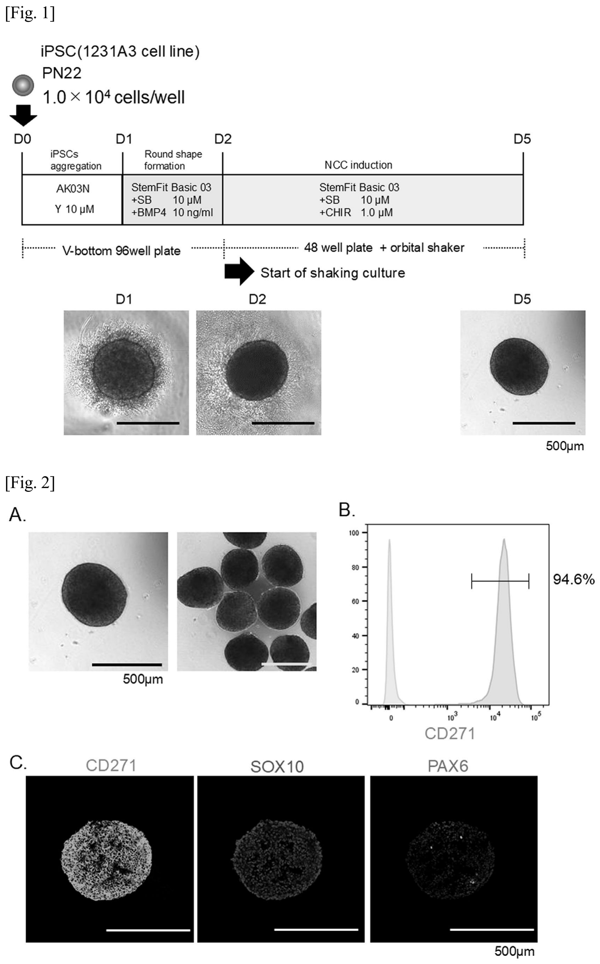

[FIG. 1] A diagram illustrating the outline of a method for inducing an NCC aggregate from iPS cells, and micrographs of the cell aggregate on Day 1 (D1), Day 2 (D2), and Day 5 (D5) after the start of culture.
[FIG. 2] (A) Micrographs of cell aggregates induced from 1231A3 iPS cells under three-dimensional conditions. It was possible to induce the cell aggregates having a uniform shape with a diameter of about 500 µm. (B) The result of analyzing the ratio of cells strongly positive for CD271 through flow cytometry after dispersing the cell aggregate in a single cell using Accutase. It was possible to induce cells strongly positive for CD271 with high efficiency. (C) Immunofluorescence-stained histological images of the cell aggregate. Most of the cells inside the cell aggregate were composed of NCCs positive for CD271 and SOX10.

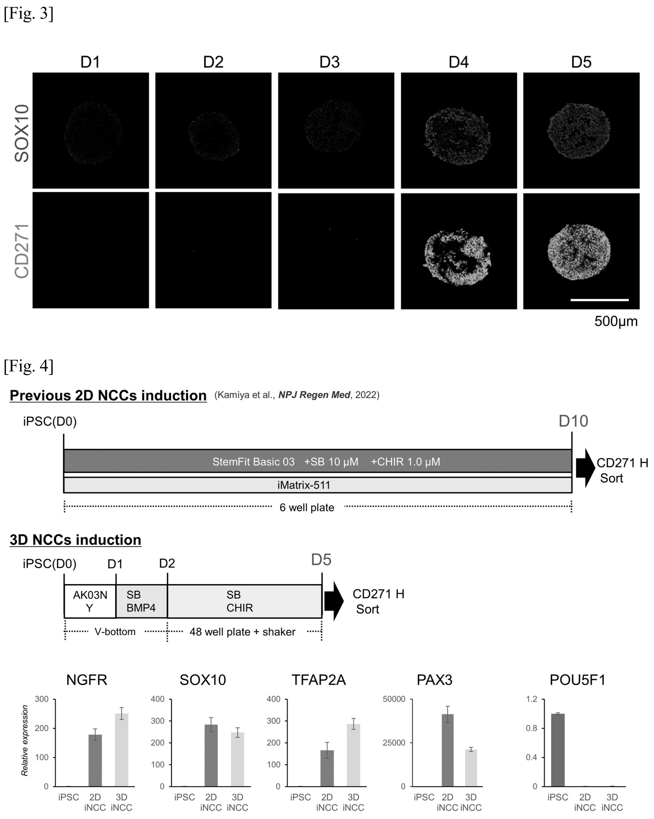

[FIG. 3] Changes in expression of CD271 and SOX10 over time from when iPS cells were seeded to when an NCC aggregate was obtained. NCCs positive for SOX10 and CD271 started to appear on Day 4 after the start of the induction (Day 2 after the start of the culture in a culture medium containing SB431542 (which may also be abbreviated as "SB" hereinafter) and CHIR99021 (which may also be abbreviated as "CHIR" hereinafter)).
[FIG. 4] The results of comparing NCC-related gene expression in NCCs induced under conventional 2D (adherent culture) conditions (Non-Patent Literature 3) and that in 3D-induced NCCs (the outline of the induction method is illustrated at the center of the diagram) using reverse-transcription quantitative PCR (RT-qPCR). It was confirmed that all the NCC-related genes (NGFR, SOX10, TFAP2A, PAX3) were expressed to the same extent, and the pluripotent cell marker POU5F1(Oct4) disappeared.

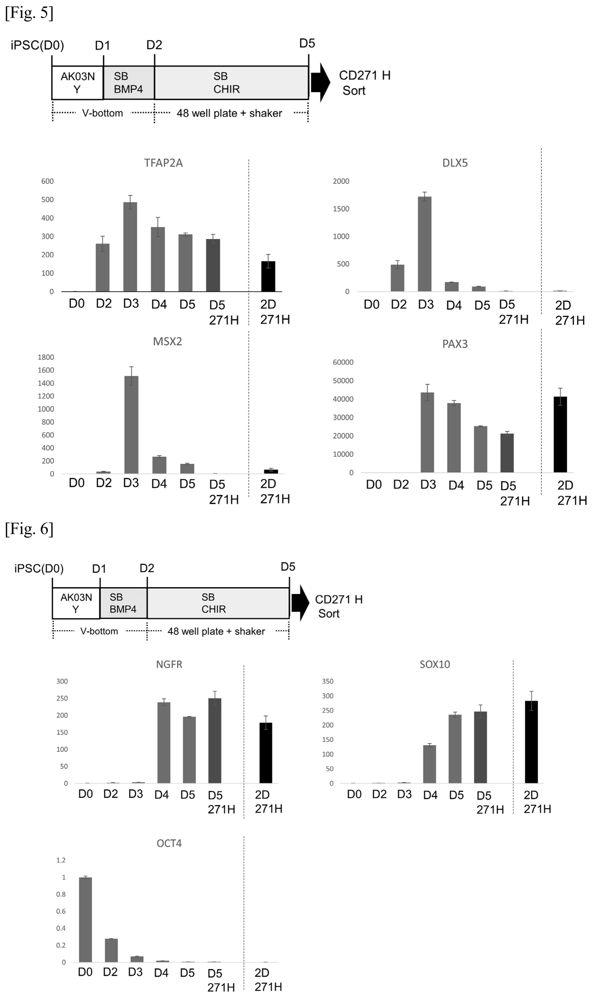

[FIG. 5] The results of comparing, using RT-qPCR, the expression levels of the NCC-related genes (TFAP2A and PAX3) and the neural plate border marker genes (DLX5 and MSX2) in cell aggregates on each day from Day 0 (D0) to Day 5 (D5) after the start of the cell culture and cell aggregates (D5 271H and 2D 271H) obtained by sorting cells strongly positive for CD271 through flow cytometry. It was shown that the expression of the neural plate border-related markers increased in D3, the cells on D5 expressed the NCC-related genes to substantially the same extent as those in the cells of the D5 271H, and the cells on D5 also expressed the NCC-related genes to substantially the same extent as those in the cells of the 2D 271H.
[FIG. 6] The results of comparing, using RT-qPCR, the expression levels of the NCC-related genes (NGFR, SOX10) and the pluripotent cell marker Oct4 in cell aggregates on each day from Day 0 (D0) to Day 5 (D5) after the start of the cell culture and cell aggregates (D5 271H and 2D 271H) obtained by sorting cells strongly positive for CD271 through flow cytometry. It was shown that the cells on D5 expressed the NCC-related genes to substantially the same extent as those in the cells of the D5 271H, and the cells on D5 also expressed the NCC-related genes to substantially the same extent as those in the cells of the 2D 271H.

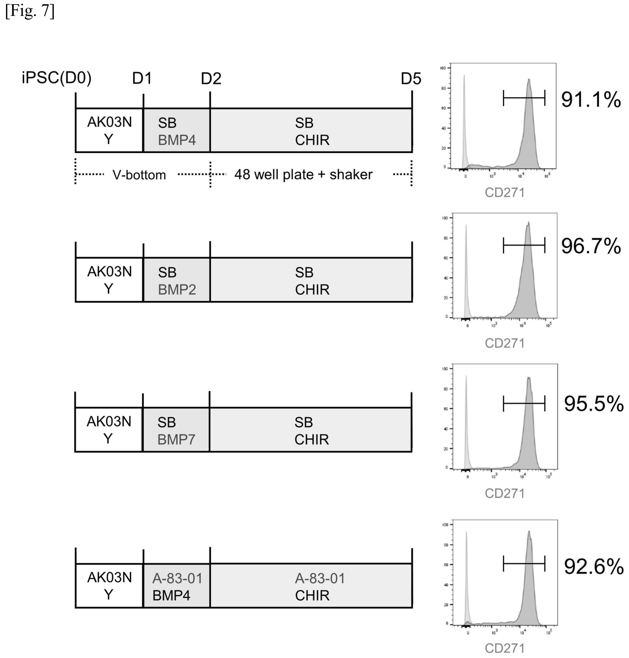

[FIG. 7] Changes in the ratio of cells strongly positive for CD271 was checked through flow cytometry when BMP4 was changed to BMP2 or BMP7 or when SB431542 was changed to another TGF-beta/Smad inhibitor A-83-01 (BMP4, BMP2, BMP7: 10 ng/ml; SB431542: 10 µM; A-83-01: 1.0 µM). It was possible to induce NCCs strongly positive for CD271 with high efficiency under all conditions.

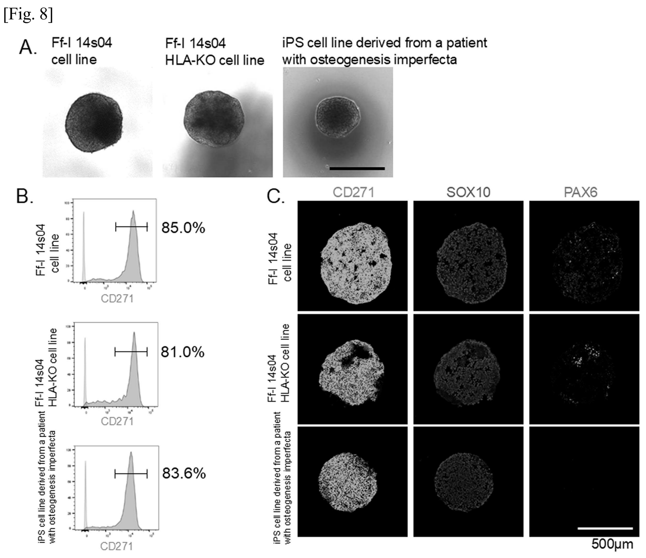

[FIG. 8] It was examined whether NCCs could also be induced from iPS cell lines (Ff-I 14s04 cell line, Ff-I 14s04 HLA-KO cell line, iPS cell line derived from a patient with osteogenesis imperfecta) other than the 1231A3 cell line in the same manner under three-dimensional conditions. (A) Micrographs of induced cell aggregates. Cell aggregates could be obtained from all the cell lines. (B) The result of analyzing the ratio of cells strongly positive for CD271 through flow cytometry. It was possible to induce cells strongly positive for CD271 with high efficiency from all the cell lines. (C) Immunofluorescence-stained histological images of the cell aggregates. Most of the cells inside the cell aggregates were composed of NCCs positive for CD271 and SOX10.

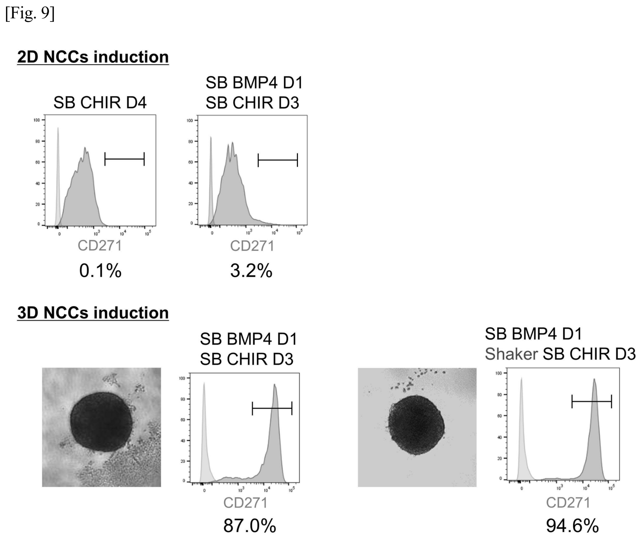

[FIG. 9] Culture was conducted using a conventional method (2D NCC induction method) under the same conditions as those of a 3D NCC induction method, except that adherent culture was conducted instead of suspension culture. It was shown from the results that with the 2D NCC induction method, substantially no CD271-high expression cells are induced (upper diagrams). Also, it was shown that the efficiency of the NCC induction is improved by conducting shaking culture in the 3D NCC induction method above (lower diagrams).

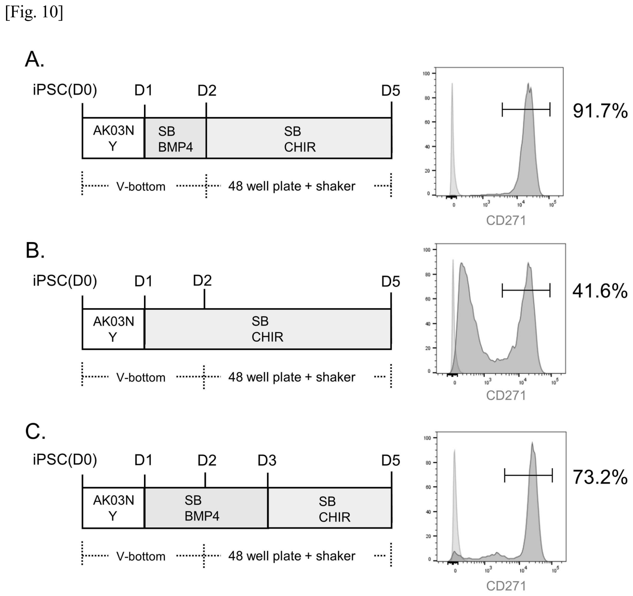

[FIG. 10] The ratio of cells strongly positive for CD271 was measured through flow cytometry while changing the period of an action step (referred to as an "SB-BMP action step" hereinafter) in order to search for the optimum action period of SB and BMP4 (the period of shaking culture was fixed). (A) NCCs were induced with high efficiency when the action time of SB and BMP4 was set to 1 day. (B) Without the action of SB and BMP4, it was observed that the ratio of cells strongly positive for CD271 obviously decreased. It was observed that the ratio of cells strongly positive for CD271 decreased when the action time of SB and BMP4 was extended.

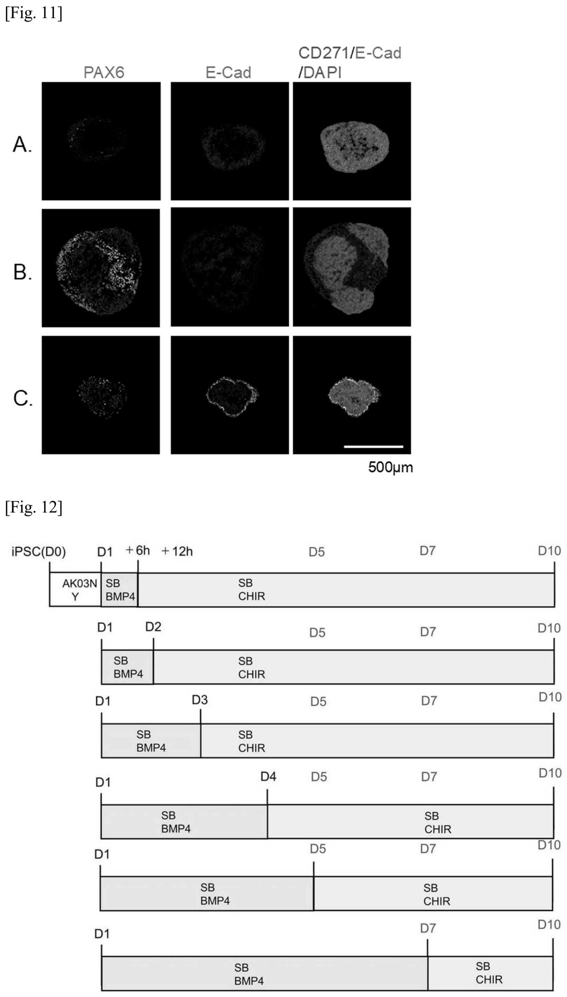

[FIG. 11] Immunofluorescent staining was conducted on CD271 (NCC), PAX6 (neuroectoderm), and E-Cadherin (epithelial cell) under the conditions shown in FIG. 10. (A) NCCs strongly positive for CD271 were induced with high efficiency and cells positive for PAX6 and E-Cadherin were not induced when the action time of SB and BMP4 was set to 1 day. (B) Without the action of SB and BMP4, it was observed that a neuroectoderm positive for PAX6 was induced. (C) It was observed that epithelial cells positive for E-Cadherin were induced around the cell aggregate when the action time of SB and BMP4 was extended.
[FIG. 12] Diagrams illustrating the outlines of the induction methods in which NCCs are induced with the action time of SB and BMP4 being changed. Shaking culture was conducted from the start of the NCC induction step.

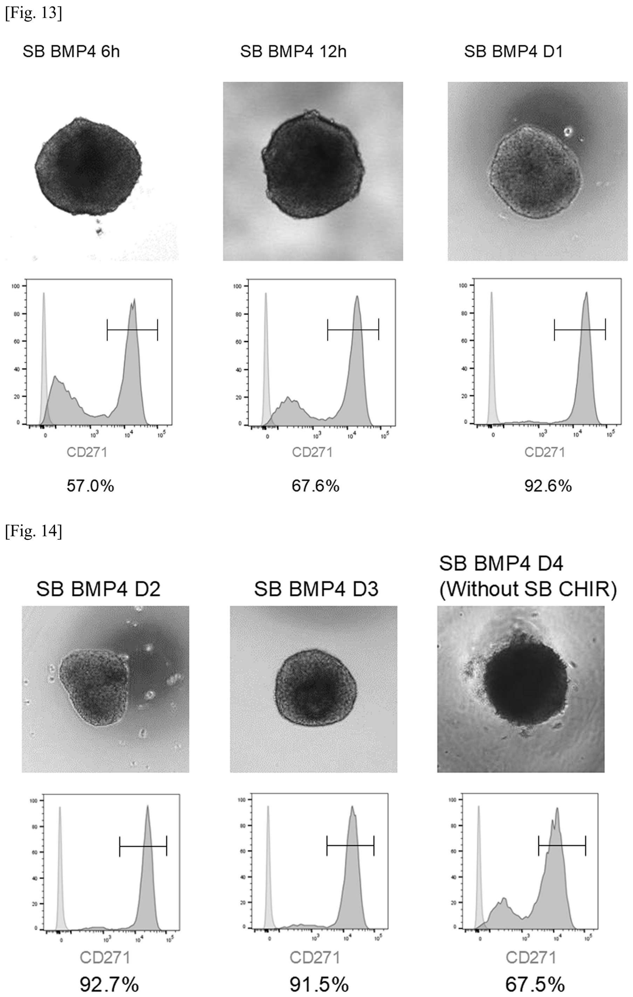

[FIG. 13] The results of measuring the ratios of cells strongly positive for CD271 through flow cytometry under the conditions shown in FIG. 12. It was shown that when SB and BMP4 were allowed to act for 6 hours (SB BMP4 6h) or 12 hours (SB BMP4 12h) and then the verification was made through flow cytometry on Day 5, the NCC induction efficiency decreased compared with the case where the action time of SB and BMP4 was set to 1 day (SB BMP4 D1).
[FIG. 14] The results of measuring the ratios of cells strongly positive for CD271 through flow cytometry under the conditions shown in FIG. 12. It was shown that when SB and BMP4 were allowed to act for 2 days (SB BMP4 D2) or 3 days (SB BMP4 D3) and then the verification was made through flow cytometry on Day 5, high NCC induction effects were obtained in both cases. Meanwhile, it was shown that when the verification was made through flow cytometry on Day 5 without the action of SB and BMP4, low NCC induction effects were obtained.

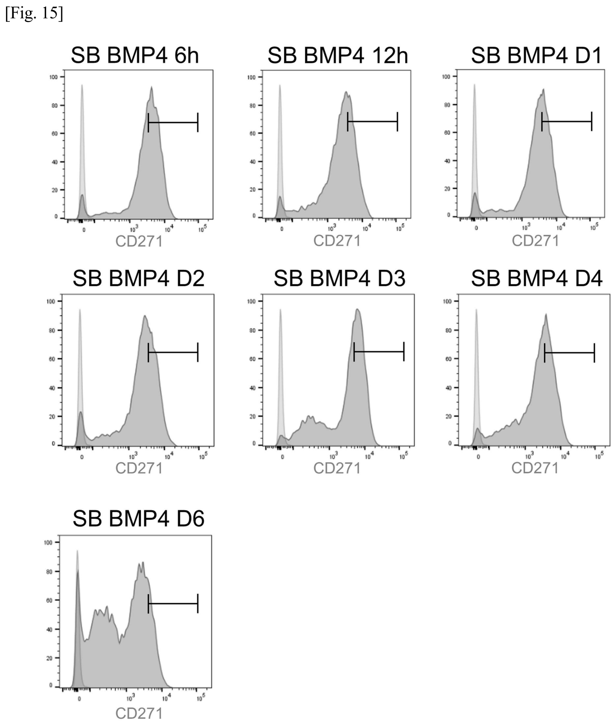

[FIG. 15] The results of measuring the ratios of cells strongly positive for CD271 through flow cytometry under the conditions shown in FIG. 12. It was shown that when SB and BMP4 were allowed to act for 6 hours (SB BMP4 6h), 12 hours (SB BMP4 12h), 1 day (SB BMP4 D1), 2 days (SB BMP4 D2), 3 days (SB BMP4 D3), 4 days (SB BMP4 D4), or 7 days (SB BMP4 D6) and then the verification was made through flow cytometry on Day 7, the expression of CD271 in the whole cell population in all the methods decreased compared with the case where the verification was made on Day 5.

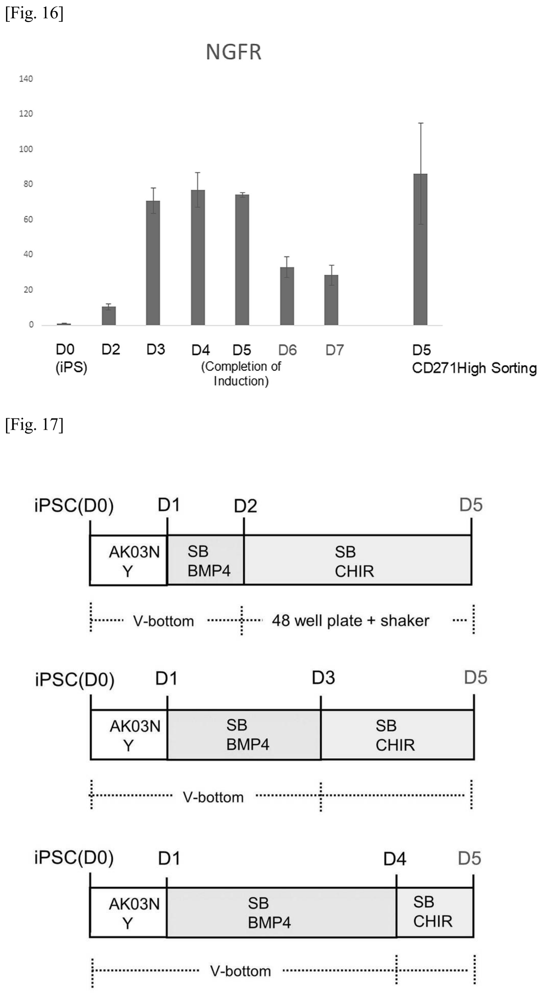

[FIG. 16] The results of measuring the expression levels of NGFR through RT-qPCR under the conditions shown in FIG. 12. It was shown from the results of the RT-qPCR that the expression level of NGFR (CD271) decreased after Day 5 from the start of the culture.
[FIG. 17] The patterns of the action time of SB and BMP4 in the methods that are preferable in terms of the NCC induction efficiency.

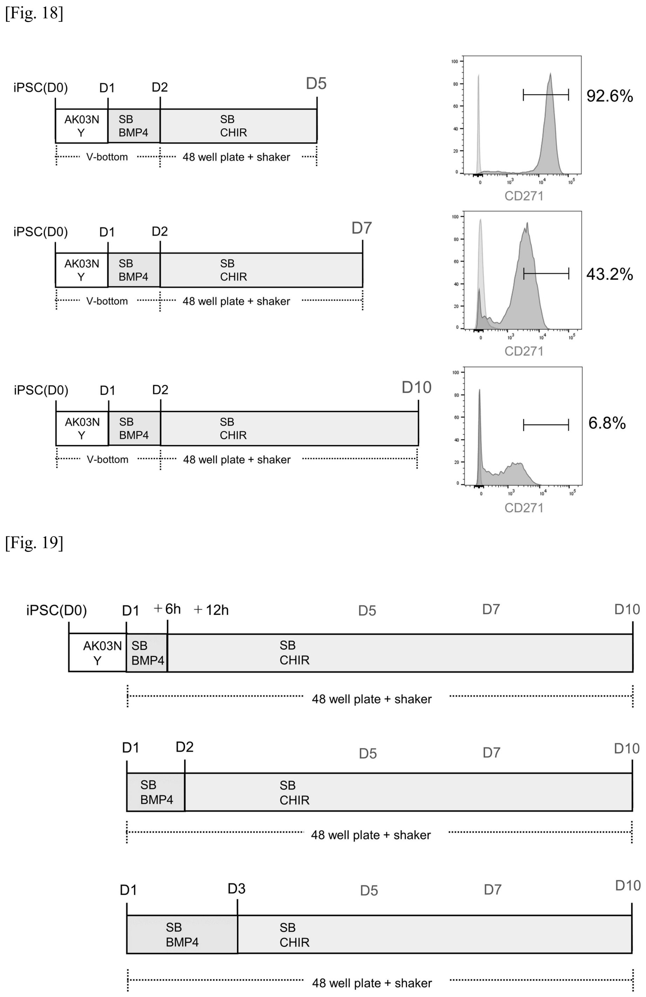

[FIG. 18] The ratio of cells strongly positive for CD271 was measured through flow cytometry when the action time of SB and CHIR was extended. When the entire culture period was extended, the ratio of cells strongly positive for CD271 decreased in a time-dependent manner.
[FIG. 19] Diagrams illustrating the outlines of the induction methods when shaking culture is conducted from the start of the action period of SB431542 and BMP4.

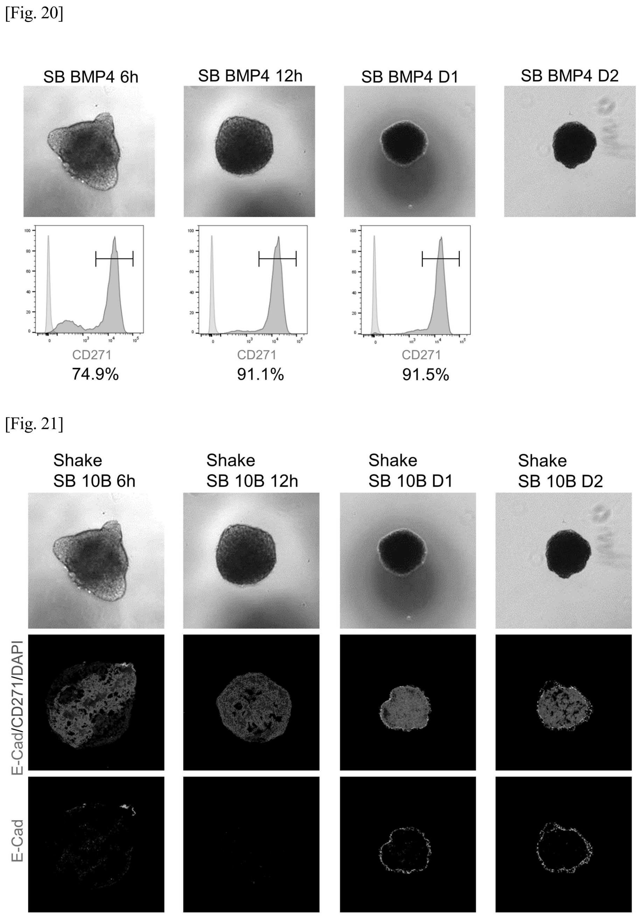

[FIG. 20] The results of measuring the ratios of cells strongly positive for CD271 through flow cytometry under the conditions shown in FIG. 12. When shaking culture was conducted from the start of the SB-BMP4 action step, NCCs could be induced on Day 5 in both cases where the action time of SB and BMP4 was 6 hours (SB BMP4 6h) and 12 hours (SB BMP4 12h). However, in the case where the action time was 12 hours, highly efficient NCC induction to the same extent as the case where the action time of SB and BMP4 was 1 day (SB BMP4 D1) was observed.
[FIG. 21] The results of verifying the expression patterns of the CD271 (NCC marker) gene and the E-Cadherin (epithelial cell marker) gene under the conditions shown in FIG. 12 through immunostaining. When the action time of SB and BMP4 was 1 day or 2 days, epithelial cells were observed around the cell aggregate on Day 5.

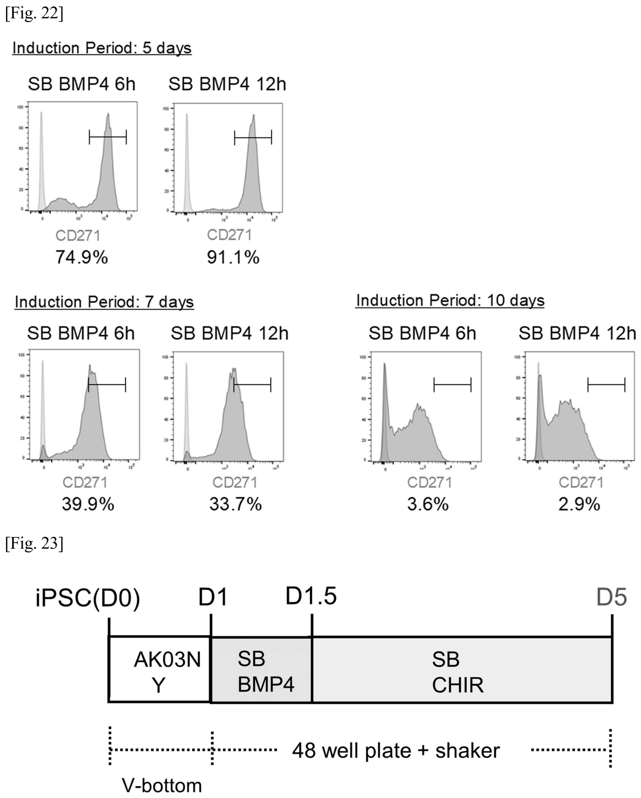

[FIG. 22] The results of measuring the ratios of cells strongly positive for CD271 through flow cytometry under the conditions shown in FIG. 12. It was shown that when the NCC induction step was extended to 7 days or 10 days, the ratio of cells strongly positive for CD271 decreased in both cases.
[FIG. 23] A diagram illustrating the outline of the method that is preferable in terms of the NCC induction efficiency when shaking culture is conducted from the start of the SB-BMP4 action step.

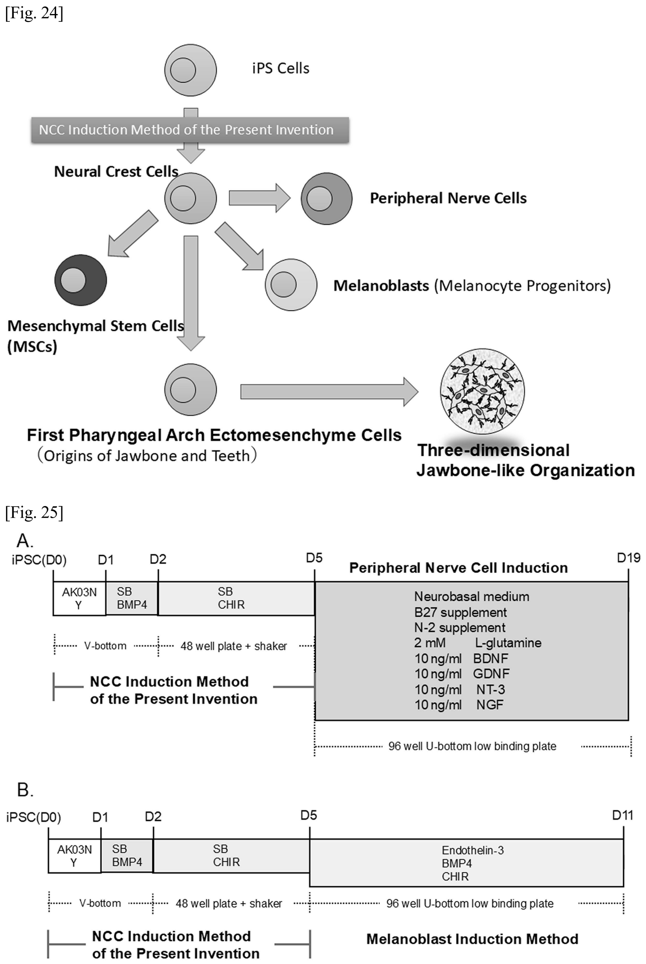

[FIG. 24] A diagram illustrating the outline of induction of differentiation of NCCs obtained using the 3D NCC induction method into various types of cells.
[FIG. 25] A diagram illustrating the outline of a method for inducing peripheral nerve cells from NCCs obtained using the 3D NCC induction method (A), and a diagram illustrating the outline of a method for inducing melanoblasts therefrom (B).

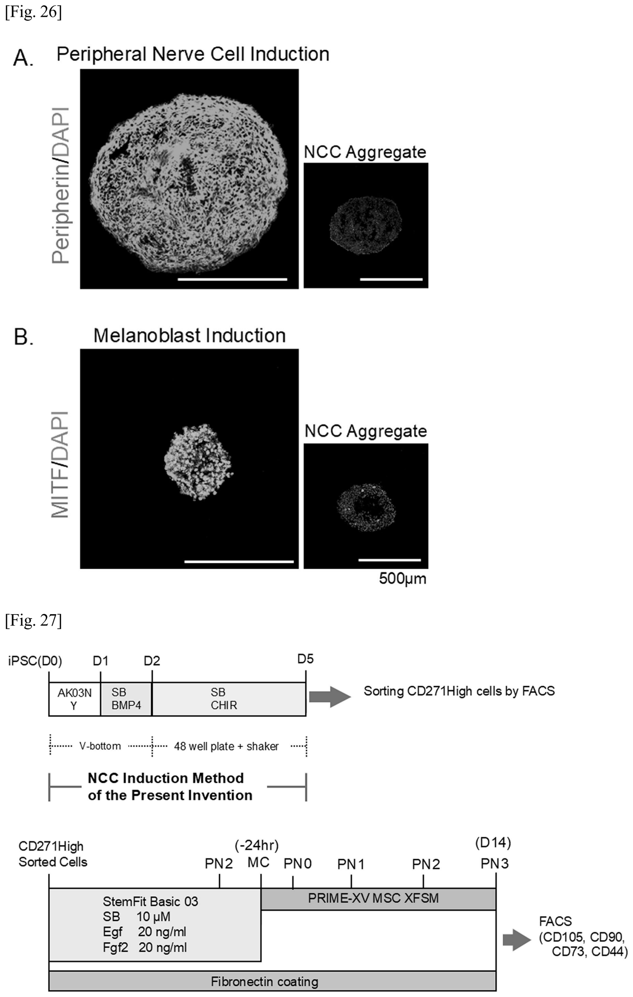

[FIG. 26] The differentiation was checked through immunofluorescent staining after peripheral nerve cells and melanoblasts were induced from NCC aggregates. (A) Peripheral nerve cells were induced from NCC aggregates. Peripherin-positive peripheral nerve cells were induced with high efficiency. (B) Melanoblasts were induced from NCC aggregates. MITF-positive melanoblasts were induced with high efficiency.
[FIG. 27] A diagram illustrating the outline of a method for inducing mesenchymal stem cells from NCCs obtained using the 3D NCC induction method.

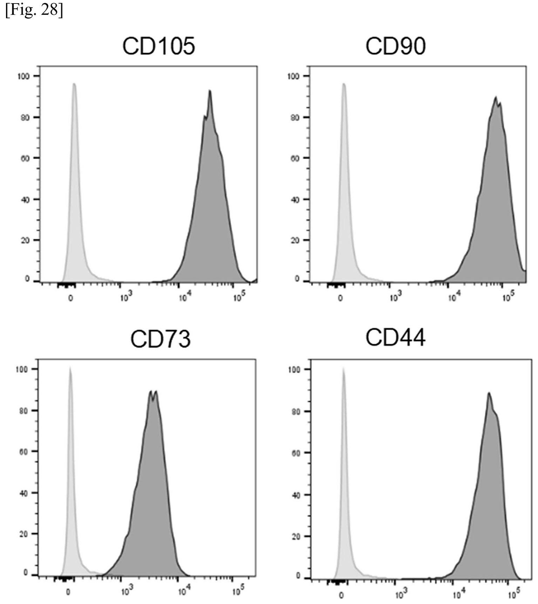

[FIG. 28] NCC aggregates were dispersed using Accutase and seeded on a culture plate, and then MSCs were induced. The MSC markers CD105, CD90, CD73 and CD44 all exhibited high expression.

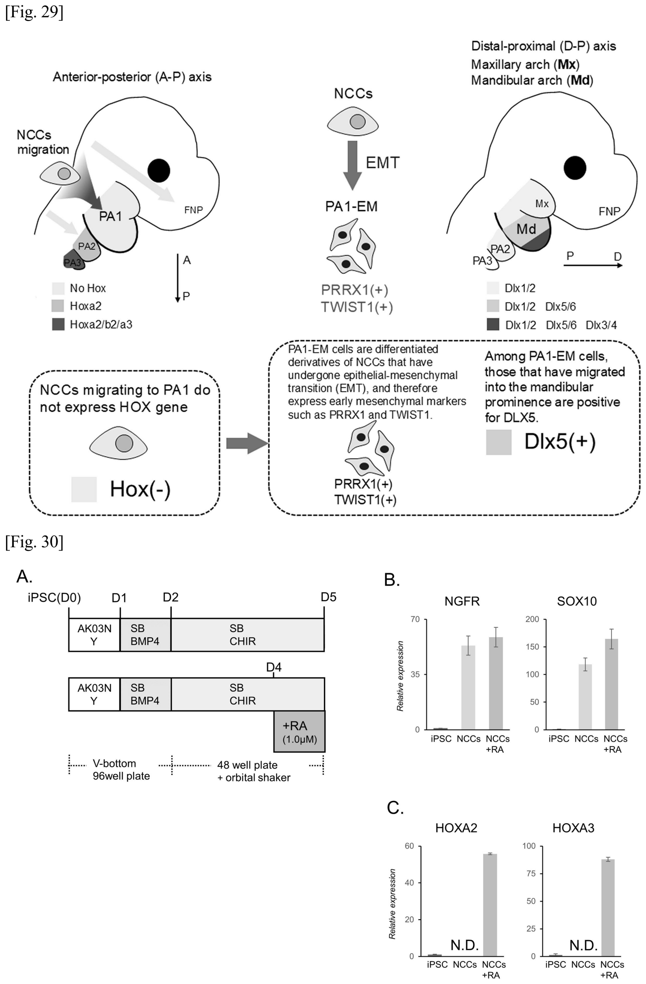

[FIG. 29] Diagrams illustrating the outline of the features of the first pharyngeal arch ectomesenchyme (PA1-EM) cells.
[FIG. 30] The expression of the HOX gene in an NCC aggregate obtained using the 3D NCC induction method was checked through RT-qPCR. An NCC aggregate in which the expression of the HOX gene was induced through retinoic acid (RA) stimulation was prepared as a positive control. (A) A method for inducing a HOX gene-positive NCC aggregate. 1.0 µM RA was added from Day 4 after the start of the induction. (B) The expression of the NCC-related genes (NGFR, SOX10) was checked through RT-qPCR. The expression levels of the NCC-related genes remained the same even when the RA stimulation was conducted. (C) The expression of the HOX genes (HOXA2, HOXA3) was checked through RT-qPCR. The HOX genes were highly expressed in the RA-stimulated NCC aggregate, whereas the expression of the HOX genes was not observed in the non-stimulated NCC aggregate.

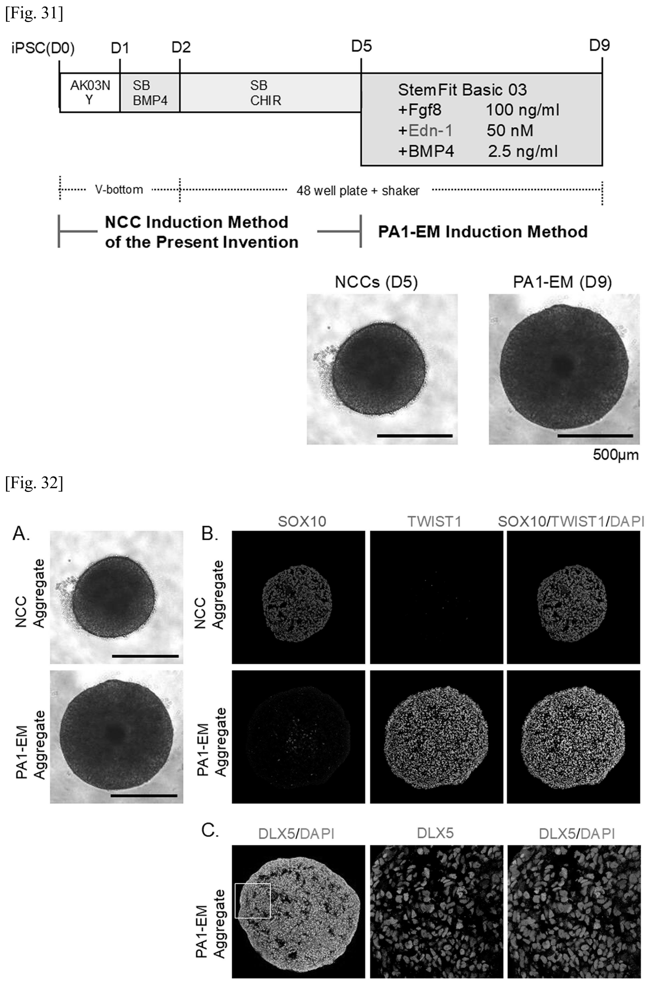

[FIG. 31] Diagrams illustrating the outline of a method for inducing PA1-EM cells from NCCs obtained using the 3D NCC induction method.
[FIG. 32] PA1-EM cells were induced from an NCC aggregate, and then the induction of the PA1-EM cells was checked through immunofluorescent staining of TWIST1 and DLX5. (A) Micrographs of an NCC aggregate and an induced PA1-EM cell aggregate. The inner cells proliferated, which resulted in an increase in the size of the aggregate. (B) Images of immunofluorescence-stained SOX10 and TWIST1. An obvious decrease of the SOX10-expression caused by the PA1-EM cell induction was observed, and the differentiation into a TWIST1-positive ectomesenchyme was confirmed. (C) Images of immunofluorescence-stained DLX5. DLX5 was highly expressed in the induced ectomesenchyme, and the genetic features of the PA1-EM were reproduced.

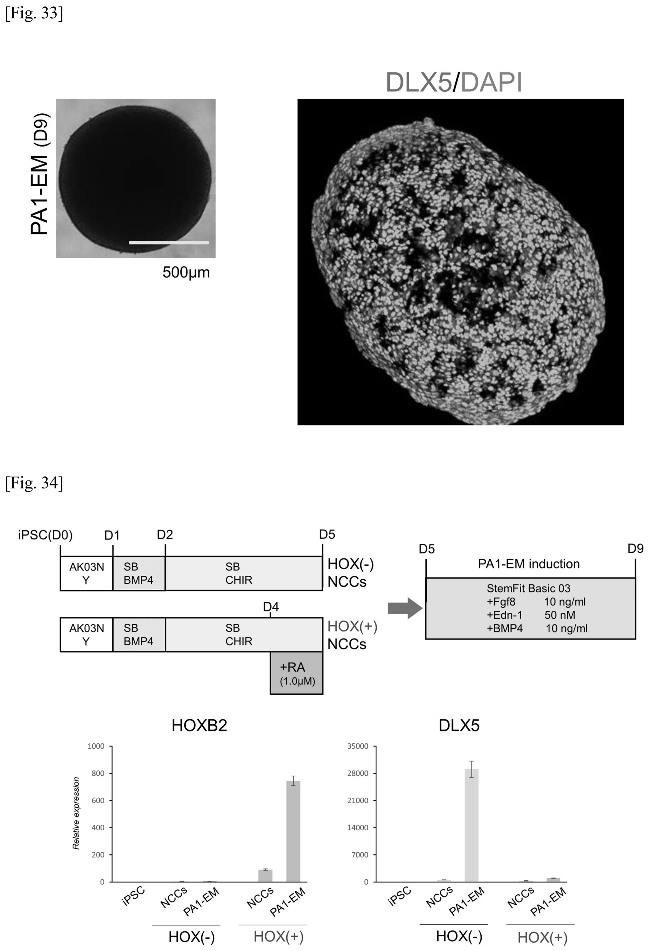

[FIG. 33] PA1-EM cells were induced from iPS cells from a patient with osteogenesis imperfecta, and then the induction of the PA1-EM cells was checked through immunofluorescent staining of DLX5.
[FIG. 34] A diagram illustrating the outline of a method for inducing HOX(-) NCCs ("-" and "⁻" refer to "negative"; the same applies hereinafter) and HOX(+) NCCs ("+" and "⁺" refer to "positive"; the same applies hereinafter) (upper diagram). The expression of HOXB2 and DLX5 in the PA1-EM cells obtained using the induction methods was checked through RT-qPCR. An increase in the expression of DLX5 was not observed in the cells obtained by inducing PA1-EM cells using HOX(+) NCCs (lower diagram).

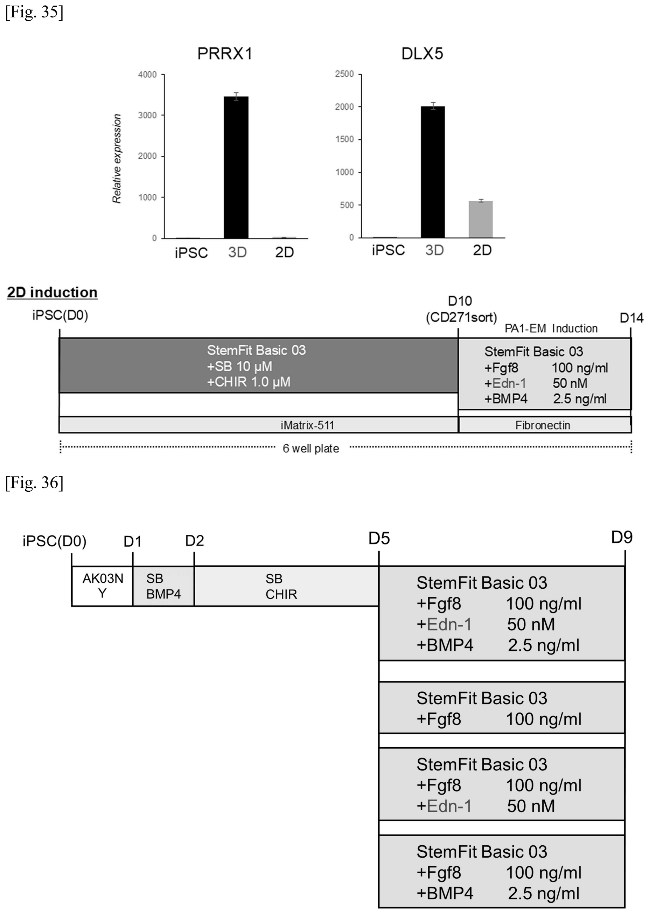

[FIG. 35] Diagrams illustrating the outline of a method for inducing PA1-EM cells from NCCs obtained using the 2D NCC induction method. It was shown that in the obtained cells, the expression of PRRX1 was significantly low, and the expression of DLX5 was also low.
[FIG. 36] A diagram illustrating the outline of an induction method for verifying the best combination of Fgf8, Edn-1, and BMP4 in the induction of PA1-EM cells.

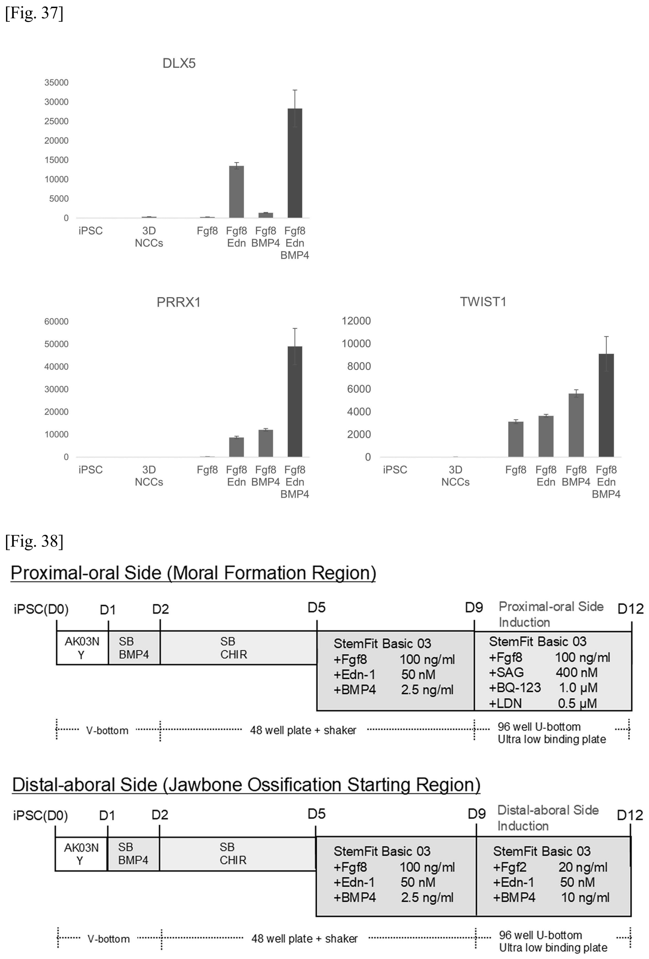

[FIG. 37] The results of measuring the expression levels of DLX, PRRX1, and TWIST1 through RT-qPCR under the conditions shown in FIG. 36. Edn-1 was shown to be essential for the expression of DLX5, and it was shown from the expression levels of DLX5, PRRX1, and TWIST1 that a condition in which all of Fgf8, Edn-1, and BMP4 are added to a culture medium is the optimum condition for the induction of PA1-EM cells.
[FIG. 38] A diagram illustrating the outline of a method for inducing proximal-oral (moral formation region) cells from PA1-EM cells (upper diagram), and a diagram illustrating the outline of a method for inducing distal-aboral (jawbone ossification starting region) cells therefrom (lower diagram).

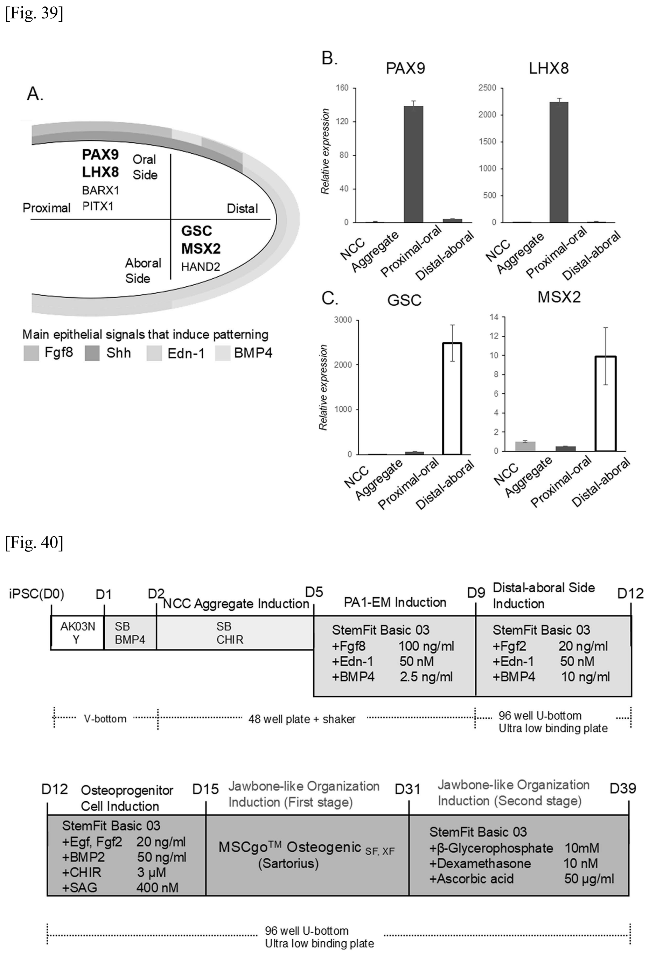

[FIG. 39] It was examined whether the patterning of the first pharyngeal arch could be reproduced and genetic patterns characteristic of the regions could be induced by allowing induced PA1-EM cells positive for DLX5 and TWIST1 to respond to epithelial signals of the first pharyngeal arch. (A) The locations of the epithelial signals in the first pharyngeal arch, and the genetic patterns induced on the proximal-oral side and the distal-aboral side. (B) PAX9 and LHX8 were highly expressed due to the induction on the proximal-oral side. (C) GSC and MSX2 were highly expressed due to the induction on the distal-aboral side.
[FIG. 40] Diagrams illustrating the outline of a method for inducing a jawbone-like tissue from distal-aboral cells.

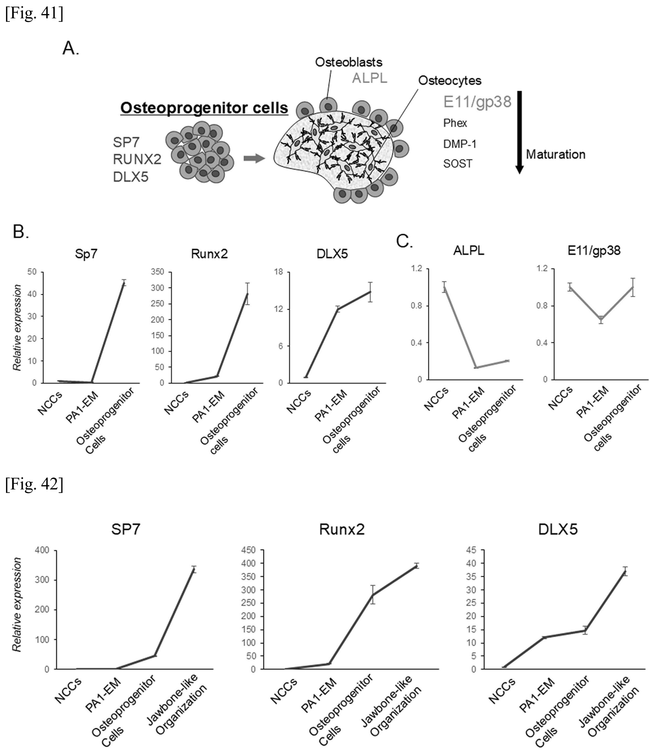

[FIG. 41] Osteoprogenitor cells were induced by conducting induction of differentiation into a bone on a PA1-EM cell aggregate subjected to induction of differentiation into distal-aboral cells. (A) An increase in the expression of the osteoprogenitor cell markers SP7, RUNX2, and DLX5 was observed. (B) An increase in the expression of the early osteoblast marker ALPL and the immature osteocyte marker E11/gp38 was not observed in this induction stage.
[FIG. 42] Two-stage induction of differentiation into a bone was conducted subsequently to the induction shown in FIG. 41. A further increase in the expression of the osteoprogenitor cell markers SP7, RUNX2, and DLX5 was observed.

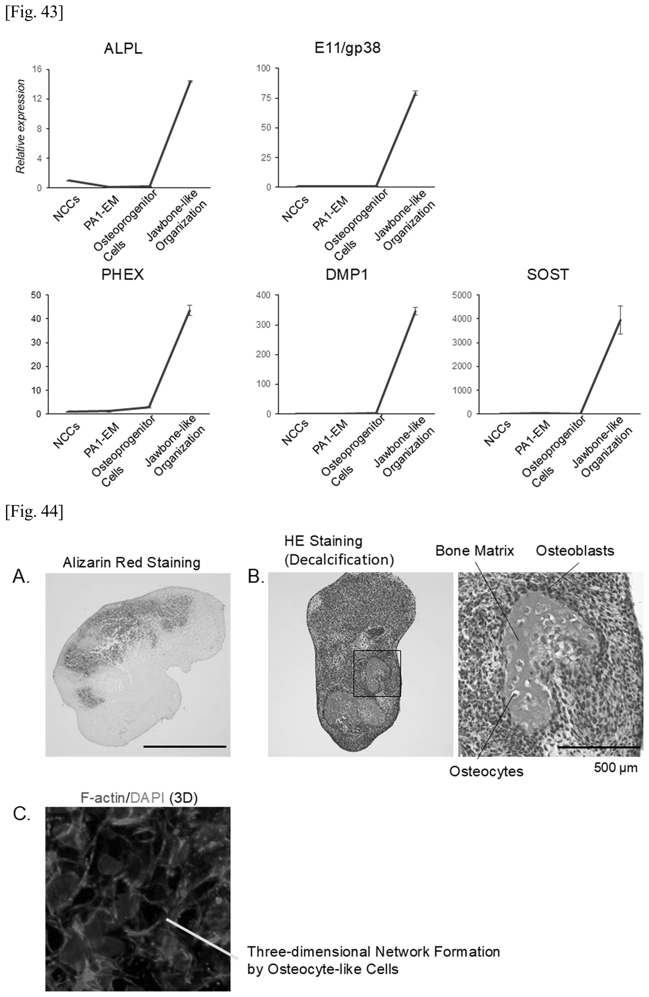

[FIG. 43] Two-stage induction of differentiation into a bone was conducted subsequently to the induction shown in FIG. 41. An obvious increase in the expression of the osteoblast marker ALPL and the osteocyte markers E11/gp38, DMP1, PHEX, and SOST was observed.
[FIG. 44] Histological images of jawbone-like tissues obtained through the above-described induction of differentiation into a bone. (A) An image of an alizarin red-stained non-decalcified section. Mineral deposits capable of being stained with alizarin red were observed. (B) An image of an HE-stained decalcified section. A jawbone-like tissue composed of an eosinophilic bone matrix, osteoblast-like cells around the bone matrix, and osteocyte-like cells buried in the bone matrix was observed inside the cell aggregate. (C) An image of immunostained F-actin in the osteocyte-like cells buried inside the bone matrix. In the bone matrix, the osteocyte-like cells formed a three-dimensional network together with surrounding cells.

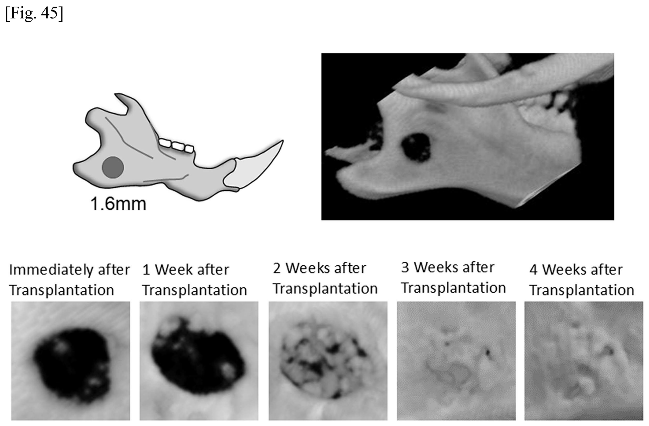

[FIG. 45] The human jawbone-like tissue shown in FIG. 44 was transplanted to a 1.6-mm bone defect formed in the ramus of the right angle of the mandible of an NSG mouse, which is an immunodeficient animal, micro-CT was conducted right after the transplantation and one, two, three, and four weeks after the transplantation to examine an effect of the jawbone-like tissue to regenerate the jawbone. At the site where the jawbone-like tissue was transplanted, blockage of the defect caused by mineral deposits was observed at an early stage three weeks after the transplantation.

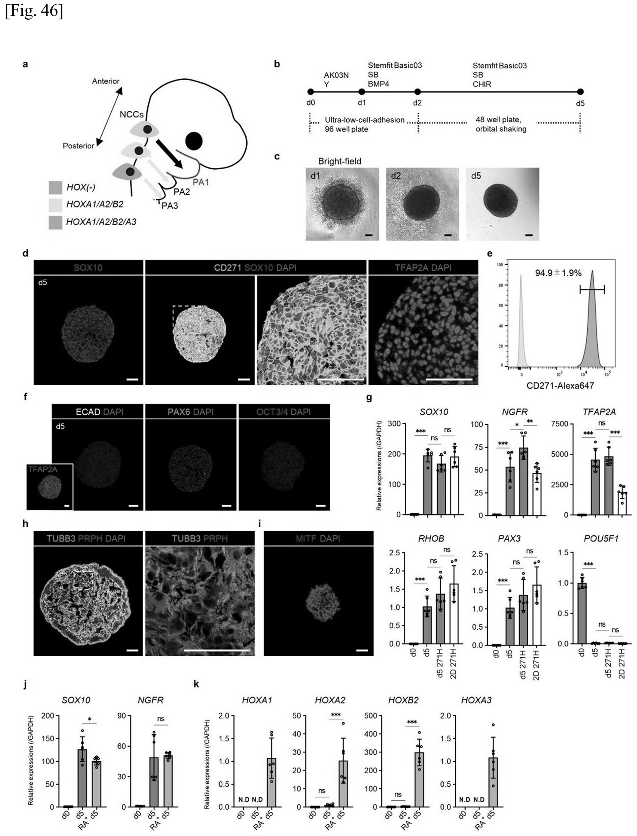

[FIG. 46] Efficient induction of HOX-negative NCCs from 3D cultured iPSCs. a, Schematic representation illustrating the regional specification of cranial neural crest cells (NCCs) along the anterior-posterior axis in an embryo. PA1-3, pharyngeal arch 1-3. b, Differentiation strategy for NCCs from iPSCs in a three-dimensional (3D) culture system. d0-5, day 0-5; Y, Y-27632; SB, SB431542 (TGF-β inhibitor); CHIR, CHIR99021 (GSK3b inhibitor). c, Bright-field images of 1231A3 aggregates on days 1, 2, and 5 in the optimized culture. Scale bars, 100 µm. d, Immunofluorescence staining for SOX10 and CD271, and TFAP2A in serial sections of day 5 aggregates. Nuclei stained with DAPI. Dashed boxes magnified on the right. (n = 34 from eleven biologically independent experiments). Scale bars, 100 µm. e, Flow cytometry analysis of NCC marker (CD271) expression in day 5 aggregates (dark grey). Isotype control shown in light grey. Results shown as mean ± s.e.m. (Six biologically independent experiments). f, Immunofluorescence staining for TFAP2A and E-Cadherin (ECAD, white), PAX6, and OCT3/4 in day 5 aggregates. Nuclei stained with DAPI. (n = 3 from three biologically independent experiments). Scale bars, 100 µm. g, Gene expression analysis for NCC markers (*SOX10, NGFR, TFAP2A, RHOB, PAX3*) and pluripotent stem cell marker (*POU5F1*) by Real-time quantitative PCR (RT-qPCR) analysis. Gene expression levels quantified using GAPDH as a reference control and compared among iPSCs (d0), day 5 aggregates (d5), CD271^{high+} NCCs sorted from day 5 aggregates (d5 271H), and CD271^{high+} NCCs sorted from previous two-dimensional (2D) protocols (2D 271H). Data normalized to d0 values. Values shown as mean ± s.e.m. (n = 6 from three biologically independent experiments). h, i, Immunofluorescence staining for Peripherin (PRPH) and class III β-Tubulin (TUBB3) in aggregates after peripheral neuron induction (h) and for MITF in aggregates after melanoblast induction (i). Nuclei stained with DAPI. Dashed boxes magnified on the right. Magnified images represent z-stack projection of 15 confocal optical sections (step size, 0.5 µm) (h). (n = 6 from three biologically independent experiments). Scale bars, 100 µm. j, k, Gene expression analysis for NCC markers (*NGFR, SOX10*) (j) and HOX genes (*HOXA1, HOXA2, HOXB2, HOXA3*) (k) by RT-qPCR. Gene expression levels quantified using GAPDH as a reference control and compared among iPSCs (d0), day 5 aggregates (d5), and retinoic acid (RA)-treated day 5 aggregates (RA⁺ d5). Data normalized to iPSCs (d0) values. Values shown as mean ± s.e.m. (n = 6 from three biologically independent experiments). g, j, k, **P* < 0.05, ***P* < 0.01, ****P* < 0.001 by one-way ANOVA. N.D, not determined; ns, not significant. c-f, h, i, Representative data are shown.

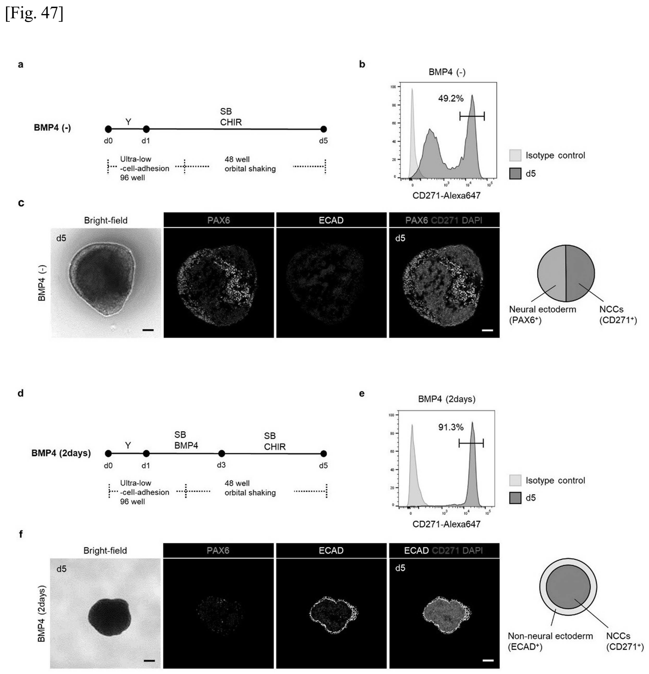

[FIG. 47] Influence of BMP4 treatment duration on NCC induction efficiency in 3D cultures. a, d, Experimental overview testing the effect of BMP4 treatment duration on NCC induction efficiency. Aggregates were treated without (-) (a) or with BMP4 for 2 days (d). SB, SB431542; CHIR, CHIR99021. b, e, Flow cytometry analysis of NCC marker (CD271) expression in day 5 aggregates (dark grey). Isotype control shown in light grey. The absence of BMP4 (-) decreased the CD271^{high+} cell population (b). c, f, Bright-field images and immunofluorescence staining for PAX6, ECAD (white), and CD271 in day 5 aggregates. Nuclei stained with DAPI. The absence of BMP4 (-) induced not only CD271^{high+} NCCs but also undesired PAX6⁺ neuroectoderm (c). Conversely, the BMP4 (2 days) condition induced E-Cadherin (ECAD)⁺ cells on the surface of aggregates (f) (n = 6 from three biologically independent experiments). Scale bars, 100 µm. b, c, e, f, Representative data are shown.

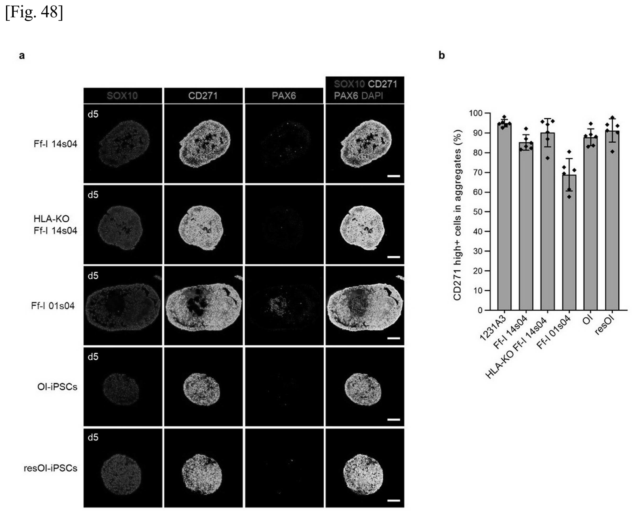

[FIG. 48] Highly efficient induction of NCCs from various iPSC lines. A, Triple-color immunofluorescence staining for SOX10, CD271, and PAX6 in aggregates generated from various iPSC lines (Ff-I 14s04, Ff-I 14s04 knockout of human leukocyte antigen (HLA)-A, B, and CIITA genes (HLA-KO Ff-I 14s04), Ff-I 01s04, Osteogenesis imperfecta (OI) patient-derived iPSCs (OI-iPSCs), and mutation-rescued OI-iPSCs (resOI-iPSCs)). Nuclei were stained with DAPI. (n = 3 from three biologically independent experiments). Representative data are shown. Scale bars, 100 µm. b, Flow cytometry analysis of NCC marker (CD271) in day 5 aggregates. Results presented as mean ± s.e.m. (six biologically independent experiments).

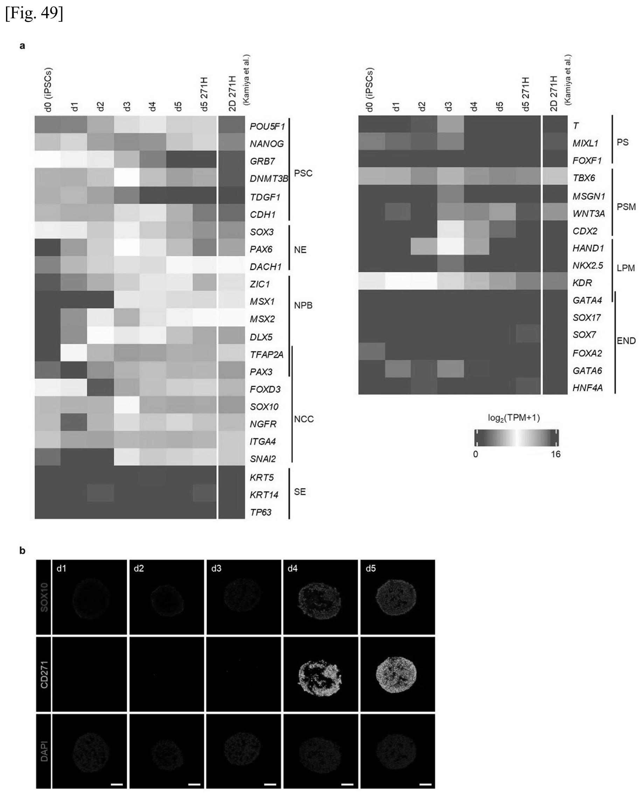

[FIG. 49] Stepwise differentiation of NCCs from iPSCs via ectodermal lineages. a, RNA-seq analysis of 1231A3 iPSCs (d0), aggregates at days 1-5, CD271^{high+} NCCs sorted from day 5 aggregates (d5 271H), and CD271^{high+} NCCs sorted from previous two-dimensional (2D) protocols (2D 271H) (Non-Patent Literature 3). Heatmap illustrates gene expression levels of pluripotent stem cell (PSC), ectodermal lineage (neural ectoderm (NE), neural plate border (NPB), neural crest cell (NCC), and surface ectoderm (SE)), primitive streak (PS), presomitic mesoderm (PSM), lateral plate mesoderm (LPM), and endoderm (END) markers. log₂(Transcripts Per Million (TPM) + 1) values were used for gene expression evaluation. b, Immunofluorescence staining for SOX10, and CD271 in aggregates at days 1-5. Nuclei stained with DAPI. (n = 3 from three biologically independent experiments). Representative data are shown. Scale bars, 100 µm.

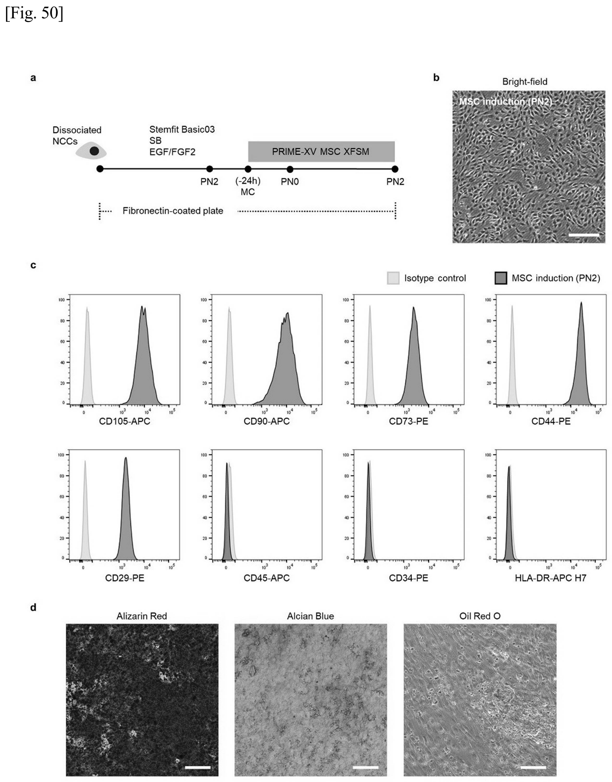

[FIG. 50] MSC induction from NCC aggregates. a, Differentiation strategy of mesenchymal stem/stromal cells (MSCs). b, Bright-field images of cells in MSC induction (PN2). Scale bars, 200 µm. c, Flow cytometry analysis of MSC-related surface markers in cells in MSC induction (PN2). MSC induction (PN2) (dark grey) and isotype control (light grey). Cells in MSC induction (PN2) were positive for CD105, CD90, CD73, CD44, and CD29, and negative for CD45, CD34, and HLA-DR. (Three biologically independent experiments). d, Differentiation properties of MSCs. Differentiation of osteogenic, chondrogenic, and adipogenic lineages confirmed by Alizarin Red, Alcian Blue, and Oil Red O staining, respectively. (Two biologically independent experiments). Scale bars, 100 µm. b-d, Representative data are shown.

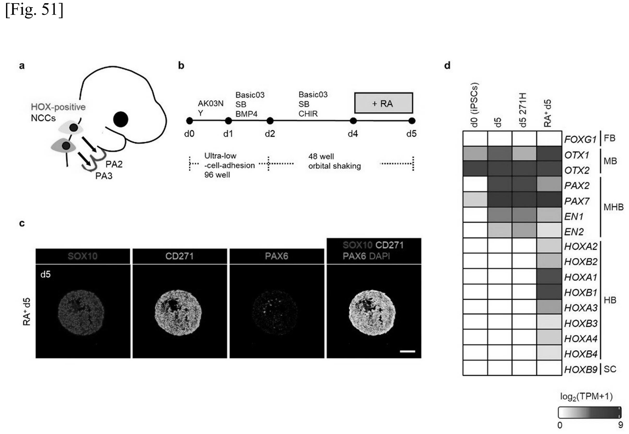

[FIG. 51] Induction of HOX-positive NCC aggregate from iPSCs. a, Schematic model of HOX-positive NCCs colonizing posterior PAs. b, Differentiation strategy for HOX-positive NCCs. c, Triple-color immunofluorescence staining for SOX10, CD271, and PAX6 in RA-treated day 5 aggregates (RA⁺ d5). Nuclei stained with DAPI. (n = 6 from three biologically independent experiments). Representative data are shown. Scale bars, 100 µm. A large number of cells in RA⁺ d5 differentiated into CD271⁺SOX10⁺ NCCs. d, Heatmap illustrating expression levels of regional markers, forebrain (FB), midbrain (MB), midbrain-hindbrain border (MHB), hindbrain (HB), and spinal cord (SC) genes in RNA-seq analysis of 1231A3 iPSCs (d0), day 5 aggregates without RA treatment (d5), CD271^{high+} NCCs sorted from day 5 aggregates (d5 271H), and RA⁺ d5.

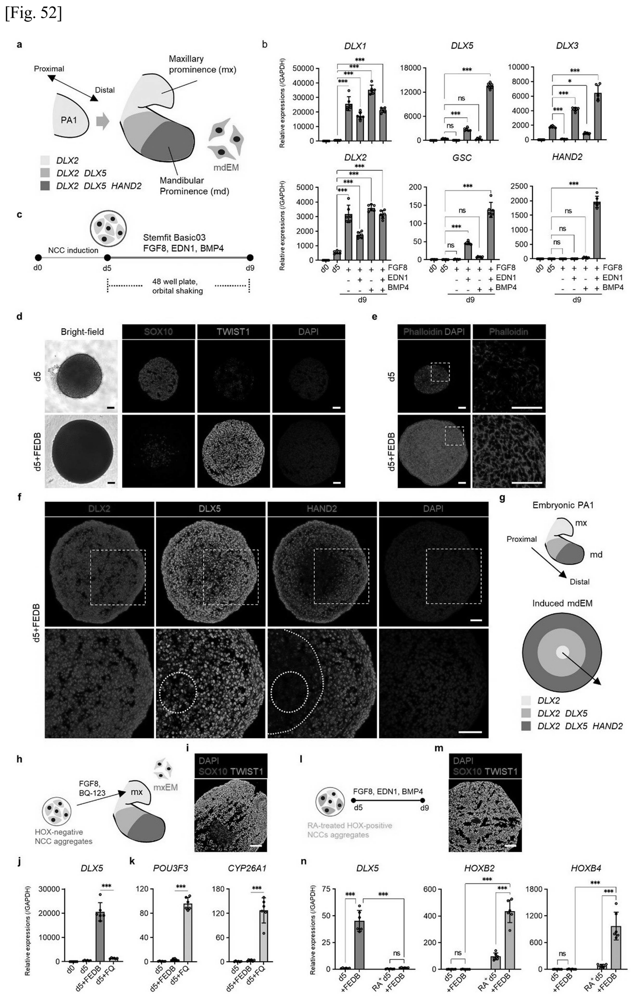

[FIG. 52] Directed differentiation of HOX-negative NCC aggregates toward mdEM. a, Schematic model illustrating the regional specification of NCC-derived pharyngeal arch ectomesenchyme along the proximal-distal (P-D) axis in an embryo. mdEM, mandibular prominence ectomesenchyme. b, Gene expression analysis for common PA1 ectomesenchyme markers (*DLX1, DLX2*), mdEM markers (*DLX5, DLX3, GSC*), and distal region marker (*HAND2*) by RT-qPCR. Gene expression levels quantified using GAPDH as a reference control and compared among 1231A3 iPSCs (d0), day 5 NCC aggregates (d5), and d5 treated with combinations of FGF8, Endothelin-1 (EDN1), and BMP4 for 4 days (d9). Data normalized to d0 values. Values shown as mean ± s.e.m. (n = 6 from three biologically independent experiments). c, Differentiation strategy of mdEM from NCC aggregates. d, Bright-field images and immunofluorescence staining for SOX10 and TWIST1 in d5 (top panels) and d5 treated with FEDB (combination of FGF8, EDN1, and BMP4) for 4 days (d5+FEDB) (bottom panels). Nuclei stained with DAPI. (n = 6 from three biologically independent experiments). Scale bars, 100 µm. e, Phalloidin staining of d5 and d5+FEDB. Nuclei stained with DAPI. (n = 6 from three biologically independent experiments). Scale bars, 50 µm. f, Triple-color immunofluorescence staining for DLX2, DLX5, and HAND2 in d5+FEDB. Nuclei stained with DAPI. Bottom panels show magnified views of the white dashed boxes in the top panels. (n = 15 from eight biologically independent experiments). White dotted circle, DLX5-weak and HAND2-negative area; white dotted line, HAND2-negative area. Scale bars, 100 µm. g, Schematic model illustrating the regional specification of embryonic PA1 and induced mdEM along the P-D axis. h, Schematic model illustrating the strategy to examine the effect of EDN1 signal on the bifurcation of maxillary prominence ectomesenchyme (mxEM) and mdEM. i, Immunofluorescence staining for SOX10 and TWIST1 in d5 treated with FGF8 and BQ-123 for 7 days (d5+FQ). Nuclei stained with DAPI. (n = 3 from three biologically independent experiments). Scale bars, 100 µm. j, k, Gene expression analysis for mdEM marker (*DLX5*) (j) and mxEM markers (*POU3F3, CYP26A1*) (k) by RT-qPCR. Gene expression levels quantified using GAPDH as a reference control and compared among d0, d5, d5+FEDB, and d5+FQ. Data normalized to d0 (j) or d5 (k) values. Values shown as mean ± s.e.m. (n = 6 from three biologically independent experiments). 1, Schematic model illustrating the strategy to examine the importance of initial A-P identity in mdEM differentiation. m, Immunofluorescence staining for SOX10 and TWIST1 in RA⁺ d5 treated with FEDB for 4 days (RA⁺ d5+FEDB). Nuclei stained with DAPI. (n = 3 from three biologically independent experiments). Scale bars, 100 µm. n, Gene expression analysis for mdEM marker (*DLX5*) and HOX genes (*HOXB2, HOXB4*)*.* Gene expression levels quantified using GAPDH as a reference control and compared among d5, d5+FEDB, RA⁺ d5, and RA⁺ d5+FEDB. Data normalized to the d5 values. Values shown as mean ± s.e.m. (n = 6 from three biologically independent experiments). b, j, k, n, **P* < 0.05, ****P* < 0.001 by one-way ANOVA. ns, not significant. d-f, i, m, Representative data are shown.

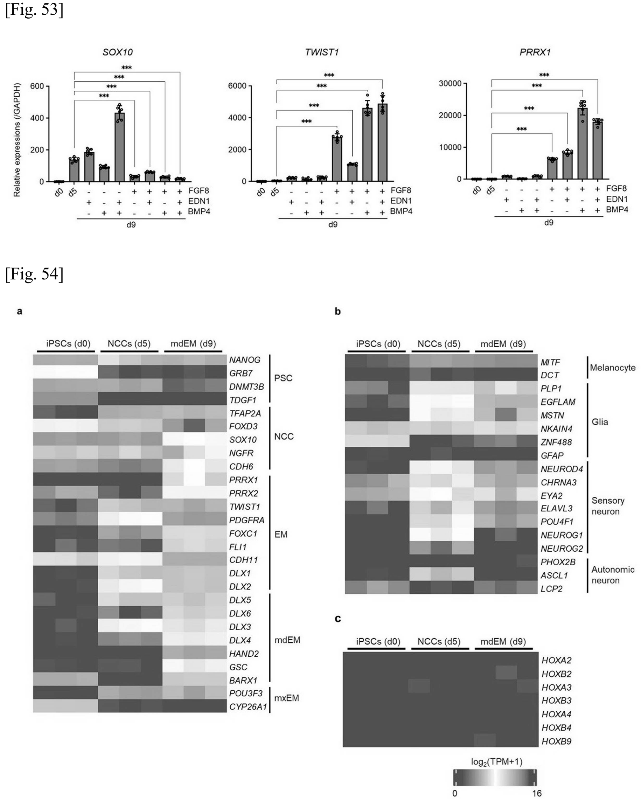

[FIG. 53] Gene expression analysis for NCC marker (*SOX10*) and early pharyngeal arch mesenchyme markers (*TWIST1, PRRX1*) by RT-qPCR. Gene expression levels quantified using GAPDH as a reference control and compared among 1231A3 iPSCs (d0), day 5 NCC aggregates (d5), and d5 treated with combinations of FGF8, Endothelin-1 (EDN1), and BMP4 for 4 days (d9). Data normalized to values of iPSCs (d0) as 1.0. Values shown as mean ± s.e.m. (n = 6 from three biologically independent experiments). ****P* < 0.001 by one-way ANOVA.
[FIG. 54] Selective induction of mdEM from NCC aggregates. a-c, RNA-seq analysis of 1231A3 iPSCs (d0), NCC aggregates (d5), and mdEM (d9). Heatmap illustrating gene expression levels of pluripotent stem cell (PSC), neural crest cell (NCC), pan-ectomesenchyme (EM), mandibular ectomesenchyme (mdEM), and maxillary ectomesenchyme (mxEM) markers (a); melanocyte, glia, sensory neuron, and autonomic neuron markers (b); HOX genes (c). log₂(Transcripts Per Million (TPM) + 1) values were used for gene expression evaluation. (n = 3 from three biologically independent experiments).

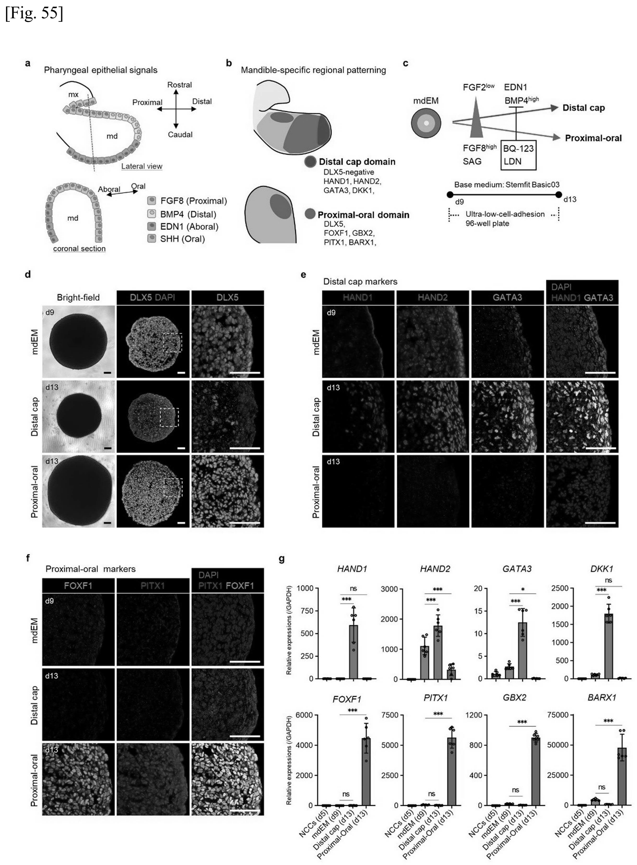

[FIG. 55] mdEM recapitulates regional patterning of distal cap and proximal-oral domain in mandibular prominence. a, b, Schematic representation of major pharyngeal epithelial signals (a) and regional patterning of mdEM (b) in the mandibular prominence. The dotted line corresponds to the coronal section. c, Strategies for inducing distal cap and proximal-oral domain from mdEM. LDN, LDN193189. d, e, f, Bright-field images and immunofluorescence staining for DLX5 (d), HAND1 and GATA3 (e), HAND2 (e), FOXF1 and PITX1 (f) in 1231A3 mdEM (d9, top panels) and aggregates after distal cap (d13, middle panels) or proximal-oral domain (d13, bottom panels) induction. Nuclei stained with DAPI. (n = 6 from three biologically independent experiments). Scale bars, 100 µm. In d, dashed boxes magnified on the right. g, Gene expression analysis for distal cap marker (*HAND1*)*,* distal region markers (*HAND2, GATA3, DKK1*)*,* proximal-oral domain related genes (*FOXF1, PITX1, GBX2, BARX1*) by RT-qPCR. Gene expression levels quantified using GAPDH as a reference control and compared among d5, d9, and d13 (distal cap or proximal-oral domain) aggregates. Data normalized to d5 values. Values shown as mean ± s.e.m. (n = 6 from three biologically independent experiments). **P* < 0.05, ****P* < 0.001 by one-way ANOVA. ns, not significant. d-f, Representative data are shown.

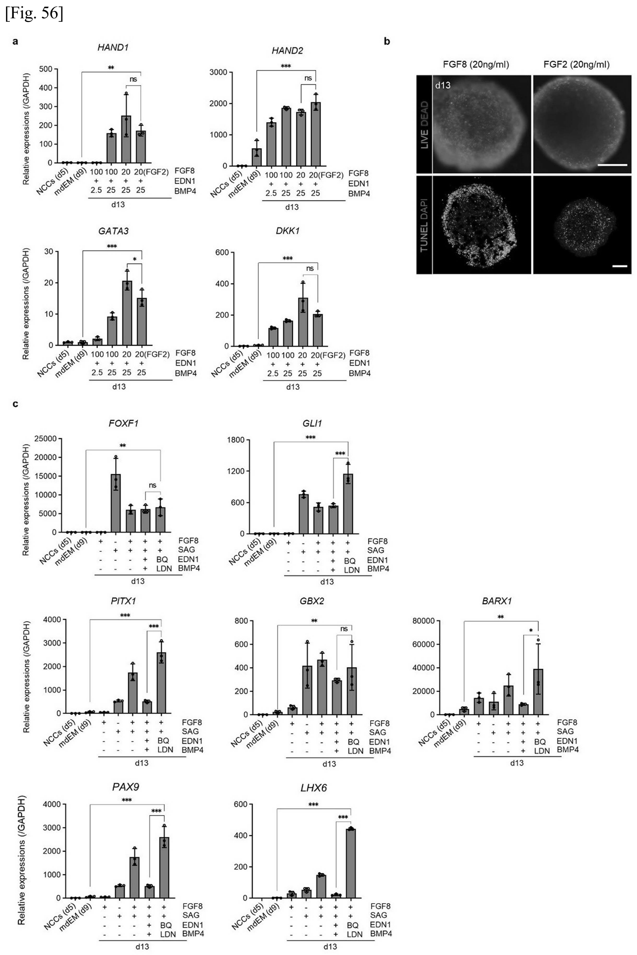

[FIG. 56] Investigation of optimized protocols for distal cap and proximal-oral (molar) domain induction. a, Gene expression analysis for distal cap marker (*HAND1*) and other distal region markers (*HAND2, GATA3, DKK1*) by RT-qPCR. Gene expression levels quantified using GAPDH as a reference control and compared among 1231A3 NCC aggregates (d5), mdEM (d9), and mdEM cultured with various combinations of FGF8 (20 and 100 ng/ml) or FGF2 (20 ng/ml), EDN1 (50 nM), and BMP4 (2.5 and 25 ng/ml) for 4 days (d13). Data normalized to iPSCs (d0) values. Values shown as mean ± s.e.m. (n = 3 from three biologically independent experiments). b, Cell viability analysis of mdEM cultured with FGF8 or FGF2 (20 ng/ml), EDN1 (50 nM), and BMP4 (25 ng/ml) from day 5 to 9. Live cells and dead cells distinguished using a LIVE/DEAD Viability/Cytotoxicity kit. Apoptotic cells detected by TUNEL staining. Nuclei stained with DAPI. (n = 3 from three biologically independent experiments). Representative data are shown. Scale bars, 100 µm. c, Gene expression analysis for proximal-oral (molar) domain-related markers (*FOXF1, GLI1, PITX1, GBX2, BARX1, PAX9, LHX6*). Gene expression levels quantified using GAPDH as a reference control and compared among NCC aggregates (d5), mdEM (d9), and mdEM cultured with various combinations of FGF8 (100 ng/ml), SAG (400 nM), BQ-123 (BQ) (10 µM), LDN193189 (LDN) (500 nM), EDN1 (50 nM), and BMP4 (2.5 ng/ml) for 4 days (d13). Data normalized to iPSCs (d0) values. Values shown as mean ± s.e.m. (n = 3 from three biologically independent experiments). a, c, **P* < 0.05, ***P <* 0.01, ****P* < 0.001 by one-way ANOVA. ns, not significant.

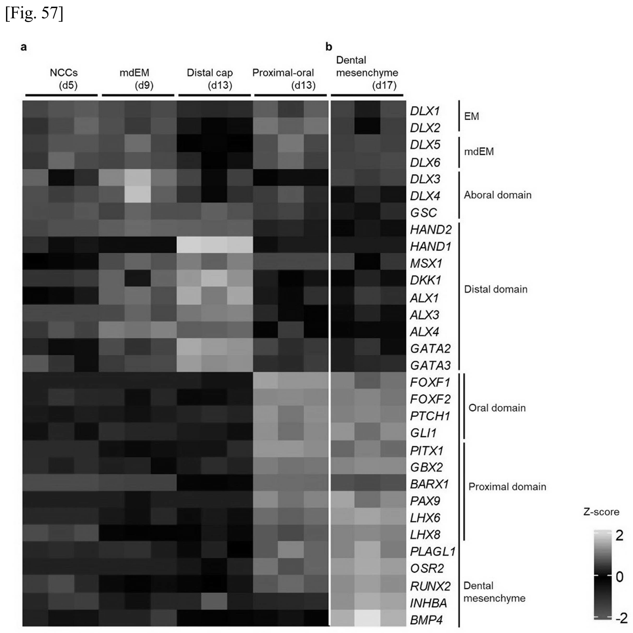

[FIG. 57] mdEM recapitulates the regional patterning of mandibular prominence. RNA-seq analysis of 1231A3 iPSC-derived NCC aggregates (d5), mdEM (d9), distal cap (d13), and proximal-oral domain (d13). Heatmap visualizing the gene expression levels of ectomesenchyme (EM), mdEM, aboral domain, distal domain, oral domain, proximal domain, and dental mesenchyme markers. (n = 3 from three biologically independent experiments). Z-scores used to evaluate gene expression levels.

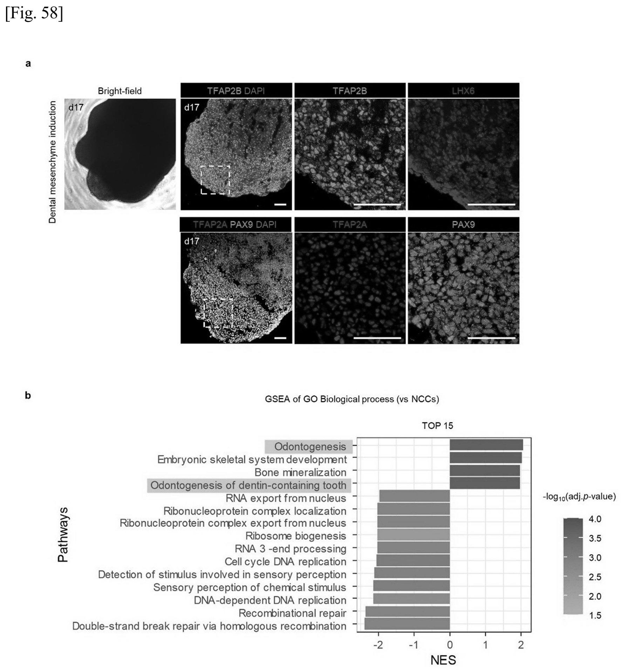

[FIG. 58] Dental mesenchyme induction from mdEM of the proximal-oral domain. a, Bright-field image and immunofluorescence staining for TFAP2B and LHX6, TFAP2A and PAX9 in day 17 aggregates after dental mesenchyme induction. Nuclei stained with DAPI. Dashed boxes magnified to the right. (n = 3 from three biologically independent experiments). Representative data are shown. Scale bars, 100 µm. b, RNA-seq analysis of NCC aggregates (d5) and dental mesenchyme (d17). Gene-Set Enrichment Analysis (GSEA). Top 15 enriched (activated and suppressed) pathways according to GO terms for biological processes shown. Pre-ranked gene lists generated by multiplying the -log10(adj. *p*-value) by the direction of the fold-change. (n = 3 from three biologically independent experiments). NES, normalized enrichment score.

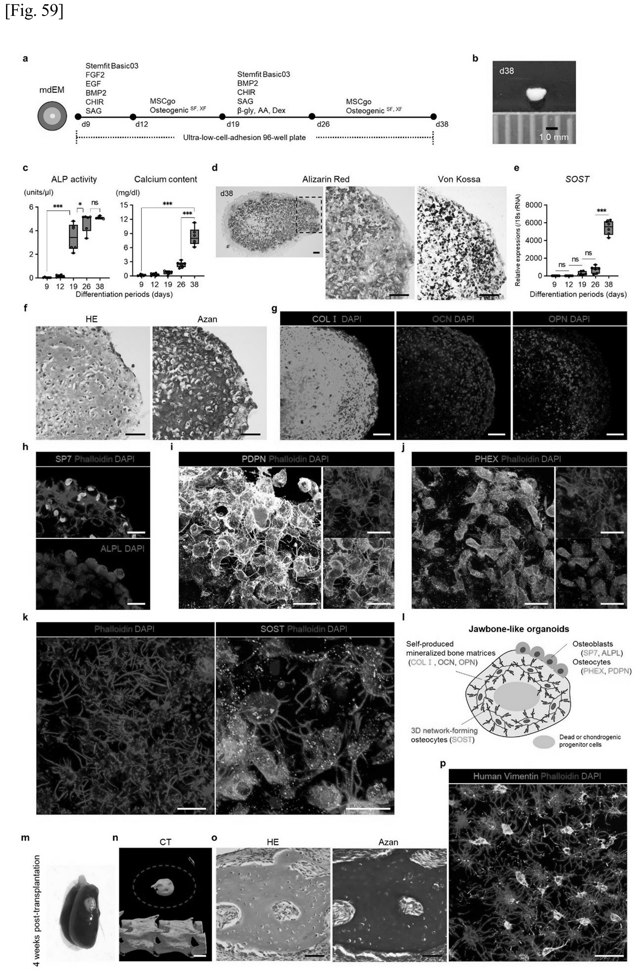

[FIG. 59] Generation and characterization of jawbone-like organoids. a, Stepwise induction strategy of jawbone-like organoids from mdEM. β-gly, β-glycerophosphate disodium salt hydrate; AA, Ascorbic acid 2-phosphate sesquimagnesium salt hydrate; Dex, Dexamethasone. b, Image of 1231A3-derived d38 cluster. Scale bar, 1.0 mm. c, Monitoring alkaline phosphatase (ALP) activity and calcium content in clusters during osteogenic induction. Values shown as mean ± s.e.m. (n = 6 from three biologically independent experiments). d, Alizarin Red and von Kossa staining in serial sections of d38 clusters. Dashed boxes magnified on the right. (Alizarin Red, n = 12; von Kossa, n = 6 from three biologically independent experiments). Scale bars, 100 µm. e, Gene expression analysis for mature osteocyte marker (*SOST*) by RT-qPCR. Gene expression levels quantified using 18s rRNA as a reference control and compared among clusters during osteogenic induction. Data normalized to d9 values. Values shown as mean ± s.e.m. (n = 6 from three biologically independent experiments). f, Haematoxylin and eosin (HE), and Azan staining in serial sections of d38 clusters (decalcified specimens). (n = 52 from twelve biologically independent experiments). Scale bars, 100 µm. g, Triple-color immunofluorescence staining for COLI, OCN, and OPN in d38 tissues (decalcified specimens). Nuclei stained with DAPI. (n = 9 from three biologically independent experiments). Scale bars, 100 µm. h-k, Immunofluorescence staining for SP7 and ALPL (h), PDPN (i), PHEX (j), SOST (k) in d38 tissues (decalcified specimens). Nuclei and F-actin stained with DAPI and Phalloidin, respectively. Images represent the z-stack projection of 40 confocal optical sections (step size, 0.5 µm) (i-k). (h, n = 6; i, n = 12; j, n = 6; k, n = 6 from four biologically independent experiments). Scale bars, 25 µm. 1, Schematic model of generated jawbone-like organoids. m, Image of a kidney of NSG mice at 4 weeks after jawbone-like organoid transplantation. n, 3D reconstructed micro-computed tomographic (micro-CT) image of the renal region at 4 weeks after transplantation. Dashed circle indicates a ectopic mineralized tissue. Scale bar, 1.0 mm. (n = 6). o, HE and Azan (decalcified specimens) staining in serial sections of transplanted tissues. (n = 6). Scale bars, 100 µm. p, Immunofluorescence staining for human Vimentin in the transplanted tissues (decalcified specimens). Nuclei and F-actin stained with DAPI and Phalloidin, respectively. Images represent the z-stack projection of 40 confocal optical sections (step size, 0.5 µm). (n = 6). Scale bars, 25 µm. c, e, **P* < 0.05, ****P* < 0.001 by one-way ANOVA. ns, not significant. b, d, f-k, m-p, Representative data are shown.

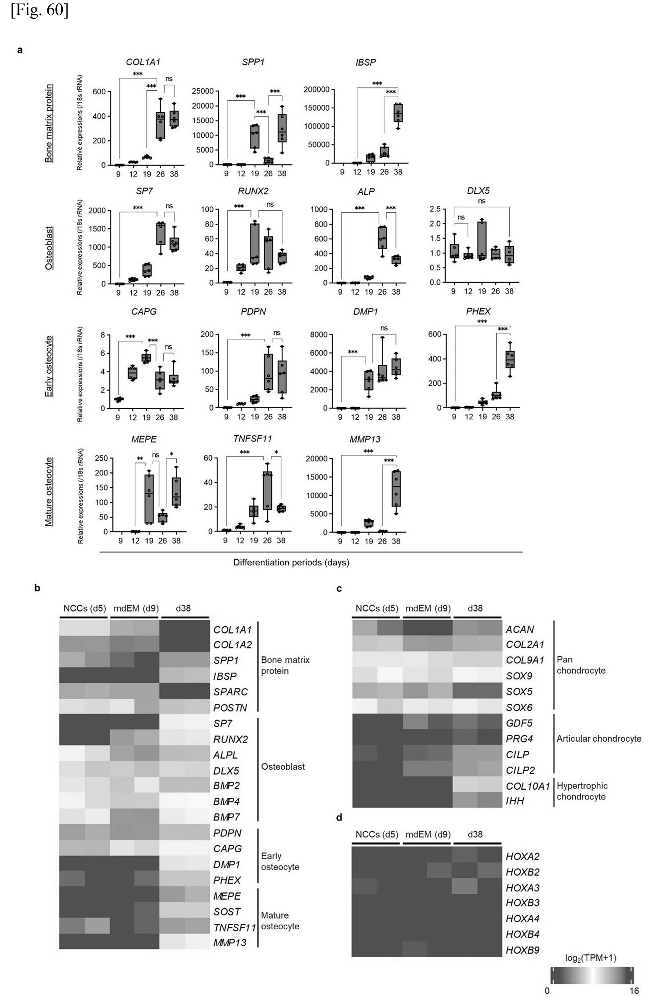

[FIG. 60] Directed differentiation of osteogenic lineage from mdEM. a, Gene expression analysis of bone matrix proteins (*COLIA1, OPN, IBSP*), osteoblast markers (*SP7, RUNX2, ALP, DLX5*), early osteocyte markers (*CAPG, PDPN, DMP1, PHEX*), and mature osteocyte markers (*MEPE, TNFSF11, MMP13*) by RT-qPCR. Gene expression levels were quantified using 18s rRNA as a reference control and compared among 1231A3 iPSC-derived mdEM (d9) and each time point of osteogenic induction. Data were normalized to d9 values. Values represent the mean ± s.e.m. (n = 6 from three biologically independent experiments). **P* < 0.05, ***P* < 0.01, ****P* < 0.001 by one-way ANOVA. ns, not significant. b-d, RNA-seq analysis of 1231A3 NCC aggregates (d5), mdEM (d9), and day 38 clusters (d38). Heatmap visualizing gene expression levels of bone matrix proteins, osteoblasts, early osteocytes, and mature osteocytes (b); chondrogenic markers (c); and HOX genes (d). log₂(TPM + 1) values were used to evaluate gene expression levels. (n = 2 from three biologically independent experiments).

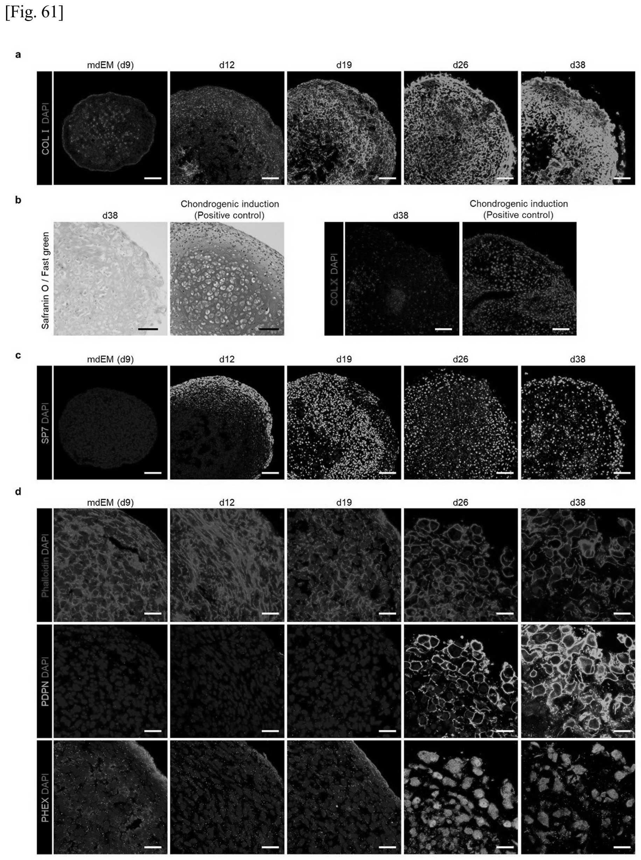

[FIG. 61] mdEM gradually produce bone matrices and differentiate into osteogenic cells. a, Immunofluorescence staining for COLI in 1231A3 iPSC-derived mdEM (d9) and at each time point of osteogenic induction. Nuclei stained with DAPI. (n = 3 from three biologically independent experiments). Scale bars, 100 µm. b, Safranin O/Fast green staining and immunofluorescence staining for COLX in mdEM and day 38 clusters. Nuclei stained with DAPI. Aggregates after chondrogenic induction served as positive controls for Safranin O and COLX expression. c, d, Immunofluorescence staining for SP7 (c), PDPN and PHEX (d) in mdEM and each point of osteogenic induction. F-actin stained with Phalloidin (d). Nuclei stained with DAPI. (n = 3 from three biologically independent experiments). a-d, Representative data are shown. Scale bars; 100 µm (a-c), 25 µm (d).

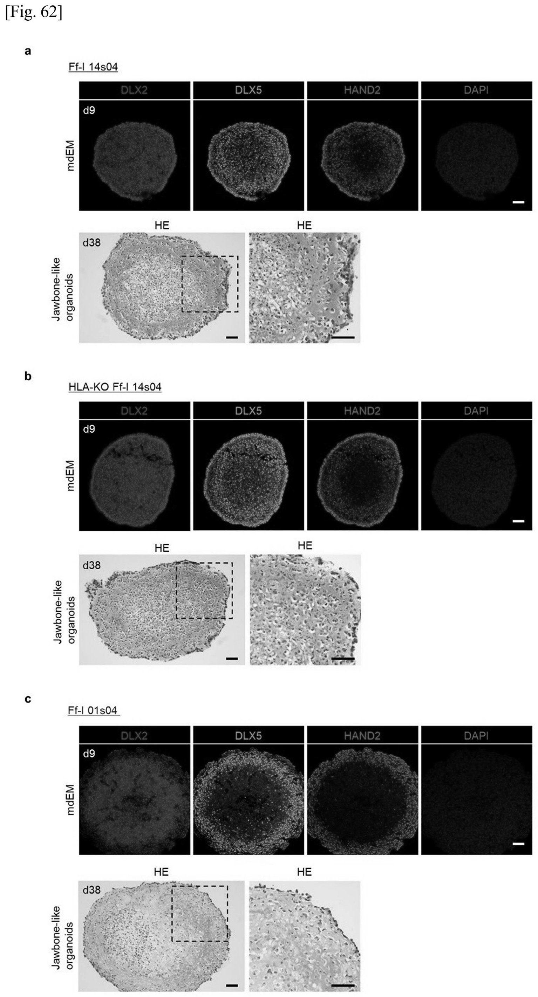

[FIG. 62] Generation of mdEM and jawbone-like organoids from several iPSC lines through mdEM. a-c, Triple-color immunofluorescence staining for DLX2, DLX5, and HAND2, and HE staining in mdEM and jawbone-like organoids generated from Ff-I 14s04 (a), HLA-KO Ff-I 14s04 (b), and Ff-I 01s04 (c) iPSCs. Nuclei stained with DAPI. (n = 3 from three biologically independent experiments). Dashed boxes magnified on the right. Representative data are shown. Scale bars, 100 µm.

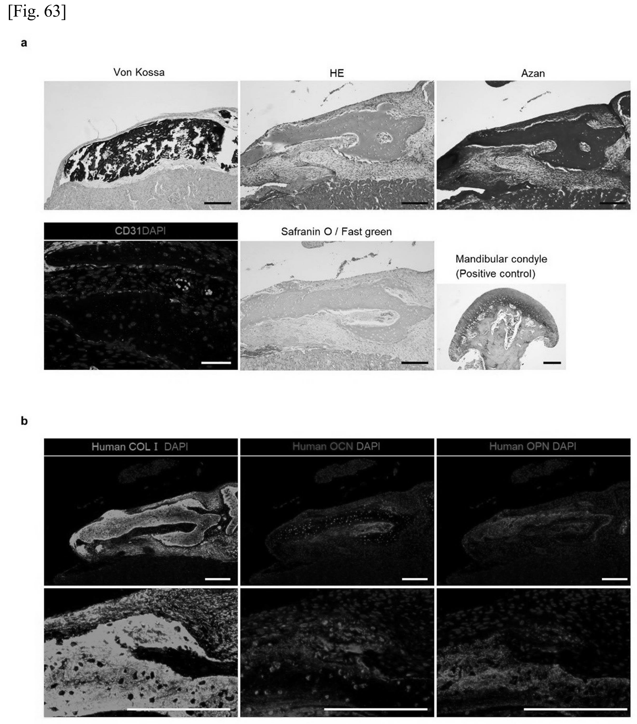

[FIG. 63] Jawbone-like organoids form bone tissues composed of human bone matrix proteins. a, Von kossa, HE, Azan, and Safranin O/Fast green staining and immunofluorescence staining for CD31 in bone-like tissues formed under NSG mouse renal capsule (n = 6). Nuclei stained with DAPI. Scale bars; 50 µm (CD31), 200 µm (the others). b, Triple-color immunofluorescence staining for human bone matrix proteins (human COLI, OCN, and OPN) in bone-like tissues. Nuclei stained with DAPI. (n = 6). a, b, Representative data are shown. Scale bars, 200 µm.

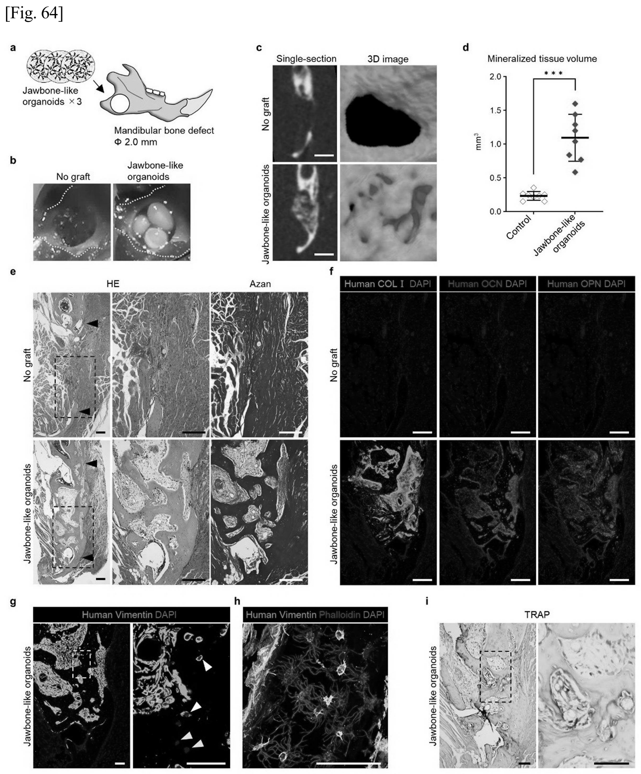

[FIG. 64] Transplantation of jawbone-like organoids promotes jawbone formation. a, Schematic representation of jawbone-like organoid (d38) transplantation into mandibular bone defects in NSG mice. b, Images depicting a mandibular bone defect without graft or with three jawbone-like organoids. Dotted lines denote the mandibular bone border. c, Single-section and 3D reconstructed micro-CT images of mandibular bone defects at 4 weeks post-transplantation. (n = 8). Scale bars, 1.0 mm. d, Quantification of newly formed mineralized tissue volume in defects at 4 weeks post-transplantation. (n = 8). ****P* < 0.001 by two-sided Student's t-test. e-g, HE (e), Azan (e), and triple-color immunofluorescence staining for human COLI, OCN, and OPN (f), and human Vimentin (g) in serial sections of transplanted tissues at 4 weeks post-transplantation (decalcified specimens). Nuclei stained with DAPI (f, g). (n = 8). Dashed boxes magnified on the right (e, g). Black arrowheads indicate the edge of the defects (e). White and light grey arrowheads indicate donor human cells and host mouse cells, respectively (g). Scale bars; 200 µm (e, f), 100 µm (g). h, Immunofluorescence staining for human Vimentin in transplanted tissues (decalcified specimens). Nuclei and F-actin stained with DAPI and Phalloidin, respectively. (n = 6). Images represent z-stack projection of 40 confocal optical sections (step size, 0.5 µm). Scale bar, 50 µm. i, Tartrate-resistant acid phosphatase (TRAP) staining in transplanted tissues. (n = 6). Scale bars, 100 µm. b, c, e-i, Representative data are shown.

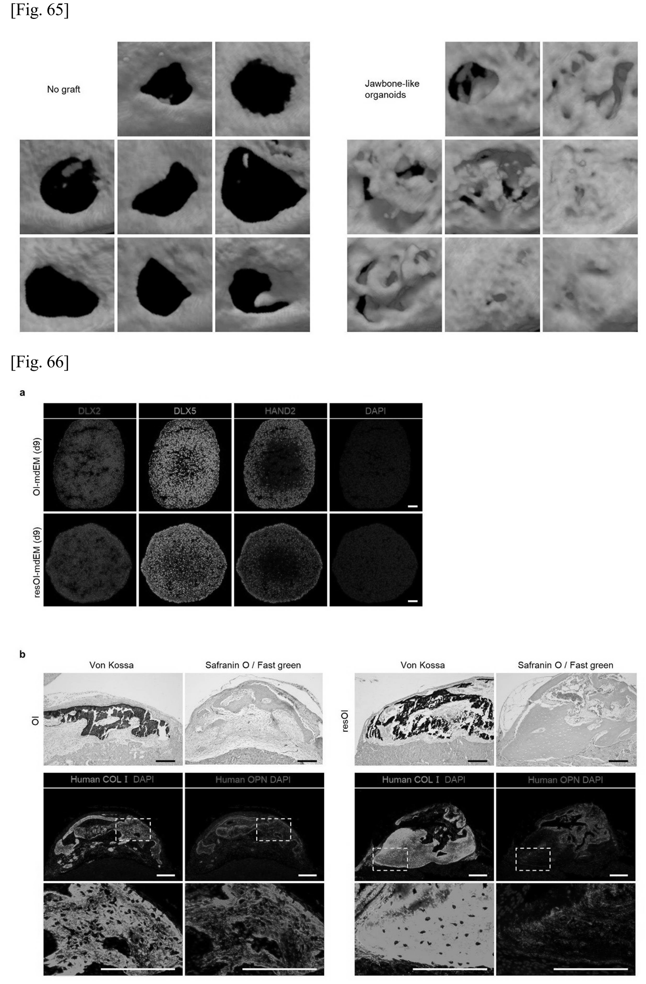

[FIG. 65] Jawbone regeneration by transplanted jawbone-like organoids. Individual micro-CT scanning images of jawbones with no graft (left panels) and with jawbone-like organoid transplantation (right panels). (n = 8/group).
[FIG. 66] Characterization of OI-iPSCs-derived mdEM and bone tissues formed under NSG mouse renal capsule. a, Triple-color immunofluorescence staining for DLX2, DLX5, and HAND2 in OI- and resOI-iPSC-derived mdEM (d9). Scale bars, 100 µm. b, Von Kossa and Safranin O/Fast green staining and immunofluorescence staining for human COLI and human OPN in OI- and resOI-iPSC-derived bone tissues formed under NSG mouse renal capsule. Nuclei stained with DAPI. (n = 3). Scale bars, 200 µm. a, b, Representative data are shown.

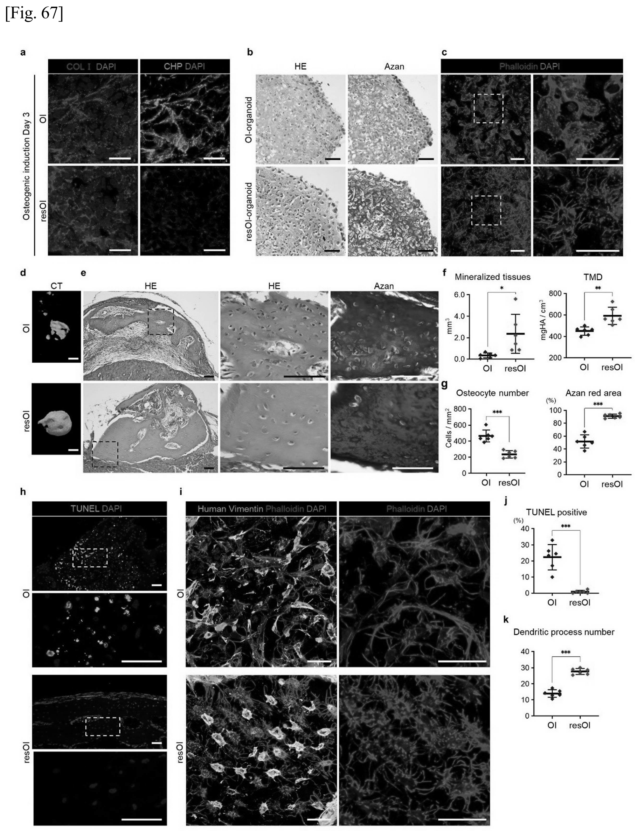

[FIG. 67] Recapitulation of disease phenotypes of osteogenesis imperfecta (OI). a, Immunofluorescence staining for COLI and collagen hybridizing peptide (CHP) in OI- and resOI-iPSC-derived aggregates after 3-day osteogenic induction. Nuclei stained with DAPI. (n = 6 from three biologically independent experiments). Scale bars, 25 µm. b, HE and Azan staining in serial sections of d38 OI- and resOI-jawbone-like organoids (decalcified specimens). (n = 6 from three biologically independent experiments). Scale bars, 100 µm. c, Phalloidin staining in OI- and resOI-organoids (decalcified specimens). Nuclei stained with DAPI. Images represent z-stack projection of 40 confocal optical sections (step size, 0.5 µm). Dashed boxes magnified on the right. (n = 6 from three biologically independent experiments). Scale bars, 25 µm. d, 3D reconstructed micro-CT images of ectopic mineralized tissues in the renal regions of NSG mice at 4 weeks after OI- or resOI-organoid transplantation. e, HE and Azan staining of the transplanted tissues (decalcified specimens). (n = 6 from three biologically independent experiments). Scale bars, 100 µm. f, Quantification of mineralized tissue volume and tissue mineral density (TMD) in OI- and resOI-organoid transplanted groups. g, Quantification of osteocytes and Azan-red stained areas. h, TUNEL staining of the transplanted tissues in OI- and resOI-organoid transplanted groups (decalcified specimens). (n = 6). Dashed boxes magnified below. Scale bars, 50 µm. i, Immunofluorescence staining for human Vimentin in the transplanted tissues (decalcified specimens). Nuclei and F-actin stained with DAPI and Phalloidin, respectively. Images represent z-stack projection of 40 confocal optical sections (step size, 0.5 µm). (n = 6). Scale bars, 25 µm. j, Quantification of TUNEL-positive apoptotic cells. k, Quantification analysis of osteocyte dendritic process number. f, g, j, k, **P <* 0.05, ***P <* 0.01, ****P* < 0.001 by two-sided Student's t-test. a-e, h, i, Representative data are shown.

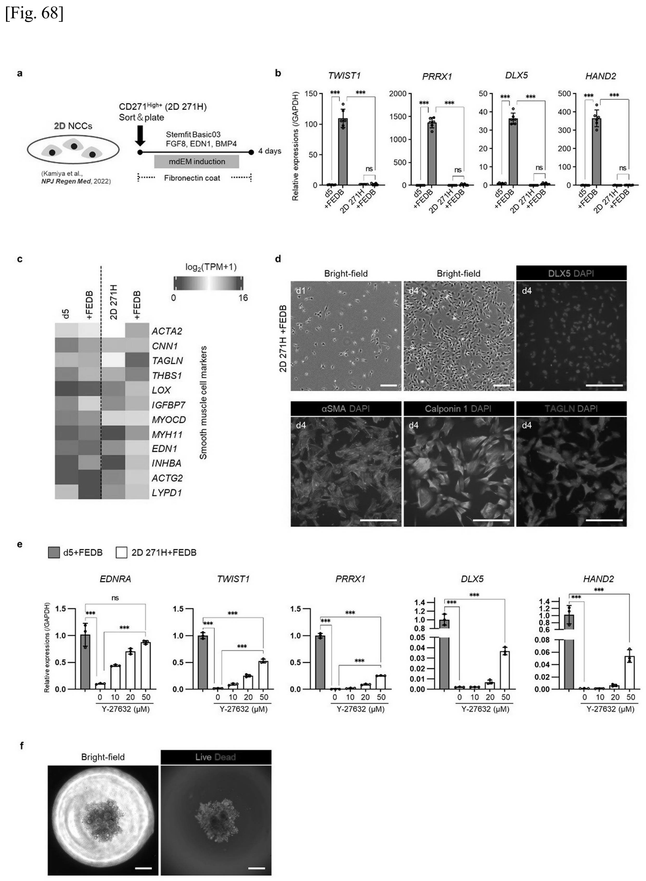

[FIG. 68] Differentiation from NCCs to mdEM. a, Schematic representation of the induction strategy for mdEM from 2D cultured NCCs. b, Gene expression analysis for early mesenchyme markers (*TWIST1, PRRX1*) and mdEM markers (*DLX5, HAND2*) by RT-qPCR. Gene expression levels quantified using GAPDH as a reference control and compared among 1231A3 iPSC-derived day 5 NCC aggregates (d5), d5 treated with FEDB (d5+FEDB), CD271^{high+} NCCs sorted from previous 2D protocols (2D 271H) (Non-Patent Literature 3), and 2D 271H treated with FEDB in 2D culture (2D 271H+FEDB). Data normalized to the d5 values. Values shown as mean ± s.e.m. (n = 6 from three biologically independent experiments). 2D 271H+FEDB hardly expressed both early mesenchyme markers and mdEM markers. c, RNA-seq analysis of d5, d5+FEDB, 2D 271H, and 2D 271H+FEDB. Heatmap visualizing the expression levels of smooth muscle cell-related genes. log₂(TPM + 1) values were used to evaluate gene expression levels. d, Bright-field images and immunocytochemistry for DLX5, αSMA, Calponin 1, TAGLN in 2D 271H+FEDB at day 1 or 4 after plating. Nuclei stained with DAPI. 2D 271H+FEDB expressed smooth muscle cell markers. (n = 3 from three biologically independent experiments). Representative data are shown. Scale bars, 100 µm. e, Gene expression analysis for EDNRA and early pharyngeal arch mesenchyme markers (*TWIST1, PRRX1*) and mdEM markers (*DLX5, HAND2*) by RT-qPCR. Gene expression levels quantified using GAPDH as a reference control and compared among 2D 271H treated with FEDB (+ 0, 10, 20, 50 ng/ml Y-27632) in 2D culture. Data normalized to the values of d5+FEDB. Results shown as mean ± s.e.m. (n = 3 from three biologically independent experiments). Inhibition of actin-stress fiber formation by Y-27632 treatment considerably rescued the expression of mdEM markers in 2D cultured cells, though not as much as 3D culture. f, Bright-field imaging and cell viability analysis. 2D 271H aggregated in FEDB medium in 96-well V-bottom ultra-low-cell-adhesion plates to promote sphere formation. After 4 days, cell viability in cell spheroids analyzed using LIVE/DEAD Viability/Cytotoxicity kit. Spheroid culture of NCCs under FEDB induced serious cell apoptosis. (n = 3 from three biologically independent experiments). b, e, ****P* < 0.001 by one-way ANOVA. ns, not significant. d, f, Representative data are shown. Scale bars, 200 µm.

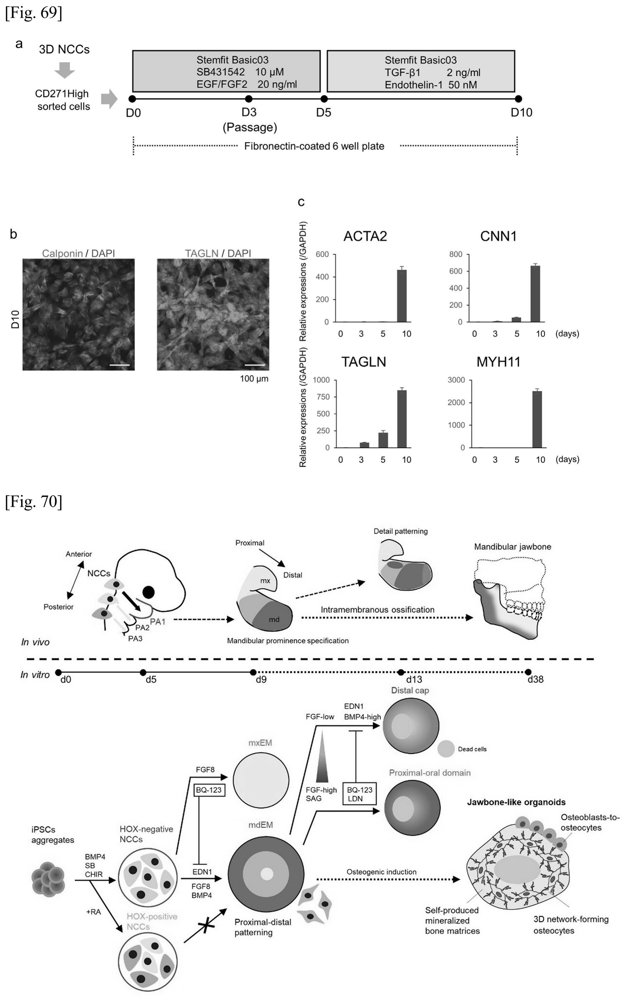

[FIG. 69] Induction of smooth muscle cells from NCC aggregates. a; Schematic diagram of the induction method. b; Immunohistochemical results for smooth muscle markers. Nuclei were stained with DAPI. High expression of Calponin and TAGLN was confirmed 10 days after smooth muscle cell induction. c; Gene expression analysis of smooth muscle markers via RT-qPCR. High expression of smooth muscle markers (ACTA2, CNN1, TAGLN) and mature smooth muscle marker (MYH11) was confirmed 10 days after smooth muscle cell induction.
[FIG. 70] Schematic model of jawbone-like organoids generation from human iPSCs via mdEM. mdEM and jawbone-like organoids were generated from human iPSCs in a stepwise manner, guided by signaling environments during embryogenesis, as depicted in the diagram. Initial SB/BMP4 treatment for one day, followed by SB/CHIR treatment, effectively induced HOX-negative NCC aggregates from iPSC aggregates (day 0-5). Transient RA treatment induced HOX-positive posterior NCC aggregates. From days 5-9, the combination of FGF8, EDN1, and BMP4 directed HOX-negative NCC aggregates to mdEM, recapitulating the proximal-distal patterning of embryonic mdEM. The regional identity of mdEM or mxEM was modulated by adjusting EDN1 signal activity. HOX-positive NCC aggregates did not differentiate into mdEM, underscoring the importance of initial NCC positional identity. mdEM exhibited later-stage regional patterning in response to exogenous mandibular epithelial signals (FGF8, SAG, EDN1, BMP4), bifurcating into distal cap and proximal-oral molar domain. From days 9-38, under osteogenic conditions, mdEM formed jawbone-like organoids consisting of osteoblasts and network-forming osteocytes embedded in self-produced mineralized bone matrices. PA1-3, pharyngeal arch 1-3; mx, maxillary prominence; mxEM, mx ectomesenchyme; md, mandibular prominence; mdEM, md ectomesenchyme; SB, SB431542 (TGF-β inhibitor); CHIR, CHIR99021 (GSK3b inhibitor); RA, retinoic acid; BQ-123 (Endothelin Receptor Type A (EDNRA) antagonist); LDN, LDN193189 (BMP inhibitor); SAG (Hedgehog signaling agonist).

### DESCRIPTION OF EMBODIMENTS

1. Method for Producing Neural Crest Cells

The present invention provides a method for producing neural crest cells (NCCs) from pluripotent stem cells. Specifically, the method includes
(1) a step of conducting suspension culture of pluripotent stem cells in a culture medium containing an ALK inhibitor and a bone morphogenetic protein; and
(2) a step of conducting suspension culture in a culture medium containing an ALK inhibitor and a GSK-3β inhibitor (this method may also be referred to as an "NCC production method of the present invention" hereinafter).

As used herein, the term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and especially human iPS cells) are preferred. When the pluripotent stem cells are ES cells or arbitrary cells derived from human embryos, the cells may be obtained by destruction of the embryo or they may be obtained without destruction of the embryo, but from an ethical point of view, preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines distributed by ESI Bio Co., H1 and H9 lines distributed by WiCell Research, KhES-1, KhES-2, KhES-3, KhES-4, KhES-5, SSES1, SSES2 and SSES3 distributed by Riken, and so on.

iPS cells are cells obtained by reprogramming of mammalian somatic cells or undifferentiated stem cells by introduction of specific factors (nuclear reprogramming factors). A large number of iPS cells currently exist, among which there may be used human iPSCs established by introduction of the 4 factors Oct3/4·Sox2·Klf4·c-Myc into human fibroblasts by Yamanaka (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y., Ding S., et al., Cell Stem Cell, (2008) Vol.3, Issue 5, 568-574, Kim JB., Scholer HR., et al., Nature, (2008) 454, 646-650; Huangfu D., Melton, DA., et al., Nature Biotechnology, (2008) 26, No 7, 795-797), or patent publications (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, etc., by Riken, 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line, etc., by Kyoto University, with the 1231A3 line being preferred.

The induced pluripotent stem cells used in the method for producing NCCs of the present invention may be cells derived from patients with hereditary diseases (for example, patients with osteogenesis imperfecta). Since cells obtained through induction of differentiation of pluripotent stem cells derived from patients with hereditary diseases can serve as disease models that reflect the pathology of such diseases, the cells are suitable for applications such as screening of therapeutic or preventive medicaments for the diseases. Alternatively, it is also possible to use the cells as therapeutic medicament for the diseases by repairing the gene through genome editing, such as the CRISPR-Cas system, in pluripotent stem cells derived from patients with hereditary diseases, and then differentiating them into desired cells or organoids. In fact, in the Examples described below, it was shown that in bone organoids induced from pluripotent stem cells in which a point mutation in COLIA1, which causes bone formation abnormality, was rescued, no bone formation abnormality was observed.

mGS cells are pluripotent stem cells derived from the testis and are the source cells for spermatogenesis. Like ES cells, differentiation of these cells into cells of various lineages can be induced, and the cells have properties such as the ability to produce chimeric mice when transplanted into mouse blastocysts (Kanatsu-Shinohara M. et al. (2003) Biol. Reprod., 69:612-616; Shinohara K. et al. (2004), Cell, 119:1001-1012). These cells are capable of auto-replication in a culture solution containing glial cell line-derived neurotrophic factor (GDNF), and by repeated passage under the same culture conditions as those for ES cells, germline stem cells can be obtained (Takebayashi, M. et al. (2008), Jikken Igaku, Vol.26, No.5 (special edition), pp.41-46, Yodosha Co., Ltd. (Tokyo, Japan)).

EG cells are established from the primordial germ cells in the embryonic period and have pluripotency similar to that of ES cells. They can be established by culturing primordial germ cells in the presence of substances such as LIF, bFGF, and stem cell factor (Matsui Y. et al. (1992), Cell, 70:841-847; J. L. Resnick et al. (1992), Nature, 359:550-551).

The source species of the pluripotent stem cells is not particularly restricted, and for example, the cells may be from a rodent such as a rat, mouse, hamster or guinea pig, a lagomorph such as a rabbit, an ungulate such as a pig, cow, goat or sheep, a carnivora such as a dog or a cat, or a primate such as a human, monkey, rhesus monkey, marmoset, orangutan or chimpanzee. The preferred source species is human.

In this specification, a "cell aggregate" means a structure composed of a mass formed by aggregated cells, and is also called a cell agglomerate. A "neural crest cell (NCC) aggregate" means a cell aggregate containing neural crest cells, and typically means a cell aggregate containing neural crest cells as a main component (e.g., at a ratio of 60% or more, 70% or more, or 80% or more). The same applies to cell aggregates of other cell strains (e.g., a first pharyngeal arch ectomesenchyme (PA1-EM) cell aggregate).

In this specification, an "ALK inhibitor" means a substance having inhibitory activity against a receptor belonging to the ALK (activin receptor-like kinase) family. The ALK is also called a "type-I TGFβ receptor" and regulates cell proliferation, cell differentiation, cell death, and the like via signaling for mainly activating Smad (R-Smad). Type-II TGFβ receptors form a dimer, and the dimer associate with two molecules of the type-I TGFβ receptor to form a heterotetramer. Due to the formation of the heterotetramer, the type-II TGFβ receptor phosphorylates the type-I TGFβ receptor. This phosphorylation induces kinase activity of the type-I TGFβ receptor, and thus Smad on the downstream side is phosphorylated.

The "ALK inhibitor" used in the present invention is not particularly limited as long as it can inhibit any stage of the above-described signaling, and examples thereof include a substance that inhibits binding of TGFβ to its receptor, a substance that inhibits phosphorylation of the type-I TGFβ receptor by the type-II TGFβ receptor, a substance that inhibits phosphorylation of Smad (e.g., Smad2, Smad3, or the like) by the phosphorylated type-I TGFβ receptor, and the like. As human ALKs, ALK-1, ALK-2, ALK-3, ALK-4, ALK-5, ALK-6, and ALK-7 are known, and an inhibitor (also referred to as a "TGFβ inhibitor") particularly against ALK-5 is preferable as the ALK inhibitor used in the present invention.

Examples of the ALK inhibitor used in step (1) or (2) of the NCC production method of the present invention include SB431542 (4-(5-benzol[1,3]dioxol-5-yl-4-pyridin-2-yl-1H-imidazol-2-yl)-benzamide, 4-[4-(1,3-benzodioxol-5-yl)-5-(2-pyridinyl)-1H-imidazol-2-yl]-benzamide, 4-[4-(3,4-methylenedioxyphenyl)-5-(2-pyridyl)-1H-imidazol-2-yl]-benzamide), A-83-01 (3-(6-methylpyridin-2-yl)-1-phenylthiocarbamoyl-4-quinolin-4-ylpyrazole), LDN193189 (4-[6-[4-(1-piperazinyl)phenyl]pyrazolo[1,5-a]pyrimidin-3-yl]-quinoline), GW788388 (4-[4-[3-(pyridin-2-yl)-1H-pyrazol-4-yl]-pyridin-2-yl]-N-(tetrahydro-2H-pyran-4-yl)benzamide), SM16 (4-[4-(1,3-benzodioxol-5-yl)-5-(6-methyl-2-pyridinyl)-1H-imidazol-2-yl]-bicyclo[2.2.2]octane-1-carboxamide), IN-1130 (3-[[5-(6-methyl-2-pyridinyl)-4-(6-quinoxalinyl)-1H-imidazol-2-yl]methyl]-benzamide), GW6604 (2-phenyl-4-[3-(pyridin-2-yl)-1H-pyrazol-4-yl]pyridine), SB505124 (2-(5-benzo[1,3]dioxol-5-yl-2-tert-butyl-3H-imidazol-4-yl)-6-methylpyridine), and the like. Among these inhibitors, SB431542 and A-83-01 are preferable. Two or more types of ALK inhibitors may be used in combination.

In step (1) or (2) of the NCC production method of the present invention, the concentration of the ALK inhibitor in the culture medium is adjusted as appropriate depending on the type of ALK inhibitor added, and is typically 0.1 to 50 µM, preferably 1 to 40 µM, and more preferably 5 to 20 µM. When SB431542 is used, the concentration thereof in the culture medium is typically 1 to 40 µM, preferably 5 to 20 µM, and more preferably 10 µM. When A-83-01 is used, the concentration thereof in the culture medium is typically 0.01 to 10 µM, preferably 0.1 to 4 µM, more preferably 0.5 to 2 µM, and even more preferably 1 µM.

A bone morphogenetic protein (BMP) is a secretory signaling molecule belonging to the TGF-β superfamily. Examples of the bone morphogenetic protein used in step (1) of the NCC production method of the present invention include BMP2, BMP3, BMP 3b, BMP4, BMP5, BMP6, BMP7, BMP8, BMP9, BMP10, BMP11, BMP12, BMP13, BMP14, BMP15, BMP16, BMP17, BMP18, and the like. Among these bone morphogenetic proteins, BMP4, BMP2, and BMP7 are preferable. Two or more types of BMPs may be used in combination.

In step (1) of the NCC production method of the present invention, the concentration of the BMP in the culture medium is adjusted as appropriate depending on the type of BMP added, and is typically 1 to 50 ng/mL, preferably 2 to 30 ng/mL, and more preferably 2.5 to 10 ng/mL.

In this specification, the term "GSK-3β inhibitor" means a substance having inhibitory activity against GSK-3β (glycogen synthase kinase 3β). GSK3 (glycogen synthase kinase 3) is one type of serine/threonine protein kinases and is involved in many signaling pathways for glycogen production, apoptosis, stem cell maintenance, and the like. GSK3 has two isoforms, α and β. The "GSK-3β inhibitor" used in the present invention is not particularly limited as long as it has inhibitory activity against GSK-3β, and it may be a substance that has inhibitory activity against GSK-3α as well as the inhibitory activity against GSK-3β.

Examples of the GSK-3β inhibitor used in step (2) of the NCC production method of the present invention include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), CP21R7 (3-(3-amino-phenyl)-4-(1-methyl-1H-indol-3-yl)-pyrrole-2,5-dione), LY2090314 (3-[9-fluoro-1,2,3,4-tetrahydro-2-(1-piperidinylcarbonyl)pyrrolo[3,2,1-jk][1,4]benzodiazepin-7-yl]-4-imidazo[1,2-a]pyridin-3-yl-1h-pyrrole-2,5-dione), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB415286 (3-[(3-chloro-4-hydroxyphenyl)amino]-4-(2-nitrophenyl)-1H-pyrrole-2,5-dione), AR-AO144-18 (1-[(4-methoxyphenyl)methyl]-3-(5-nitro-1,3-thiazol-2-yl)urea), CT99021, CT20026, BIO ((2'Z,3'E)-6-bromoindirubin-3'-oxime), BIO-acetoxime, a pyridocarbazol-cyclopentadienylruthenium complex, OTDZT, α-4-dibromoacetophenone, lithium, and the like. Two or more types of these inhibitors may be used in combination. An antisense oligonucleotide and an siRNA for mRNA of GSK-3β, an antibody capable of binding to GSK-3β, a dominant negative mutant of GSK-3β, and the like can also be used as the GSK-3β inhibitor, and these are commercially available or can be synthesized in accordance with known methods.

In step (2) of the NCC production method of the present invention, the concentration of the GSK-3β inhibitor in the culture medium is adjusted as appropriate depending on the type of the GSK-3β inhibitor added, and is, for example, 0.01 to 20 µM, and preferably 0.1 to 10 µM. For example, when CHIR99021 is used, the concentration thereof is typically 0.1 to 10 µM, preferably 0.5 to 2 µM, and more preferably 1 µM.

As shown in the examples below, it was shown that the culture period can be shortened due to step (1) of the NCC production method of the present invention including a step of conducting shaking culture (e.g., FIG. 20), and the NCC induction efficiency can be improved due to step (2) including a step of conducting shaking culture (FIG. 9). Accordingly, it is preferable that at least one step of steps (1) and (2) above includes a step of conducting shaking culture. The shaking culture may be conducted over the entire period of each step, or for only a part of the period.

The shaking culture can be conducted using a shaking apparatus (shaker). The culture apparatus may be a swirling (orbital) shaking apparatus, a reciprocating shaking apparatus, a figure-of-eight shaking apparatus, or a shaking apparatus having the function of appropriately switching these shaking methods. The shaking direction may be a horizontal direction or a vertical direction, and is typically a horizontal direction. The shaking speed is not particularly limited and is usually set to be within a range of 50 to 450 rpm and preferably a range of 100 to 200 rpm (120 rpm in an aspect).

Without wishing to be bound by any theory, it is assumed that one of the reasons why the NCC induction efficiency is improved by shaking culture is that the infiltration of culture medium supplements into the cell aggregate is promoted through shaking. Accordingly, it is assumed that the NCC induction efficiency is also improved by other methods capable of achieving such an object, such as stirred culture. Therefore, the stirred culture may be conducted instead of the shaking culture or in combination with the shaking culture. The stirred culture can be conducted using a culture apparatus (e.g., bio reactor) provided with a stirring bar or stirring blade. The rotation speed of the stirring bar or stirring blade during the stirred culture is typically 10 to 100 rpm.

The culture period in step (1) of the NCC production method of the present invention is typically 3 hours to 7 days, and is preferably 6 hours to 6 days, more preferably 6 hours to 5 days, even more preferably 12 hours to 4 days, and even more preferably 1 day to 3 days, from the viewpoint of the NCC induction efficiency. When the shaking culture and/or the stirred culture is conducted, the period of step (1) can also be shortened, and the culture period can be set to, for example, 6 hours to 3 days, 6 hours to 2 days, 12 hours to 1 day, or the like.

The culture period in step (2) of the NCC production method of the present invention is typically 12 hours to 5 days, and is preferably 1 day to 6 days, more preferably 1 day to 4 days, even more preferably 2 days to 3 days, and even more preferably 1 day to 3 days, from the viewpoint of the NCC induction efficiency. The period above can be changed as appropriate according to the period of step (1) of the NCC production method of the present invention, presence or absence of the shaking culture and/or the stirred culture, and the like.

The culture period in steps (1) and (2) (total culture period in steps (1) and (2)) of the NCC production method of the present invention is typically 1 day to 6 days, preferably 2 days to 5 days, and more preferably 3 days to 4 days. The period above can be changed as appropriate according to the period of step (1) of the NCC production method of the present invention, presence or absence of the shaking culture and/or the stirred culture, and the like.

A step of conducting suspension culture of pluripotent stem cells in a culture medium containing a ROCK inhibitor may be conducted prior to step (1) of the NCC production method of the present invention. It is thus possible to form a cell aggregate composed of the pluripotent stem cells. Instead of this step, a step of preparing a cell aggregate composed of pluripotent stem cells may be conducted. The period of this step is not particularly limited, and is typically 12 hours to 3 days and preferably 1 day. Accordingly, when the NCC production method of the present invention includes the step of forming a cell aggregate composed of pluripotent stem cells, the period of the NCC production method of the present invention is typically 2 days to 7 days, preferably 3 days to 6 days, and more preferably 4 days to 5 days.

The ROCK inhibitor used in the present invention is not particularly limited as long as it can suppress the functions of a Rho-kinase (ROCK), and examples thereof include Y-27632 (see, for example, Ishizaki et al., Mol. Pharmacol. 57, 976-983 (2000); Narumiya et al., Methods Enzymol. 325, 273-284 (2000)), fasudil/HA1077 (see, for example, Uenata et al., Nature 389: 990-994 (1997)), SR3677 (see, for example, Feng Y et al., J Med Chem. 51: 6642-6645(2008)), GSK269962 (see, for example, Stavenger RA et al., J Med Chem. 50: 2-5 (2007) or WO2005/037197), GSK429286A, H1152 (see, for example, Sasaki et al., Pharmacol. Ther. 93: 225-232 (2002)), Wf-536 (see, for example, Nakajima et al., Cancer Chemother Pharmacol. 52(4): 319-324 (2003)), thiazovivin and salts or derivatives thereof, and the like. Other examples of the ROCK inhibitor include an antisense nucleic acid for ROCK, an RNA interference inducing nucleic acid (e.g., siRNA), a dominant negative mutant, and expression vectors thereof. Other known low-molecular-weight compounds and salts or derivatives thereof can also be used as the ROCK inhibitor (see, for example, U.S. Publication No. 2005/0209261, U.S. Publication No. 2005/0192304, U.S. Publication No. 2004/0014755, U.S. Publication No. 2004/0002508, U.S. Publication No. 2004/0002507, U.S. Publication No. 2003/0125344, U.S. Publication No. 2003/0087919, WO 2003/062227, WO 2003/059913, WO 2003/062225, WO 2002/076976, and WO 2004/039796). Among these inhibitors, Y-27632 is preferable. Only one type or two or more types of ROCK inhibitors may be used. The concentration of the ROCK inhibitor in the culture medium can be selected as appropriate by a person skilled in the art in accordance with the type of ROCK inhibitor used. For example, when Y-27632 or a salt thereof (e.g., Y-27632 dihydrochloride) is used, the concentration is typically 0.1 µM to 100 µM, preferably 1 µM to 50 µM, more preferably 5 µM to 20 µM, and even more preferably 10 µM.

NCCs may be sorted (i.e., isolated, concentrated, or purified) from an NCC aggregate obtained in step (2) of the NCC production method of the present invention, using the expression of the NCC marker (e.g., high CD271 expression) as an index. Known methods can be used as appropriate to sort NCCs, and examples thereof include a method in which cells are labeled with an antibody against an NCC marker such as CD271 and flow cytometry or mass cytometry is used, a magnetic cell sorting method, a method in which cells are sorted using an affinity column or the like to which a desired antibody is immobilized, and the like.

The culture density of cells in the NCC production method of the present invention is not particularly limited as long as the cells can be proliferated. The culture density is typically 1.0×10¹ to 1.0×10⁹ cells/ml, preferably 1.0×10² to 1.0×10⁸ cells/ml, more preferably 1.0×10³ to 1.0×10⁷ cells/ml, and even more preferably 1.0×10³ to 1.0×10⁶ cells/ml (e.g., 2.0×10⁴ cells/ml, 3.0×10⁴ cells/ml, 1.0×10⁵ cells/ml, and the like).

In at least one of step (1) and step (2) of the NCC production method of the present invention, cells may be cultured under feeder-free conditions and/or xeno-free conditions. All the steps of the NCC production method of the present invention may be conducted under feeder-free conditions and xeno-free conditions. Also, in any steps other than steps (1) and (2) of the NCC production method of the present invention, cells may be cultured under feeder-free conditions and/or xeno-free conditions. In this specification, the term "feeder-free" means a culture medium or culturing conditions free of auxiliary cells (i.e., feeder cells) that are used to adjust culturing conditions for the cells to be cultured. Also, the term "xeno-free" means a culture medium or culturing conditions free of components derived from organisms that are different from the biological species from which cells to be cultured are derived.

A basal culture medium used in the NCC production method of the present invention is not particularly limited, but examples thereof include StemFit (registered trademark) AK02 culture medium (Ajinomoto Co., Inc.), StemFit (registered trademark) AK03 culture medium (Ajinomoto Co., Inc.), StemFit (registered trademark) Basic03 culture medium, CTS (registered trademark) KnockOut SR XenoFree Medium (Gibco), mTeSR1 culture medium, TeSR1 culture medium (Stem Cell Technologies), Iscove's modified Dulbecco's medium (GE HealthCare Technologies Inc.), Improved MEM (Thermo Fisher Scientific Inc.), and the like. Among these culture media, StemFit (registered trademark) AK03 culture medium is preferable. These culture media can also be used for culture under feeder-free and xeno-free conditions. Examples of the basal culture medium include RPMI-1640 culture medium, EagleMEM (EMEM), Dulbecco's Modified MEM, Glasgow's MEM (GMEM), α-MEM, 199 culture medium, IMDM, DMEM, Hybridoma Serum free culture medium, KnockOut (trademark) DMEM, Advanced TM culture medium (e.g., Advanced MEM, Advanced RPMI, and Advanced DMEM/F-12), Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL culture medium (e.g., CMRL-1066), Williams' medium E, Brinster's BMOC-3 Medium, E8 Medium, StemPro 34, and MesenPRO RS (these are available from Thermo Fisher Scientific Inc.); ReproFF2, Primate ES Cell Medium, and ReproStem (these are available from REPROCELL Inc.); ProculAD (available from ROHTO Pharmaceutical Co., Ltd.); MSCBM-CD and MSCGM-CD (these are available from Lonza); EX-CELL302 culture medium (available from SAFC) or EX-CELL-CD-CHO (available from SAFC); ReproMed (trademark) iPSC Medium (available from REPROCELL Inc.); mixtures thereof; and the like, but there is no limitation thereto.

As necessary, the culture medium may include, for example, one or more serum alternatives such as Knockout Serum Replacement (KSR), N2 Supplement (Invitrogen), B27 Supplement (Invitrogen), albumin, transferrin, apotransferrin, a fatty acid, insulin, a collagen precursor, a trace element, 2-mercaptoethanol, and 3'-thiolglycerol, and moreover, it may also contain one or more substances selected from a lipid, an amino acid, L-glutamine, Glutamax (Invitrogen), a nonessential amino acid, a vitamin, a growth factor, a low-molecular-weight compound, an antibiotic, an antioxidant, pyruvic acid, a buffer, an inorganic salt, selenic acid, progesterone, putrescine, and the like.

In this specification, the term "suspension culture" means culture conducted under conditions that maintain a state in which cells or cell aggregates float in a culture solution, namely culture conducted under conditions that prevent a firm cell-substratum junction from being formed between a cell or a cell aggregate and the culture vessel. When suspension culture is conducted, cells before and after the suspension culture are typically in the form of a cell aggregate.

The culture vessel to be used for suspension culture is not particularly limited as long as "suspension culture" can be conducted using it, and a person skilled in the art could select one as appropriate. Examples of such a culture vessel include a flask, a tissue culture flask, a dish, a petri dish, a tissue culture dish, a multi-dish, a microplate, a microwell plate, a micropore, a multi-plate, a multi-well plate, a chamber slide, a laboratory dish, a tube, a tray, a culture bag, a roller bottle, and the like. Furthermore, a bioreactor is exemplified as a vessel for suspension culture. These culture vessels are preferably cell non-attachable vessels in order to enable suspension culture. As the cell non-attachable culture vessel, a culture vessel whose surface is not artificially treated (e.g., not coated with extracellular matrix or the like) for the purpose of improving the adhesion to the cells, and the like can be used. The bottom shape of wells of these culture vessels is not particularly limited, and examples thereof include a flat shape, a U-shape, and a V-shape. In step (1) of the NCC production method of the present invention, it is preferable to use a culture vessel having a well with a V-shaped bottom (e.g., 96-well V-bottom ultra-low adhesion culture plate (manufactured by Sumitomo Bakelite Co., Ltd.)). In step (2) of the NCC production method of the present invention, it is preferable to use a culture vessel having a well with a flat bottom (e.g., 48-well low adhesion culture plate (manufactured by Iwaki)).

The culture temperature is not particularly limited, but is about 30°C to 40°C and preferably about 37°C. The culture is conducted under a CO₂-containing air atmosphere, and the CO₂ concentration is preferably about 2% to 5%.

Embryologically, NCCs develop between a neuroectoderm and a surface ectoderm during the formation of a neural tube from neural plates in early development, and have multipotency, which is a capacity to differentiate into a large variety of cells such as nerve cells, glial cells, mesenchymal stem cells, osteocytes, chondrocytes, and melanocytes, and self-proliferation capacity. In this specification, the "neural crest cell (NCC)" is a cell that expresses one or more genes selected from CD271 (NGFR), SOX10, TFAP2A, and PAX3 (desirably all of the four genes). Typically, the NCC is strongly positive for CD271.

NCCs derived from pluripotent stem cells are obtained through step (2) of the NCC production method of the present invention. Therefore, in another aspect of the present invention, also provided are NCCs (which may be referred to as "NCCs of the present invention" hereinafter) that are obtainable (or obtained) by the NCC production method of the present invention. The NCCs of the present invention typically have all of the following features (A) to (C).
(A) derived from pluripotent stem cells
(B) expressing one or more (preferably all) genes selected from CD271, SOX10, TFAP2A, and PAX3
(C) having a capacity to differentiate into PA1-EM cells (at least mandibular PA1-EM cells)

The NCCs of the present invention further has at least one (preferably all) of the following features (D) to (F).
(D) having a capacity to differentiate into melanoblasts
(E) having a capacity to differentiate into nerve cells
(F) having a capacity to differentiate into mesenchymal stem cells

In another aspect, the present invention also provides NCCs that have all of the features (A) to (C) above and preferably have at least one (preferably all) of the features (D) to (F) above. Such NCCs may be obtainable by the NCC production method of the present invention or may be obtainable by another method. Hereinafter, the above-mentioned NCCs may also be referred to as "NCCs of the present invention".

When the wording ""express" a gene" or ""positive for" a gene" is used in this specification, it implicates at least "production of mRNA encoded by the gene" unless otherwise stated, and preferably further implicates "production of a protein encoded by the mRNA". Therefore, when the production of mRNA encoded by a gene is detected through at least a method (RT-qPCR) described in Examples below, it conceivable that the cells express the gene. Meanwhile, when the production of mRNA encoded by a gene is not detected through a method (RT-qPCR) described in Examples below (i.e., the production level is below the detection limits), is at the background level, or is one hundredth or less of that of a positive control, it is conceivable that the cells do not express the gene or are negative.

In this specification, the term "positive" encompasses a case of having a high expression ability (also referred to as "being strongly positive"). Having a high expression ability is sometimes expressed as "High" or "Bright". For example, CD271-high expression cells may be expressed as "CD271^{High+} cells" or CD271^{Bright+} cells. The high expression ability can be determined based on a chart obtainable through flow cytometry. The positions on the chart may fluctuate depending on the voltage setting of the apparatus, the sensitivity setting, an antibody clone used, the staining conditions, a pigment used, and the like, but a person skilled in the art can draw a line as appropriate so as not to divide a cell population recognized as one group on the obtained chart.

The wording "having a capacity to differentiate into various cells (cells of interest) such as PA1-EM cells" means that the cells of interest are obtainable from the NCCs using any method. The PA1-EM cells are classified into maxillary PA1-EM (mxEM) and mandibular PA1-EM (mdEM), but in this specification, PA1-EM encompasses both the maxillary PA1-EM and the mandibular PA1-EM unless otherwise stated, and particularly the mandibular PA1-EM is intended in many cases.

### 2. Differentiation Induction Method, and Differentiated Cells or Organoids Obtainable by the Method

As described above, the NCCs of the present invention have a capacity to differentiate into various cells. Therefore, in another aspect, provided are a method for producing differentiated cells or organoids that includes a step of inducing differentiation of NCCs of the present invention (this method may be referred to as the "differentiation induction method of the present invention" hereinafter), and differentiated cells or organoids that are obtainable (or obtained) by the method.

Cells obtainable using the differentiation induction method of the present invention may be undifferentiated cells such as stem cells and progenitor cells, or terminally differentiated cells. In the description below, a term "differentiated cell" may be used as a term that encompasses both an undifferentiated cell and a terminally differentiated cell. In this specification, the term "undifferentiated cells" means cells that do not reach terminal differentiation in the cell lineage, and examples of the undifferentiated cells include stem cells other than pluripotent stem cells, and progenitor cells. Examples of the stem cells or progenitor cells include neural stem cells, glial progenitor cells, pigment stem cells, melanoblasts, mesenchymal stem cells, first pharyngeal arch ectomesenchyme cells, cardiac muscle stem cells, vascular endothelial progenitor cells, vascular pericytes, skeletal muscle stem cells, fat stem cells, osteoprogenitor cells, osteoblasts, chondroblasts, and the like.

In this specification, the term "terminally differentiated cells" means cells that have reached terminal differentiation in the cell lineage. The terminally differentiated cells are not particularly limited, and examples thereof include nerve cells, glial cells, melanocytes, adipocytes, corneal endothelial cells, vascular endothelial cells, cardiac muscle cells, skeletal muscle cells, osteocytes, chondrocytes, smooth muscle cells, and the like.

In this specification, the term "organoid" means a structure containing a plurality of types of cells, and the organoid typically has a structure and functions similar to those of an organ in a living organism. Examples of the organoid obtainable by the differentiation induction method of the present invention include a neural organoid (e.g., a cerebral cortex organoid, a cerebellar organoid, a spinal organoid, a mesencephalic organoid, a choroid plexus organoid, a hippocampal organoid, a hypothalamic organoid, an anterior pituitary organoid, a basal ganglia organoid, a retinal organoid, or the like), a cartilage organoid (also referred to as a "cartilage-like organization"), a heart organoid, a bone organoid (also referred to as a "bone-like organization"), a skeletal muscle organoid, and the like. In an embodiment of the present invention, the organoid is a bone organoid such as a jawbone organoid (also referred to as a "jawbone-like organization").

The bone organoid may be a cartilage bone organoid or a membranous bone organoid, and is preferably an NCC-derived bone (e.g., a presphenoid bone, an ethmoid bone, a membranous bone, or the like) organoid. Among these organoids, a membranous bone organoid is more preferable. Examples of the membranous bone include a frontal bone, a parietal bone, a temporal bone, an occipital bone, a jawbone (a maxilla and a mandible), and the like. Among these membranous bones, a jawbone (particularly a mandible) is preferable. In this specification, a mandibular organoid means an organoid derived from DLX5-positive PA1-EM cells. In Examples below, a phenotype of abnormal bone formation was observed in a bone organoid induced from pluripotent stem cells with a point mutation in COLIA1, which causes abnormal bone formation. Accordingly, the bone organoid may represent a phenotype of a bone disease (e.g., osteogenesis imperfecta).

It is possible to confirm whether or not a structure is an organoid by, for example, confirming whether or not a layered structure is formed by observing, under a microscope, a sample stained as necessary (through, for example, alizarin red staining, HE staining, Azan staining, or the like), or checking the expression of a marker protein. Specifically, the bone organoid has a bone matrix, osteocytes included inside the bone matrix, and an osteoblast layer surrounding the bone matrix. Typically, in the bone organoid, a three-dimensional network formed by osteocytes linked together via a protein such as F-actin involved in the cytoskeleton is observed. Also, the bone organoid has an ability to restore a bone defect when transplanted to the defect. The "bone matrix" is an accumulation of a substance that is produced by osteocytes and secreted extracellularly, and is composed of, for example, calcium phosphate and the like and contains proteins such as osteocalcin, osteopontin, osteonectin, and collagen.

A cerebral cortex organoid has a dome-like neuroepithelium throughout which Foxg1 is expressed, and the neuroepithelium has a layered structure. In the structure above, it is observed that a neural progenitor cell layer corresponding to the ventricular zone positive for Pax6 and Sox2 is present inside the epithelium, and a first layer composed of Cajal Retzius cells positive for Reelin/Carletinin and a deep layer positive for Ctip2/Tbr1 are present outside it.

It is also possible to produce, from NCCs, cells such as neural progenitor cells, nerve cells, glial cells, mesenchymal stem cells, osteocytes, chondrocytes, odontogenic mesenchymal cells, smooth muscle cells, corneal endothelial cells, melanoblasts, and melanocytes. For example, differentiation into these cells can be induced using methods described in Examples below, Fukuta M. et al., PLoS One, 2014, 9(12): e112291, Horikiri T. et al., PLoS One, 2017, 12(1): e0170342, Non-Patent Literature 3, and the like. Differentiation into cells of interest may be confirmed by conducting flow cytometry analysis or immunostaining analysis of cells for expression of a surface antigen or a marker gene after the culture. In this specification, the term "mesenchymal stem cells" means cells that have a self-proliferative capacity and have a capacity to differentiate into at least osteocytes, chondrocytes, and adipocytes.

In an embodiment of the present invention, cells or organoids (cells or organoids of interest) obtainable using the differentiation induction method of the present invention are mesenchymal cells (e.g., mesenchymal stem cells, first pharyngeal arch ectomesenchyme cells, osteoprogenitor cells, osteoblasts, osteocytes, chondrocytes, myoblasts, odontogenic mesenchymal cells, smooth muscle cells, and the like), nerve cells, melanoblasts, or bone organoids (e.g., jawbone organoids).

Expansion culture of NCCs may be conducted before the differentiation induction method of the present invention is conducted. The expansion culture can be conducted in accordance with, for example, the method described in Patent Literature 1. Specifically, the expansion culture can be conducted by conducting suspension culture of neural crest cells in a culture medium containing a GSK-3β inhibitor and a basic fibroblast growth factor (bFGF). The GSK-3β inhibitor used in the expansion culture of NCCs may be the same as the GSK-3β inhibitor used in the NCC production method of the present invention, and SB431542 is preferable. The bFGF (also called FGF-2) used in the expansion culture of NCCs may be a bFGF commercially available from FUJIFILM Wako Pure Chemical Corporation or the like. In addition, the culture medium used for the expansion culture may also contain an ALK inhibitor and an EGF (Epidermal Growth Factor). The ALK inhibitor may be the same as the ALK inhibitor used in the NCC production method of the present invention.

It is also possible to obtain MSCs by conducting adherent culture of neural crest cells in a culture medium for proliferation of mesenchymal stem cells about for 14 days. Such a culture medium is not particularly limited as long as the differentiation of NCCs of the present invention into MSCs can be induced through the culture, and a basal culture medium (e.g., αMEM culture medium) containing bovine serum or the like may be used. A commercially available xeno-free culture medium for proliferation of mesenchymal stem cells is preferable. Examples of the commercially available xeno-free culture medium for proliferation of mesenchymal stem cells include PRIME-XV MSC Expansion XSFM (Irvine Scientific Sales Company, Inc.), Cellartis (registered trademark)) MSC Xeno-Free Culture Medium (Takara Bio Inc.), MSC NutriStem (registered trademark) XF culture medium (Sartorius Stedim Japan K.K.), and the like.

It is also possible to produce differentiated cells by culturing the MSCs in a culture medium suitable for induction of differentiation into cells of interest. Examples of a culture medium for induction of differentiation into osteocytes include basal culture media (e.g., αMEM) containing 10% FBS, 0.1 µM dexa-methasone, 50 µg/ml ascorbic acid, and 10 mM β-glycerophosphate, commercially available xeno-free culture media for induction of differentiation into osteocytes (e.g., MSCgo (trademark) Osteogenic XF Medium (Sartorius AG), and MSCgo (trademark) Rapid Osteogenic Differentiation Medium (Sartorius AG)), and the like. Specifically, the osteocyte differentiation method is conducted as follows, for example: 4×10⁴ mesenchymal stem cells are seeded in a 12-well plate coated with gelatin and are cultured in the above-described culture medium for induction of differentiation into osteocytes for 30 days. Differentiation into osteocytes may be confirmed by detecting calcified nodules through alizanin red staining.

Examples of a culture medium for induction of differentiation into chondrocytes include basal culture media (e.g., DMEM/F12) containing 1% (v/v) ITS+ premix, 0.17 mM AA2P, 0.35 mM proline, 0.1 mM dexamethasone, 0.15% (v/v) glucose, 1 mM sodium pyruvate, 2 mM GlutaMAX, 40 ng/ml PDGF-BB, 100 ng/mL TGF-β3, 10 ng/ml BMP4, and 1% (v/v) FBS, commercially available xeno-free culture media for induction of differentiation into chondrocytes (e.g., MSCgo (trademark) Chondrogenic XF (Sartorius AG)), and the like. Specifically, the chondrocyte differentiation method is conducted as follows, for example: 5 µl of a mesenchymal stem cell suspension is spotted on a plate coated with fibronectin, followed by 1-hour culture, and 1 ml of the above-described culture medium for induction of differentiation is added thereto after 1 hour, followed by 14-day culture. Differentiation into chondrocytes may be confirmed through alcian blue staining.

Examples of a culture medium for induction of differentiation into fat cells include basal media containing 60 µM indomethacin, 0.5 mM IBMX, and 0.5 µM hydrocortisone, and commercially available xeno-free culture media for induction of differentiation into adipocytes (e.g., hMSC - Human Mesenchymal Stem Cell Adipogenic Differentiation Medium (Lonza), MSCgo (trademark) Adipogenic XF (Sartorius AG)), and the like. Specifically, the adipocyte differentiation method is conducted as follows, for example: 4×10⁴ mesenchymal stem cells are seeded on a plate coated with gelatin and are cultured in the above-described culture medium for induction of differentiation into fat cells for 32 days. Differentiation into adipocytes may be confirmed through oil red O staining.

Neural progenitor cells and nerve cells can be obtained by seeding NCCs in a low cell adhesion culture vessel (e.g., 96-well V-bottom ultra-low adhesion culture plate (manufactured by Sumitomo Bakelite Co., Ltd.) or the like) and culturing the NCCs in a basal culture medium (e.g., Neurobasal medium) supplemented with B27 Supplement, N-2 supplement, L-glutamine, BDNF, GDNF, NT-3, and NGF for about 14 days, or conducting adherent culture of NCCs in a basal culture medium (e.g., DMEM/F12) supplemented with N-2 Supplement, BDNF, GDNF, NT-3, and NGF for about 14 days.

Induction of melanoblasts from NCCs is also described in, for example, Mica et al., Cell Reports, 2013; 3(4): 1140-1152, Callahan et al., J. Vis Exp, 2016; (109): e53806, and the like. These methods may be used without modification, but as described in Examples below, differentiation into melanoblasts can be induced with high efficiency by changing adherent culture to suspension culture. Specifically, melanoblasts can be obtained by seeding NCC aggregates in a low cell adhesion culture vessel and culturing the NCC aggregates in a basal culture medium (e.g., StemFit Basic03 culture medium) supplemented with CHIR99021, BMP4, and endothelin-3 for about six days.

It is also possible to induce smooth muscle cells from NCCs or MSCs. Such a method may be, for example, a method in which smooth muscle cells are obtained by conducting adherent culture of NCCs in a culture medium for proliferation of mesenchymal stem cells for about 5 days and then conducting adherent culture in a culture medium for induction of smooth muscle cells for about 5 days, or the like. The culture medium for induction of smooth muscle cells may be, for example, a basal culture medium (e.g., StemFit Basic03 culture medium) supplemented with TGF-β1 and endothelin-1, or the like.

Induction of mandibular PA1-EM cells from the NCCs of the present invention (also referred to as "PA1-EM cell induction" hereinafter) can be conducted by, for example, culturing the NCCs in a culture medium (e.g., StemFit Basic03 culture medium) containing endothelin and a fibroblast growth factor. The endothelin used for the PA1-EM cell induction may be any of endothelin-1 (Edn-1), endothelin-2 (Edn-2), and endothelin-3 (Edn-3), and is preferably Edn-1. These endothelins may be used in combination. The concentration of the endothelin in the culture medium can be selected as appropriate by a person skilled in the art in accordance with the type of endothelin used. For example, when Edn-1 is used, the concentration is typically 1 to 500 nM, preferably 10 to 300 nM, and more preferably 20 to 100 nM (e.g., 50 nM). Meanwhile, it is also possible to induce maxillary PA1-EM cells by using an Edn-1 inhibitor instead of the endothelin or culturing the NCCs using a culture medium containing none of Edn-1, Edn-2, and Edn-3 (i.e., culturing the NCCs in the absence of these substances) in the PA1-EM cell induction method. The Edn-1 inhibitor used in the present invention need only be capable of inhibiting at least binding of Edn-1 to the endothelin A receptor, and examples thereof include BQ-123, bozentan, tezosentan, darusentan, atrasentan, sitaxsentan, and the like. The concentration of the Edn-1 inhibitor in the culture medium can be selected as appropriate by a person skilled in the art in accordance with the type of Edn-1 inhibitor used. For example, when BQ-123 is used, the concentration is typically 0.1 µM to 100 µM, preferably 1 µM to 50 µM, and more preferably 5 µM to 20 µM (e.g., 10 µM). Two or more types of the Edn-1 inhibitors may be used in combination.

It is preferable to conduct suspension culture during the PA1-EM cell induction. Furthermore, it is also preferable to conduct shaking culture and/or stirred culture. The culture period for inducing PA1-EM cells is also not particularly limited as long as PA1-EM cells are obtainable, and it is typically 3 days to 6 days, but can be changed as appropriate depending on whether or not shaking culture is conducted, and the like.

The fibroblast growth factor (FGF) used to induce PA1-EM cells may be an FGF commercially available from FUJIFILM Wako Pure Chemical Corporation or the like. Twentytwo types of human FGFs are known, and any of them can be used as long as it can function as a cell growth factor, but it is preferable to use FGF-8 to induce PA1-EM cells from the NCCs of the present invention. FGF-8a, FGF-8b, FGF-8e, and FGF-8F are known as isoforms having the functions of the human FGF-8, and any of them may be used, but it is preferable to use FGF-8b. Two or more types of FGFs may be used in combination. The concentration of the FGF in the culture medium can be selected as appropriate by a person skilled in the art in accordance with the type of FGF used. For example, when FGF-8 is used, the concentration is typically 1 to 500 ng/ml, preferably 10 to 300 ng/ml, and more preferably 50 to 200 ng/ml (e.g., 100 ng/ml).

As described in Examples below, it was shown that the PA1-EM cell induction efficiency is improved by using a BMP for PA1-EM cell induction. Accordingly, it is preferable that the culture medium for the PA1-EM cell induction contains a BMP. Examples of the BMP include BMP2, BMP3, BMP3b, BMP4, BMP5, BMP6, BMP7, BMP8, BMP9, BMP10, BMP11, BMP12, BMP13, BMP14, BMP15, BMP16, BMP17, BMP18, and the like. Among these BMPs, BMP4, BMP2, and BMP7 are preferable, and BMP4 is particularly preferable. Two or more types of BMPs may be used in combination. The concentration of the BMP in the culture medium is adjusted as appropriate depending on the type of BMP added. For example, when BMP4 is used, the concentration is typically 1 to 50 ng/mL, preferably 2 to 30 ng/mL, and more preferably 2.5 to 10 ng/mL.

In yet another aspect of the present invention, PA1-EM cells (also referred to as "PA1-EM cells of the present invention" hereinafter) are also provided. The PA1-EM cells are mandibular PA1-EM cells and typically have all of the following features (a) to (c).
(a) derived from neural crest cells that do not express HOX
(b) expressing DLX5, PRRX1, and TWIST1
(c) responding to the epithelial signal and enabling induction of patterning

The PA1-EM cells may be obtainable by the PA1-EM cell induction method above or may be obtained using another method. Also, the PA1-EM cells of the present invention may have a feature (d): having an actin filament (also referred to as an "F-actin" or "actin stress fiber"). PA1-EM cells that have the features (a) and (c) and a feature (b'): expressing POU3F3 and CYP26A1 (and may further have the feature (d) above) can be considered as maxillary PA1-EM cells.

The PA1-EM cells of the present invention are typically in the form of a cell aggregate. In an aspect, the PA1-EM cell aggregate has a surface region (surface zone), a central region (central zone), and an intermediate region (intermediate zone) located between the surface zone and the central zone. The central zone may contain DLX2 single positive cells, the intermediate zone may contain DLX2⁺ DLX5⁺ cells, and the surface zone may contain DLX2⁺ DLX5⁺ HAND2⁺ cells. Accordingly, in another aspect of the present invention, provided is a cell aggregate (mandibular PA1-EM cell aggregate) that includes the central zone containing DLX2 single positive cells, the intermediate zone containing DLX2⁺ DLX5⁺ cells, and the surface zone containing DLX2⁺ DLX5⁺ HAND2⁺ cells.

The wording "that do(does) not express HOX" in the feature (a) above means that at least HOXA2 and HOXA3 are not expressed. Typically, cells that do not express HOX can be produced in accordance with the NCC production of the present invention. The term "epithelial signal" in the feature (c) above means an epithelial signal expressed in the first pharyngeal arch, which temporarily develops during the fetal period of a vertebrate. Specific examples of the epithelial signal include a combination of Fgf8 and sonic hedgehog (Shh), which is a proximal-oral signal; a combination of Shh and BMP4, a combination of Fgf8 and Shh, and Edn-1 and BMP4, which are distal-oral signals; a combination of Edn-1 and BMP4, which is a distal-aboral signal; Edn-1, which is a proximal-aboral signal; and the like (see also FIG. 39A). The wording "responding to the epithelial signal and enabling induction of patterning" means that the PA1-EM cells express a specific gene in response to each of the epithelial signals above. For example, the PA1-EM cells express the PAX9 gene, the LHX8 gene, and the like in response to the proximal-oral signal, Fgf8 and Shh, and the PA1-EM cells express the GSC gene, the MSX2 gene, and the like in response to the distal-aboral signals, Edn-1 and BMP4. In the feature (d) above, the actin filament is a filamentous polymer formed through polymerization of a large number of globular actin (G-actin) molecules, and the presence of the actin filament can be checked through phalloidin staining, which will be described below.

As described in Examples below, PA1-EM cells can be efficiently induced from not only the NCCs of the present invention but also NCCs obtained using a conventional two-dimensional culture method by inhibiting the formation of actin filaments. Accordingly, NCCs used to produce the PA1-EM cells of the present invention are not particularly limited. In another aspect, also provided is a method for producing PA1-EM cells that includes a step of inducing differentiation of NCCs (NCCs cultured under two-dimensional conditions in an aspect) into PA1-EM cells in the presence of an actin filament formation inhibitor. All the descriptions regarding the above-described method for inducing mandibular PA1-EM cells from the NCCs of the present invention are applied to a specific method for inducing differentiation into PA1-EM cells.

Examples of the actin filament formation inhibitor used in the present invention include Y-27632, cytochalasin A, cytochalasin B, cytochalasin C, cytochalasin D, cytochalasin E, latrunculin A, latrunculin B, wiskostatin, BDM, latrunculin, chaetoglobosin A, and the like. The concentration of the actin filament formation inhibitor in the culture medium can be selected as appropriate by a person skilled in the art in accordance with the type of actin filament formation inhibitor used. For example, when Y-27632 or a salt thereof (e.g., Y-27632 dihydrochloride) is used, the concentration is typically 0.5 µM to 500 µM, preferably 2 µM to 200 µM, and more preferably 10 µM to 100 µM (50 µM in an aspect). Two or more types of actin filament formation inhibitors may be used in combination.

More specifically, for example, when PA1-EM cells express PAX9 and LHX8 after being cultured in a basal culture medium (e.g., StemFit Basic03 culture medium) supplemented with Fgf8b, SAG, BQ-123, and LDN193189 for about 3 days, it can be determined that the PA1-EM cells respond to the proximal-oral epithelial signal and can induce patterning. Also, for example, when PA1-EM cells express GSC and MSX2 after being cultured in a basal culture medium (e.g., StemFit Basic03 culture medium) supplemented with Fgf2, Endothelin-1, and BMP4 for about 3 days, it can be determined that the PA1-EM cells respond to the distal-aboral epithelial signal and can induce patterning.

Differentiation of the thus induced proximal-oral cells expressing PAX9 and LHX8 and distal-aboral cells expressing GSC and MSX2 can also be further induced. It is also possible to induce odontogenic mesenchymal cells by, for example, culturing the proximal-oral cells in a basal culture medium (e.g., Basic03 culture medium) supplemented with FGF8b, SAG, CHIR99021, and activin A (R&D) for about 4 days. Since odontogenic mesenchymal cells can be starting cells for dentine, which is a main component of teeth, the PA1-EM cells and the odontogenic mesenchymal cells of the present invention may be useful for regeneration of teeth.

Bone organoids can be produced through, for example, a step (I) of inducing distal-aboral cells from the PA1-EM cells of the present invention using the method above, a step (II) of inducing bone progenitor cells from the distal-aboral cells, and a step (III) of inducing bone organoids from the bone progenitor cells. The step (II) above is conducted as follows, for example: suspension culture of the distal-aboral cells in a culture medium for induction of bone progenitor cells (e.g., StemFit Basic03 culture medium supplemented with Egf, Fgf2, BMP2, CHIR99021, and SAG) is conducted for 2 days to 4 days. The step (III) above is conducted as follows: suspension culture of the osteoprogenitor cells in a culture medium for induction of differentiation into osteocytes (e.g., MSCgo (trademark) Osteogenic XF Medium) for 20 days to 30 days. In the middle of the step (III) (e.g., somewhere between 10 days and 20 days after the start of the step (III), or somewhere between 6 days and 10 days), the culture media may be replaced with another culture medium for induction of differentiation into osteocytes (e.g., StemFit Basic03 culture medium containing dexamethasone (typically used in a concentration that is one-tenth the concentration when used to induce osteoblasts), β-glycerophosphate, and ascorbic acid. In this case, typically, the first half of the step is mainly intended to induce differentiation into osteoblasts and osteocyte, and the latter half of the step is intended to mature osteocytes. This may suppress the occurrence of dead cells during the maturation of osteocytes. When the culture medium is replaced with another culture medium for induction of differentiation into osteocytes in the middle of the step (III), the culture medium may be replaced with yet another culture medium (e.g., the culture medium for induction of differentiation into osteocytes used at the start of the step (III)) several days (e.g., six days to 10 days) after the replacement. FIGS. 40 and 59 show the outlines of specific methods, but there is no limitation to these methods.

The bone organoids produced using the method above may be further matured. Examples of such a method include a method in which the bone organoids are transplanted to a living organism such as a mammal (for example, the bone organoids are transplanted under the renal capsule), and the like. As described in Examples below, the bone organoids transplanted to a living organism can mature into bone organoids in which the formation of calcified substances and the formation of a dense bone tissue are observed, and the formation of a dense network of osteocytes of the donor is further observed inside the bone tissue.

Bone organoids can also be produced using MSCs in accordance with the method described in WO 2020/226043. Specifically, bone organoids can be produced as follows: a three-dimensional mesenchymal stem cell aggregate is obtained by proliferating MSCs in a sheet shape (cell sheet) through adherent culture, separating the cell sheet from the culture vessel, and then conducting suspension culture of the cell sheet, and the cell aggregate is embedded in a gel and is then cultured in a culture medium for induction of differentiation into a bone. In this method, the gel is preferably a gel containing a polysaccharide as the main component, and may be, for example, Vitrogel (TheWell Bioscience Inc.).

Neural organoids can be produced by conducting suspension culture of the cell aggregate containing neural progenitor cells induced from NCCs in a culture medium for induction of differentiation into nerve cells. Neural organoids can be induced in a self-organizing manner by continuing the culture of the cell aggregate under the conditions of the suspension culture above. The culture medium for induction of differentiation into nerve cells may be, for example, a basal culture medium (e.g., N2 culture medium) containing a Wnt signal inhibitor and an ALK inhibitor. Examples of the Wnt signal inhibitor include a DKK1 protein, sclerostin, IWR-1e, IWP-2, IWP-3, IWP-4, IWP-L6, C59, ICG-001, FH535, WIKI4, KYO2111, PNU-74654, XAV939, derivatives of these substances, and the like. Examples of the ALK inhibitor include the same ALK inhibitors as those described above.

Examples of a culture vessel to be used for adherent culture include culture vessels whose surfaces have been subjected to artificial treatment (e.g., coating treatment with an extracellular matrix such as a basal membrane preparation, fibronectin, laminin or a fragment thereof, entactin, collagen, gelatin, Synthemax, or vitronectin, or a macromolecule such as polylysine or polyornithine, or surface processing such as positive charge treatment) for the purpose of improving adhesion to cells. Among these culture vessels, a culture vessel coated with fibronectin is preferable.

In the differentiation induction method of the present invention, the culture may be conducted under feeder-free conditions and/or xeno-free conditions over the entire period of the culture or during a part of the period of the culture. From the viewpoint of a clinical application, it is preferable to conduct the differentiation induction method of the present invention under feeder-free conditions and xeno-free conditions throughout the entire period.

The differentiation induction method of the present invention may include a step of harvesting obtained cells or organoids of interest. The harvested cells may be cryopreserved using a cell cryopreservation solution. The number of cells harvested in a vessel may be counted using a cell counter, and the cells may be labeled with an antibody against cell surface marker and then sorted using flow cytometry, mass cytometry, a magnetic cell sorting method, or the like.

In another aspect, provided are cells or organoids obtained using the differentiation induction method of the present invention (the cells or organoids may also be referred to as the "differentiated cells or organoids of the present invention" hereinafter).

All the contents described in "1. Method for Producing Neural Crest Cells" above are applied to the specific examples, the definitions, and the like of the culture conditions of suspension culture, shaking culture, stirred culture, and the like, the culture methods, the method for sorting cells of interest, the additives for a culture medium, and the culture vessel.

### 3. Uses of Differentiated Cells or Organoids

The differentiated cells or organoids of the present invention can be favorably used in regenerative medicine, and therefore, in another aspect, an agent for transplantation therapy containing the differentiated cells or organoids of the present invention (this agent may be referred to as the "agent for transplantation therapy of the present invention" hereinafter) is provided. In addition, the present invention also encompasses a method for treating or preventing an injury (including defect) of a tissue (e.g., a bone tissue, a cartilage tissue, an adipose tissue, and a muscle tissue (e.g., skeletal muscle tissue)) or a disease, the method including administering or transplanting an effective amount of the differentiated cells or organoids of the present invention to a mammal (e.g., a human, a mouse, a rat, a monkey, a cow, a horse, a pig, or a dog) to be treated. Also, the "treatment of a tissue injury" encompasses regeneration of an injured tissue. The regeneration of an injured tissue encompasses not only complete tissue regeneration but also formation of a tissue-like structure. For example, in the case of a bone tissue, it is not necessary to determine whether a regenerated site is composed of a bone tissue through CT (Computed Tomography) scan.

The agent for transplantation therapy of the present invention can be used for administration to or transplantation into the body of a subject in need thereof. It is preferable to conduct the transplantation in a region in a living organism where the differentiated cells or organoids of the present invention can be fixed at a certain position, and the transplantation can be conducted under the skin, in the abdominal cavity, on the peritoneal epithelium, on the bone tissue (e.g., jawbone tissue), on the cartilage tissue, on the greater omentum, in the adipose tissue, in the muscular tissue, under the tunic of an organ such as the pancreas or the kidney, and the like. It is sufficient that a therapeutically-effective amount of the cells or organoids to be transplanted is administered, and the amount may vary depending on the age, the weight, the size of the transplantation site, the disease severity, or the like of a transplantation target and is not particularly limited, but is, for example, about 10×10⁴ cells to 10×10¹¹ cells.

The purpose of the transplantation of the differentiated cells or organoids of the present invention to a living organism may be direct regeneration of an injured tissue or an indirect effect (e.g., paracrine effect) by a factor secreted by the differentiated cells or organoids of the present invention. Mesenchymal stem cells, for example, can have a therapeutic effect on diseases such as acute myocardial infarction, a stroke, multiple system atrophy (MSA), a graft-versus-host disease, Crohn's disease, ischemic cardiomyopathy, spinal cord injury, and the like. Melanoblasts and melanocytes, for example, can have a therapeutic effect on diseases such as dyschromatosis including oculocutaneous albinism, vitiligo, and the like. Osteocytes and bone organoids, for example, can have a therapeutic effect on diseases such as intractable bone fracture, periodontitis, and a case of a wide-range osseous defect after bone extirpation. Neural progenitor cells, nerve cells, or neural organoids, for example, can have a therapeutic effect of diseases such as a cerebrovascular disorder, a brain injury, a spinal cord injury, and neurodegenerative disease.

When the differentiated cells or organoids of the present invention are used as an agent for transplantation therapy, it is desirable to use cells or organoids derived from iPS cells established from somatic cells with the same or substantially the same HLA genotype as that of a transplantation target individual from the viewpoint of preventing rejection. Here, "substantially the same" means that the HLA genotypes are identical to an extent that the immunological reaction to the transplanted cells can be suppressed using an immunosuppressive agent, and, for example, somatic cells with the HLA type in which three genetic loci (HLA-A, HLA-B, and HLA-DR) are respectively identical, four genetic loci (HLA-C in addition to the three loci above) are respectively identical, or the like are used. When a sufficient amount of cells cannot be obtained due to the age, diathesis, or the like, the cells can be embedded in a capsule made of polyethylene glycol or silicone, a porous vessel, or the like for the purpose of avoiding rejection and then transplanted.

A parenteral preparation such as an injection, a suspension, or a drip is produced by, for example, mixing the differentiated cells or organoids of the present invention with a pharmaceutically acceptable carrier in accordance with a conventional procedure. Therefore, in an aspect, also provided is a method for producing an agent for transplantation therapy containing a step of formulating the differentiated cells or organoids of the present invention. This production method may include a step of preparing the differentiated cells or organoids of the present invention. In addition, the production method can also include a step of preserving the differentiated cells or organoids of the present invention.

Examples of the pharmaceutically acceptable carrier that can be contained in the parenteral preparation include aqueous liquids for injection such as a physiological saline solution and an isotonic solution containing glucose and other adjuvants (e.g., D-sorbitol, D-mannitol, and sodium chloride). The cells of the present invention may be blended with a buffering agent (e.g., a phosphate buffer solution or sodium acetate buffer solution), a soothing agent (e.g., benzalkonium chloride or procaine hydrochloride), a stabilizer (e.g., human serum albumin or polyethylene glycol), a preservative, an antioxidant, and the like.

The agent for transplantation therapy of the present invention can be used as follows: it is provided in a state of being cryopreserved under the conditions commonly used for cryopreservation of cells, and is thawed before use. In such a case, the agent may further contain serum or an alternative thereof, an organic solvent (e.g., DMSO), and the like. In such a case, the concentration of the serum or the alternative thereof is not particularly limited, but can be about 1% (v/v) to about 30% (v/v) and preferably about 5% (v/v) to about 20% (v/v). The concentration of the organic solvent is not particularly limited, but can be 0% (v/v) to about 50% (v/v) and preferably about 5% (v/v) to about 20% (v/v).

The differentiated cells or organoids of the present invention can also be used in a method for screening a candidate of a drug useful for treatment or prevention of a tissue injury or a disease. Accordingly, in yet another aspect of the present invention, provided is a method for screening a disease therapeutic or preventive medicament, including a step of culturing the differentiated cells or organoids of the present invention in the presence or absence of a test substance. Examples of the tissue injury or the disease include the same tissue injuries or diseases as those to be a treatment target of the agent for transplantation therapy of the present invention. The differentiated cells or organoids for use in the screening may present a phenotype of a disease such as a bone disease (e.g., osteogenesis imperfecta). In this case, it is also possible to screen a candidate drug based on a change in the phenotype.

Examples of the test substance used in the screening method include a cell extract, a cell culture supernatant, a microbial fermentation product, an extract derived from a marine organism, a plant extract, a purified or crude protein, a peptide, a non-peptide compound, a synthetic low-molecular-weight compound, a natural compound, and the like.

The test substance above can also be obtained using any of many approaches in combinatorial library methods known in the art, including (1) a biological library, (2) a synthetic library method using deconvolution, (3) a "one-bead one compound" library method, and (4) a synthetic library method using affinity chromatography sorting. The biological library method using affinity chromatography sorting is limited to a peptide library, but four other approaches can be applied to a peptide library, a non-peptide oligomer library, or a low-molecular-weight compound library of compounds (Lam (1997) Anticancer Drug Des. 12: 145-67). Examples of a method for synthesizing a molecular library can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; Gallop et al. (1994) J. Med. Chem. 37: 1233-51). The compound library can be produced as solutions (see Houghten (1992) Bio/Techniques 13: 412-21) or beads (Lam

(1991) Nature 354: 82-4), chips (Fodor (1993) Nature 364: 555-6), bacteria (U.S. Patent No. 5,223,409), spores (U.S. Patent No. 5,571,698, U.S. Patent No. 5,403,484, and U.S. Patent No. 5,223,409), plasmids (Cull et al. (1992) Proc. Natl. Acad. Sci. USA 89: 1865-9), or phages (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; U.S. Publication No. 2002103360).

Hereinafter, the present invention will be described more specifically by way of examples, but the present invention is not limited to these examples by any means.

### Examples

### Example 1: Induction of NCC Aggregate

### <Maintenance Culture of Human iPS Cells>

Human iPS cells (1231A3 cell line, Ff-I 14s04 cell line, Ff-I 14s04 HLA-KO cell line, iPS cell line derived from a patient with osteogenesis imperfecta) produced using an episomal vector were cultured using the StemFit AK03N culture medium (manufactured by Ajinomoto Co., Inc.) on a culture plate coated with iMatrix-511 (manufactured by Nippi, Inc.). The culture medium was replaced every two days.

### <Induction of NCC Aggregate from iPS Cells>

The outline of a method for inducing an NCC aggregate from iPS cells (three-dimensional (3D) NCC induction method) is shown in FIG. 1. Specifically, the method was conducted in accordance with the following procedure. After the human iPS cells (1231A3 cell line (xeno-free cell line for a study)) that had been subjected to the maintenance culture were dispersed into single cells using Accutase (manufactured by Innovative Cell Technologies Inc.), the cells were suspended in a culture solution prepared by adding 10 µM Y-27632 (manufactured by FUJIFILM Wako Pure Chemical Corporation) to StemFit Basic03 (corresponding to AK03N containing no bFGF) (manufactured by Ajinomoto Co., Inc.) and were seeded on a 96-well V-bottom ultra-low adhesion culture plate (manufactured by Sumitomo Bakelite Co., Ltd.) (1.0×10⁴ cells / 100 µl / well). This plate was centrifuged at 1200 rpm for 3 minutes, and thus the cells were gathered on the bottom of the plate. After being cultured for 1 day, these cells coagulated and formed a cell aggregate. Next, the cell aggregates were cultured for 1 day in a culture solution prepared by adding 10 µM SB431542 (manufactured by Selleck Chemicals) and 10 ng/mL BMP4 (manufactured by R&D Systems Inc.) to the StemFit Basic03 culture medium (this procedure corresponds to the steps D1 and D2 in FIG. 1 and may be referred to as the "SB-BMP action step" hereinafter). Thereafter, the cell aggregates were transferred to a 48-well low adhesion culture plate (manufactured by Iwaki) one by one, and shaking culture (120 rpm) was conducted in a culture solution prepared by adding 10 µM SB431542 and 1 µM CHIR99021 (manufactured by Axon Medchem) to the StemFit Basic03 culture medium using a shaking culture vessel (CS-LR (manufactured by TAITEC Corporation) for 3 days (this procedure corresponds to the steps D2 and D3 in FIG. 1 and may be referred to as the "NCC induction step" hereinafter). As a result, globular cell aggregates having a diameter of about 500 µm were obtained (FIGS. 1, 2A, 3 and 8A).

### <Analysis of Characteristics of NCCs>

The ratio of CD271-positive cells in the obtained cell aggregate was measured through flow cytometry. First, the cell aggregate was dispersed into single cells using Accutase (manufactured by Innovative Cell Technologies Inc.), and then reaction with a CD271 antibody (manufactured by BD Biosciences, 560326) in an antibody dilution prepared by diluting the antibody by a factor of 200 in a FACS buffer (PBS containing 0.1% human serum) was conducted at 4°C for 60 minutes. An isotype control was provided to eliminate nonspecific background signals. After the reaction with the antibody, the cells were filtered through a 35-µm filter (manufactured by Falcon) and were analyzed through flow cytometry using FACS Aria II (manufactured by BD Biosciences) to measure the ratio of cells strongly positive for CD271. Images were created from the FCM data using FlowJo software (manufactured by BD Biosciences). The results of the flow cytometry showed that the ratio of cells strongly positive for CD271 contained in the cell aggregate was very high (94.6%) (FIG. 2B).

Next, the expression of CD271, SOX10, and PAX6 in the cell aggregate was checked through immunostaining. First, the cell aggregate was fixed using 4% paraformaldehyde - phosphate buffer and was then embedded in the Tissue-Tek OCT compound (manufactured by Sakura Finetek Japan Co., Ltd.). The resulting sample was cut into thin sections using a cryostat (manufactured by Leica) to produce 12-µm-thick cryosections. After being washed with PBS, the sections were permeabilized with 3% Triton X-100 (manufactured by Sigma) at room temperature for 15 minutes. Blocking treatment was conducted using Blocking one Histo (manufactured by NACALAI TESQUE, INC.) at room temperature for 1 hour. Reaction with a CD271 antibody (manufactured by Advanced Targeting Systems, AB-N07), a SOX10 antibody (manufactured by R&D Systems, AF2864), and a PAX6 antibody (manufactured by Abcam Limited., ab195945) in an antibody dilution prepared by diluting the antibodies by a factor of 200 in Can Get Signal (trademark) immunostain Solution B (manufactured by TOYOBO Co., Ltd.) was conducted at 4°C overnight. Reaction with a secondary antibody in an antibody dilution prepared by diluting the antibody by a factor of 200 in Can Get Signal (trademark) immunostain Solution B was conducted at room temperature for 3 hours. To stain the nucleus, reaction with 4',6-diamidino-2-phenylindole (DAPI) (manufactured by Dojindo Laboratories) was conducted at room temperature for 10 minutes. The sections were mounted using VECTASHIELD (registered trademark) Antifade Mounting Medium (manufactured by VECTOR LABORATORIES). Fluorescent signals of the samples were observed using a confocal laser microscope Olympus FV3000 (manufactured by Olympus Corporation). The results of the immunostaining showed that the inner portion of the cell aggregate was mainly composed of NCCs that were positive for CD271 and SOX10 and negative for PAX6 (FIG. 2C). Also, the expression of CD271, SOX10, and PAX6 in the cell aggregates on each day from Day 1 (D1) to Day 5 (D5) after the start of the cell culture was checked through immunostaining. It was shown that NCCs positive for SOX10 and CD271 appeared on D4 (after 2 days elapsed from the start of the NCC induction step) and the ratio of the NCCs increased on D5 (FIG. 3).

The gene expression pattern was compared with that of NCCs (two-dimensional (2D) NCC induction method) produced using adherent culture in accordance with a conventional method (upper diagram in FIG. 4). In the 2D NCC induction method, iPS cells were cultured in a culture solution containing 10 µM SB431542 and 1 µM CHIR99021 using 6 wells coated with iMatrix-511 for 10 days, and NCCs were isolated by sorting cells strongly positive for CD271 from the obtained NCC aggregates through flow cytometry (the obtained NCCs are abbreviated as "2D iNCCs" or "2D 271H"). Also, in the 3D NCC induction method, NCCs were isolated by sorting cells strongly positive for CD271 from the NCC aggregate on D5 through flow cytometry (the obtained NCCs are abbreviated as "3D iNCCs" or "D5 271H") (central diagram in FIG. 4).

Subsequently, the expression levels of the NCC-related genes (NGFR, SOX10, TFAP2A, and PAX3) and the pluripotent cell marker POU5F1(Oct4) in each type of NCC were measured through RT-qPCR. First, total RNA of the cell aggregate was collected using RNeasy Mini Kit (manufactured by Qiagen N.V.), and genome DNA was removed using DNase-one Kit (manufactured by Qiagen N.V.). Reverse transcription reaction was conducted using PrimeScript RT Master Mix (manufactured by Takara Bio Inc.) to synthesize single-stranded cDNA from 500 ng of the total RNA. The cDNA was mixed with Thunderbird SYBR qPCR Mix (manufactured by TOYOBO Co., Ltd.), and RT-qPCR measurement was conducted using StepOnePlus (trademark) Real-Time PCR System (manufactured by Applied Biosystems). It was observed that the NCC-related genes were expressed at substantially the same levels in both the 2D iNCCs and 3D iNCCs, and it was shown that the expression of the pluripotent cell marker POU5F1 disappeared in both types of NCCs (lower diagram in FIG. 4). The primers used in RT-qPCR were the same as those listed in Supplementary Table 2 of Non-Patent Literature 3.

The expression levels of the NCC-related genes (NGFR, SOX10, TFAP2A, and PAX3), the neural plate border marker genes (DLX5 and MSX2), and the pluripotent cell marker Oct4 were measured through RT-qPCR in the cell aggregates (CD271-high expression cells had not been sorted from all of them) on each day from Day 0 (D0) to Day 5 (D5) after the start of the cell culture, D5 271H, and 2D 271H. The results are shown in FIGS. 5 and 6. FIG. 5 shows that the expression level of the neural plate border-related marker increased after 1 day elapsed from when the SB-BMP action step was changed to the NCC induction step (on D3). FIGS. 5 and 6 show that the cells on D5 expressed the NCC-related genes to substantially the same extent as those in the cells of the D5 271H, and the cells on D5 also expressed the NCC-related genes to substantially the same extent as those in the cells of the 2D 271H. The base sequences of the primers other than those listed in Supplementary Table 2 of Non-Patent Literature 3 out of the primers used in RT-qPCR are shown in Table 1. The base sequences of primers used in examples other than this example are also listed in Table 1 (in the table, Fw represents a forward primer, and Rev represents a reverse primer).

**[Table 1]**

| Gene name | Sequence (5' → 3') | SEQ ID NO. |
|---|---|---|
| HOXA2 | Fw: GCATCACCAAACAAAAACTCCTTTG | 1 |
| | Rev: TGGGTCAGGGAATCACTAAACAGAA | 2 |
| HOXA3 | Fw: ACCAACTAACGCCTAAACCTCGGTA | 3 |
| | Rev: GCAGATTTTTGGAGCAATTCTTTCC | 4 |
| HOXB2 | Fw: TTCACCAGTACGCTCTGTGC | 5 |
| | Rev: TTTTCCAGTAGACGGCCAAG | 6 |
| DLX5 | Fw: GGAAGCGCTTTGCACAAACTA | 7 |
| | Rev: TTCACCATTCTCACCTCGGG | 8 |
| PRRX1 | Fw: TGATGCTTTTGTGCGAGAAGA | 9 |
| | Rev: AGGGAAGCGTTTTTATTGGCT | 10 |
| TWIST1 | Fw: GCCAGGTACATCGACTTCCTCT | 11 |
| | Rev: TCCATCCTCCAGACCGAGAAGG | 12 |
| PAX9 | Fw: ACAGTGCTGCTCCTTCTGGT | 13 |
| | Rev: ATGTGAGACCTGGGAATTGG | 14 |
| LHX8 | Fw: CAAGCACAATTTGCTCAGGA | 15 |
| | Rev: GGCACGTAGGCAGAATAAGC | 16 |
| GSC | Fw: GAGGAGAAAGTGGAGGTCTGGTT | 17 |
| | Rev: CTCTGATGAGGACCGCTTCTG | 18 |
| MSX2 | Fw: AGCGCAAGTTCCGTCAGAAA | 19 |
| | Rev: ACCTGGGTCTCTGTGAGGTT | 20 |

Even when BMP2 (manufactured by R&D Systems) or BMP7 (manufactured by R&D Systems) was used at 10 ng/mL as the bone morphogenetic protein instead of BMP4, NCC aggregates were obtained in a similar manner (FIG. 7). Furthermore, even when A-83-01 (manufactured by TOCRIS Bioscience) was used at 1.0 µM as an ALK inhibitor instead of SB431542, NCC aggregates were obtained in a similar manner (FIG. 7).

Also, even when iPS cell lines (Ff-I 14s04 cell line, Ff-I 14s04 HLA-KO cell line, iPS cell line derived from a patient with osteogenesis imperfecta) other than the 1231A3 cell line were used, NCC aggregates were obtained in a similar manner (FIG. 8). In this case, the number of cells seeded and the concentration of BMP4 were adjusted for each cell line. Specifically, in the case of the Ff-I 14s04 cell line and the Ff-I 14s04 HLA-KO cell line, the cells were seeded at 3.0×10³ cells / 100 µl/ well, and the concentration of BMP4 was set to 2.5 ng/mL. In the case of the iPS cell line derived from a patient with osteogenesis imperfecta, the cells were seeded at 2.0×10³ cells / 100 µl / well, and the concentration of BMP4 was set to 2.5 ng/mL.

Culture was conducted using a conventional method under the same conditions as those of the 3D NCC induction method, except that adherent culture was conducted instead of suspension culture. Specifically, the method was conducted in accordance with the following procedure. Cell aggregates were obtained by culturing iPS cells in a culture solution containing 10 µM SB431542 and 1 µM CHIR99021 using 6 wells coated with iMatrix-511 for 4 days (SB CHIR D4 in FIG. 9), or culturing iPS cells in a culture solution containing 10 µM SB431542 and 10 ng/mL BMP4 for 1 day and then culturing the cells in a culture solution containing 10 µM SB431542 and 1 µM CHIR99021 for 3 days (SB BMP4 D1 SB CHIR D3 in FIG. 9). As a result of analyzing the obtained cell aggregates through flow cytometry, it was shown that all the cell aggregates contained substantially no CD271-high expression cells. Also, an influence of shaking culture on the NCC induction efficiency in the 3D NCC induction method above was evaluated. As a result, it was shown that even when shaking culture was not conducted, CD271-high expression NCCs were obtained with high efficiency (87.0%), but the efficiency of the CD271-high expression NCCs was further improved by conducting shaking culture in the NCC induction step (lower diagram in FIG. 9).

### Example 2: Verification of Culture Conditions for Induction of NCC Aggregate

### <Verification of SB-BMP Action Step 1>

A change in the ratio of cells strongly positive for CD271 was verified through flow cytometry while changing the action period of SB431542 and BMP4 in order to search for the optimum action period of SB-BMP action step. Also, the expression patterns of the CD271 (NCC marker) gene, PAX6 (neuroectoderm marker), and the E-Cadherin (epithelial cell marker) gene were verified through immunostaining. In this case, the period of shaking culture was fixed. The results are shown in FIGS. 10 and 11. As shown in FIG. 10, NCCs were induced with high efficiency when the period of the SB-BMP action step was set to 1 day (A), whereas a decrease in the ratio of cells strongly positive for CD271 was observed when the SB-BMP action step was not conducted or the period of the SB and BMP action step was extended (this step was conducted for 2 days) (B and C). As shown in FIG. 11, NCCs strongly positive for CD271 were induced with high efficiency and cells positive for PAX6 and E-Cadherin were not induced when the period of the SB-BMP action step was set to 1 day. Also, it was observed that neuroectoderm positive for PAX6 was induced when the SB-BMP action step was not conducted (B), and it was observed that epithelial cells positive for E-Cadherin were induced around the cell aggregate when the period of the SB-BMP action step was extended (this step was conducted for 2 days).

### <Verification of SB-BMP Action Step 2>

A change in the ratio of cells strongly positive for CD271 was verified through flow cytometry while changing the action period of SB431542 and BMP4 and conducting culture for up to 10 days. Also, the expression level of the CD271 (NGFR) gene was measured through RT-qPCR. In this case, shaking culture was conducted from the start of the NCC induction step. The outline of a method for inducing an NCC aggregate from iPS cells is shown in FIG. 12. The results are shown in FIGS. 13 to 16. As shown in FIG. 13, it was shown that when the SB-BMP action step was conducted for 6 hours (SB BMP4 6h) or 12 hours (SB BMP4 12h) and then the verification was made through flow cytometry on Day 5, the NCC induction efficiency decreased compared with the case where the period of the SB-BMP action step was set to 1 day (SB BMP4 D1). It is assumed that this is because not only NCCs but also neuroectoderm was simultaneously induced due to the short period of the SB-BMP action step. As shown in FIG. 14, it was shown that when the SB-BMP action step was conducted for 2 days (SB BMP4 D2) or 3 days (SB BMP4 D3) and then the verification was made through flow cytometry on Day 5, high NCC induction effects were obtained in both cases. Meanwhile, it was shown that when the verification was made through flow cytometry on Day 5 without conducting the SB-BMP action step, low NCC induction effects were obtained. As shown in FIG. 15, it was shown that when the culture period was extended to 7 days, the expression of CD271 in the whole cell population decreased irrespective of the period of the SB-BMP action step. It is assumed that this is because the differentiation of the NCCs into other types of cells progressed between Day 5 and Day 7 after the start of the induction. Also, it was shown from the results of the RT-qPCR that the expression level of NGFR (CD271) decreased after Day 5 from the start of the culture (FIG. 16).

Based on the results above, regarding the SB-BMP action step, the patterns of methods that are preferable in terms of the NCC induction efficiency are exemplified in FIG. 17.

### <Verification of NCC Induction Step>

Next, the optimum action period of the NCC induction step was searched for. The ratio of cells strongly positive for CD271 when the NCC induction period was extended from 3 days to 5 days or 8 days was verified through flow cytometry. As a result, it was shown that when the NCC induction period was extended, the ratio of cells strongly positive for CD271 decreased in a time-dependent manner (FIG. 18).

### <Verification of Shaking Culture>

Finally, an influence of shaking culture on the NCC induction efficiency was verified. Shaking culture was started at the start of the SB-BMP action step, the ratio of cells strongly positive for CD271 was measured through flow cytometry on Days 5, 7, and 10 after the start of the culture. Also, the expression patterns of the CD271 (NCC marker) gene and the E-Cadherin (epithelial cell marker) gene were verified through immunostaining. In this case, the BMP action period was set to 6 hours, 12 hours, 1 day, or 2 days. The outline of a method for inducing an NCC aggregate from iPS cells is shown in FIG. 19. The results are shown in FIGS. 20 to 22. As shown in FIG. 20, when shaking culture was conducted from the start of the SB-BMP action step, NCCs could be induced on Day 5 in both cases where the period of the SB-BMP action step was 6 hours (SB BMP4 6h) and 12 hours (SB BMP4 12h). However, in the case where the the period was 12 hours, highly efficient NCC induction to the same extent as the case where the period of the SB-BMP action step was 1 day (SB BMP4 D1) was observed. However, when the SB-BMP action step was conducted for 1 day or 2 days, epithelial cells were observed around the cell aggregate on Day 5 through immunostaining (FIG. 21). Furthermore, it was shown that when the NCC induction step was extended to 7 days or 10 days, the ratio of cells strongly positive for CD271 decreased in both cases (FIG. 22).

Based on the results above, the outline of a method that is preferable in terms of the NCC induction efficiency when shaking culture is conducted from the start of the SB-BMP action step is exemplified in FIG. 23.

### Example 3: Verification of Differentiation Capacity of NCCs

The differentiation capacity of the NCCs obtained in Example 1 (the NCC induction method of the present invention) was verified. The outline is shown in FIG. 24. Specifically, the capacity of the NCCs induced in Example 1 to differentiate into peripheral nerve cells, melanoblasts, mesenchymal stem cells, and first pharyngeal arch ectomesenchyme cells was verified.

### <Induction of Peripheral Nerve Cells from NCC Aggregate>

The outline of this induction method is shown in FIG. 25A. Specifically, the method was conducted in accordance with the following procedure. The NCC aggregates obtained in Example 1 were transferred to a 96-well U-bottom ultra-low adhesion culture plate (manufactured by Sumitomo Bakelite Co., Ltd.) and were cultured for 14 days in an induction culture medium prepared by adding the B27 supplement (manufactured by Thermo Fisher Scientific), the N-2 supplement (manufactured by Thermo Fisher Scientific), 2 mM L-glutamine (manufactured by FUJIFILM Wako Pure Chemical Corporation), 10 ng/mL BDNF (manufactured by FUJIFILM Wako Pure Chemical Corporation), GDNF (manufactured by FUJIFILM Wako Pure Chemical Corporation), NT-3 (manufactured by FUJIFILM Wako Pure Chemical Corporation), NGF (manufactured by FUJIFILM Wako Pure Chemical Corporation) to the Neurobasal medium (manufactured by Thermo Fisher Scientific). The culture medium was replaced every three days. Observation through immunofluorescent staining of Peripherin, which is a peripheral nerve cell marker, showed that the NCCs could differentiate into peripheral nerve cells (FIG. 26A).

### <Induction of Melanoblasts from NCC Aggregate>

The methods for inducing melanoblasts from NCCs obtained through adherent culture are known (e.g., Mica et al., Cell Reports, 2013; 3(4): 1140-1152, Callahan et al., J. Vis Exp, 2016; (109): e53806, and the like), but with these methods, many dead cells are generated, and the induction efficiency varies and cannot be said to be sufficient. Therefore, it was verified whether or not differentiation of the NCC aggregates obtained in Example 1 into melanoblasts can be induced through suspension culture. The outline of this induction method is shown in FIG. 25B. Specifically, the method was conducted in accordance with the following procedure. The NCC aggregates obtained in Example 1 were transferred to a 96-well U-bottom ultra-low adhesion culture plate (manufactured by Sumitomo Bakelite Co., Ltd.) and were cultured for 6 days in an induction culture medium prepared by adding 1 µM CHIR99021, 25 ng/mL BMP4, and 100 nM Endothelin-3 (manufactured by TOCRIS Bioscience) to the StemFit Basic03 culture medium. The culture medium was replaced on Day 3 after the start of the induction. Observation through immunofluorescent staining of MITF, which is a melanoblast marker, showed that the NCCs could differentiate into melanoblasts (FIG. 26B).

### <Induction of Mesenchymal Stem Cells (MSCs) from NCC Aggregate>

The outline of this induction method is shown in FIG. 27. Specifically, the method was conducted in accordance with the following procedure. The NCC aggregates obtained in Example 1 were dispersed into single cells using Accutase, and cells strongly positive for CD271 were sorted through flow cytometry. The sorted cells were suspended in a culture solution prepared by adding 10 µM SB431542, 20 ng/mL Egf (manufactured by FUJIFILM Wako Pure Chemical Corporation), and Fgf2 (manufactured by FUJIFILM Wako Pure Chemical Corporation) to the StemFit Basic03 culture medium, and were seeded on a fibronectin-coated culture plate at a cell concentration of 1×10⁴ cells/cm². Then, the cells were cultured. The culture medium was replaced every three days. Thereafter, NCCs that had been passaged twice were dispersed into single cells using Accutase, were suspended in a culture solution prepared by adding 10 µM SB431542, 20 ng/mL Egf, and Fgf2 to the StemFit Basic03 culture medium, and were seeded on a fibronectin-coated culture plate at a cell concentration of 1×10⁴ cells/cm². The culture medium was replaced with the PRIME-XV MSC Expansion XSFM medium (manufactured by Irvine Scientific Sales Company, Inc.) 1 day after the seeding, and MSC induction was started. Subsequent passage was conducted every 4 days using Accutase, and the cells were seeded on a culture plate at 1×10⁴ cells/cm² and were cultured. The expression of human MSC markers (CD105, CD90, CD73, CD44) was checked using flow cytometry on Day 14 after the start of the MSC induction culture, indicating that the cells could differentiated into MSCs (FIG. 28).

### <Induction of First Pharyngeal Arch Ectomesenchyme Cells from NCC Aggregate>

During the development of vertebrates, NCCs separate from the dorsal crest of the neural tube, and then migrate and differentiate into various organs (e.g., the first pharyngeal arch ectomesenchyme (PA1), the second pharyngeal arch ectomesenchyme (PA2), the third pharyngeal arch ectomesenchyme (PA3), the frontonasal process (FNP), and the like) during the formation of nerves in the developing embryo. However, success in selective induction of first pharyngeal arch ectomesenchyme (PA1-EM) cells in vitro from NCCs has not been reported. The features of PA1-EM are outlined in FIG. 29.

In order to induce PA1-EM cells from an NCC aggregate, the NCC aggregate needs to be negative for the expression of the HOX gene, and therefore, first, the expression of the HOX gene in the NCC aggregate obtained in Example 1 was checked through RT-qPCR. An NCC aggregate in which the expression of the HOX gene was induced through retinoic acid (RA) stimulation was prepared as a positive control. The outlines of the methods are shown in FIG. 30A. An NCC aggregate positive for the HOX gene was induced by culturing the cells in a culture medium containing 1.0 µM RA from Day 4 after the start of the induction. Specifically, the method was conducted in accordance with the following procedure. The NCC aggregate positive for the HOX gene was induced by culturing the cells in a culture solution prepared by adding 10 µM SB431542, 1 µM CHIR99021, and 1.0 µM RA (manufactured by Sigma) to the StemFit Basic03 culture medium for 1 day from Day 4 from the start of the induction of the NCC aggregate. The expression of the NCC-related genes (NGFR, SOX10) in the NCC aggregate was checked through RT-qPCR. The expression levels of the NCC-related genes remained the same even when the RA stimulation was conducted (FIG. 30B). Next, the expression of the HOX genes (HOXA2, HOXA3) in the NCC aggregate was checked through RT-qPCR. The HOX genes were highly expressed in the RA-stimulated NCC aggregate, whereas the expression of the HOX genes was not observed in the RA-non-stimulated NCC aggregate (FIG. 30C). The base sequences of the primers other than those listed in Supplementary Table 2 of Non-Patent Literature 3 out of the primers used in RT-qPCR are shown in Table 1.

Next, induction of PA1-EM cells from the NCC aggregate was conducted. The outline of the induction method is shown in FIG. 31. Fig8, Edn-1 (Endothelin-1), and BMP4 were added to the culture medium as a factor necessary for existence and proliferation of the cells, a factor necessary for mandibular selection, and a factor necessary for distal induction, respectively. Specifically, the method was conducted in accordance with the following procedure. Shaking culture of the NCC aggregates in an induction culture medium prepared by adding 50 nM Endothelin-1 (manufactured by Sigma), 100 ng/mL Fgf8b (manufactured by FUJIFILM Wako Pure Chemical Corporation), and 2.5 ng/mL BMP4 to the StemFit Basic03 culture medium was conducted using a shaking culture vessel for 4 days. The induction of PA1-EM cells was confirmed through morphometry and immunofluorescent staining of TWIST1, which is an early mesenchymal cell marker, and DLX5, which is a first pharyngeal arch mandibular prominence ectomesenchyme marker. The results are shown in FIG. 32. The micrographs show that in the PA1-EM cell aggregate induced from the NCC aggregate, the inner cells proliferated, which resulted in an increase in the size of the aggregate (A). The images of immunofluorescence-stained SOX10 and TWIST1 show that an obvious decrease in the SOX10-expression caused by the PA1-EM cell induction was observed, and the differentiation into TWIST1-positive ectomesenchyme cells was confirmed (B). The images of immunofluorescence-stained DLX5 show that DLX5 was highly expressed in the induced ectomesenchyme cells, and these cells reproduced the genetic features of the PA1-EM cells. Similarly, PA1-EM cells were successfully induced from the iPS cell line derived from a patient with osteogenesis imperfecta (FIG. 33).

Furthermore, it was checked if NCCs used to induce PA1-EM cells need to be HOX-negative. Induction of PA1-EM cells was conducted using NCCs (HOX(+) NCCs) induced without RA treatment and NCCs (HOX(-) NCCs) subjected to the RA treatment to express the HOX gene. The outlines of the methods are shown in the upper diagram in FIG. 34. The expression levels of HOXB2 and DLX5 in each type of PA1-EM cells were measured through RT-qPCR. As a result, an increase in the expression of DLX5 was not observed in the cells obtained by inducing PA1-EM cells using the NCCs expressing the HOX gene (lower diagram in FIG. 34). Also, when NCCs obtained through adherent culture (2D) were used to induce PA1-EM cells in the presence of the same additives, it was shown that the PRRX1 expression was significantly low, and the DLX5 expression was also low (upper diagram in FIG. 35). The outline of this method is shown in the lower diagram in FIG. 35. These results showed that it is important to start from the NCCs obtained through suspension culture (3D) in order to induce PA1-EM with high efficiency. The base sequences of the primers used in RT-qPCR are shown in Table 1.

### <Verification of Culture Conditions for Induction of PA1-EM cells from NCC Aggregate>

The best combination of Fgf8, Edn, and BMP4 in the induction of PA1-EM cells was verified through RT-qPCR. When Fgf8 was not added to the culture medium, obvious cell death was induced, and therefore, Fgf8 was added in all the conditions. The outlines of the methods are shown in FIG. 36. The expression levels of DLX, PRRX1, and TWIST1 in the cells obtained using the methods are shown in FIG. 37. As shown in FIG. 37, Edn-1 was shown to be essential for the expression of DLX5, and this result reproduces the phenomenon in animal experiments, which has been reported. Also, it was shown from the expression levels of DLX5, PRRX1, and TWIST1 that a condition in which all of Fgf8, Edn-1, and BMP4 are added to a culture medium is the optimum condition for the induction of PA1-EM cells. The base sequences of the primers used in RT-qPCR are shown in Table 1.

### Example 4: Verification of Capacity of PA1-EM Cell Aggregate to Induce Patterning inside First Pharyngeal Arch

### <Induction of Proximal-Oral Cells of First Pharyngeal Arch>

It is known that PA1-EM cells respond to the epithelial signals and thus induce patterning (FIG. 39A). Therefore, it was verified whether or not PA1-EM cells obtained using the method above have a capacity to induce patterning inside the first pharyngeal arch. Proximal-oral (moral formation region) cells of the first pharyngeal arch were induced from the PA1-EM cell aggregate. The outline of the induction method is shown in the upper diagram in FIG. 38. Specifically, the method was conducted in accordance with the following procedure. PA1-EM cell aggregates obtained in Example 3 were transferred to a 96-well U-bottom ultra-low adhesion culture plate. In order to induce proximal-oral cells of the first pharyngeal arch, the PA1-EM cell aggregates were cultured for 3 days in an induction culture medium prepared by adding 100 ng/mL Fgf8b, 400 nM SAG (manufactured by Selleck Chhemicals), 1.0 µM BQ-123 (manufactured by Selleck Chhemicals), and 0.5 µM LDN193189 (manufactured by TOCRIS Bioscience) to the StemFit Basic03 culture medium. When the proximal-oral markers of the first pharyngeal arch, PAX9 and LHX8, were analyzed through RT-qPCR, the induction of proximal-oral cells of the first pharyngeal arch was observed (FIG. 39B). The base sequences of the primers used in RT-qPCR are shown in Table 1.

### <Induction of Distal-Aboral Cells of First Pharyngeal Arch>

Distal-aboral (jawbone ossification starting region) cells of the first pharyngeal arch were induced from the PA1-EM cell aggregate. The outline of the induction method is shown in the lower diagram of FIG. 38. Specifically, the PA1-EM cell aggregates obtained in Example 3 were cultured for 3 days in an induction culture medium prepared by adding 20 ng/mL Fgf2, 50 nM Endothelin-1, and 10 ng/mL BMP4 to the StemFit Basic03 culture medium. When the distal-aboral markers of the first pharyngeal arch, GSC and MSX2, were analyzed through RT-qPCR, the induction of distal-aboral cells of the first pharyngeal arch was observed (FIG. 39C).

### Example 5: Induction of Jawbone-Like Organization and Transplantation of Said Organization to Mouse

### <Induction of Jawbone-Like Organization from Jawbone Ossification Starting Region Cells>

A jawbone-like organization was induced by inducing differentiation of the PA1-EM cell aggregates obtained in Example 3 into a bone in a stepwise manner. The outline of the induction method is shown in FIG. 40. Specifically, first, osteoprogenitor cells were induced by culturing the PA1-EM cell aggregates for 3 days in an induction culture medium prepared by adding 20 ng/mL Egf, Fgf2, 50 ng/mL BMP2, 3 µM CHIR99021, and 400 nM SAG to the StemFit Basic03 culture medium. When the osteoprogenitor cell markers SP7, RUNX2, and DLX5 were analyzed through RT-qPCR, the induction of osteoprogenitor cells was observed (FIG. 41A). Also, by analyzing the early osteoblast marker ALPL and the immature osteocyte marker E11/gp38 through RT-qPCR, it was shown that differentiation into osteoblasts and osteocytes did not progress (FIG. 41B).

Next, in order to induce a jawbone-like tissue, the cell aggregates were cultured for 16 days in MSCgo Osteogenic SF, XF (manufactured by Sartorius AG), and were then cultured for 8 days in a bone-differentiation induction culture medium prepared by adding 10 nM dexamethasone (manufactured by Sigma), 10 mM β-glycerophosphate (manufactured by Sigma), and 50 µg/ml ascorbic acid (manufactured by Sigma) to the StemFit Basic03 culture medium. As a result, a further increase in the expression of the osteoprogenitor cell markers SP7, RUNX2, and DLX5 was observed (FIG. 42). Also, when the osteoblast marker ALPL and the osteocyte markers E11/gp38, DMP1, PHEX, and SOST were analyzed through RT-qPCR, the induction of differentiation into a bone was observed (FIG. 43). Histological observation through alizarin red staining (FIG. 44A) and HE staining (FIG. 44B) was conducted on the obtained jawbone-like tissue. Also, immunofluorescent staining of F-actin was conducted to check the formation of an osteocyte network inside the jawbone-like tissue (FIG. 44C).

### <Transplantation of Jawbone-Like Tissue to Jawbone Defect of Immunodeficient Mouse>

In order to examine the effect of transplantation of the obtained jawbone-like tissue to a jawbone defect to regenerate the jawbone, the effect was evaluated using a model with a jawbone defect of an immunodeficient NSG mouse. Specifically, a bone defect with a diameter of 1.6 mm was formed using a round bar in the ramus of the right angle of the jawbone of an NSG mouse. An illustration and a micro CT image of the bone defect-induced jawbone are shown in the upper diagrams in FIG. 45. Thereafter, two pieces of the jawbone-like tissue were directly transplanted to the defect without using an artificial scaffold material. Micro CT imaging was conducted over time right after the transplantation and 1 week, 2 weeks, 3 weeks, and 4 weeks after the transplantation to observe restoration of the defect (lower diagrams in FIG. 45). As a result, it was shown that a bone-like structure was formed in most of the jawbone defect 4 weeks after the transplantation.

The experiments in the following Examples were conducted in accordance with the following procedure.

### <Human iPSCs culture and maintenance>

Human induced pluripotent stem cells (iPSCs), reprogrammed with episomal vectors, were maintained under feeder-free and xeno-free conditions, as previously described (Nakagawa, M. et al. Nakagawa, M. et al. A novel efficient feeder-Free culture system for the derivation of human induced pluripotent stem cells. Sci. Rep. 4, 1-7 (2014)). iPSCs were cultured on iMatrix-511-coated (human laminin-511 E8 fragment; Nippi, Tokyo, Japan) cell culture plates in StemFit AK03N medium (Ajinomoto, Tokyo, Japan). Upon reaching approximately 80% confluency, typically every 4 to 5 days, the cells were detached and dissociated into single cells using Accutase (Innovative Cell Technologies, CA, USA). After centrifugation, the cells were resuspended in StemFit AK03N supplemented with 10 µM Y-27632 (ROCK inhibitor; FUJIFILM Wako, Tokyo, Japan) and seeded onto iMatrix-511-coated culture plates. The following day, the culture medium was replaced with fresh StemFit AK03N without Y-27632. The medium was subsequently changed every 2 days until the next passages. The study utilized the following human iPSC lines: 1231A3, HLA homozygous iPSCs (Ff-I 14s04, Ff-I 14s04 knockout of human leukocyte antigen (HLA)-A,B, and CIITA genes (HLA-KO Ff-I 14s04: clone Ff-I 14s04-AB II-KO-13), Ff-I 014s04) provided by CiRA Foundation. Osteogenesis imperfecta (OI) patient-derived iPSCs (OI-iPSCs: clone OI08-12, exhibiting a point mutation at the splicing donor site of intron 32 in COLIA1 (c.2235+1G>A), classified as Sillence Type III). Mutation-rescued OI-iPSCs (resOI-iPSCs) (Kawai, S. et al. Nat. Biomed. Eng. 3, 558-570 (2019)).

### <NCC induction from iPSCs in three-dimensional (3D) conditions>

To initiate aggregation culture, iPSCs were detached from the culture dish using Accutase and dissociated into single cells. These cells were promptly reaggregated in 100 µl of StemFit Basic03 (equivalent to AK03N without bFGF, Ajinomoto) supplemented with 10 µM Y-27632 in 96-well V-bottom ultra-low-cell-adhesion plates (Sumitomo Bakelite Co., Ltd., Tokyo, Japan). The cell concentrations applied to aggregation culture were as follows: 1231A3 (1.0×10⁴ cells/well), Ff-I 14s04, HLA-KO Ff-I 14s04, Ff-I 014s04, OI-iPSCs, and resOI-iPSCs (3.0×10³ cells/well). Cell numbers were determined using a Countess II FL (Thermo Fisher Scientific, Tokyo, Japan). Aggregation was facilitated by centrifugation at 1200 rpm for 3 min (day 0). For neural crest cell (NCC) induction, these cell aggregates were maintained in a 37°C incubator with 5% CO₂. After 24 h (day 1), the culture medium was replaced with 150 µl of StemFit Basic03 containing 10 µM SB431542 (TGF-β inhibitor; FUJIFILM Wako) and BMP4 (R&D Systems, MN, USA) (1231A3, Ff-I 14s04, HLA-KO Ff-I 14s04 (10 ng/ml), Ff-I 014s04, OI-iPSCs, resOI-iPSCs (2.5 ng/ml)). After another 24 h (day 2), the aggregates were individually collected and transferred into 48-well non-coated plates (IWAKI, Tokyo, Japan) and cultured in 250 µl of StemFit Basic03 containing 10 µM SB431542 and 1 µM CHIR99021 (GSK-3 inhibitor, Axon Medchem, VA, USA) on an orbital shaker (CS-LR; TAITEC, Saitama, Japan) at 120 rpm from day 2 to 4.

For HOX-negative NCC induction, the aggregates were cultured without medium change from day 4 to 5. For HOX-positive NCC induction, day 4 aggregates were cultured with 250 µl of StemFit Basic03 containing 10 µM SB431542, 1 µM CHIR99021, and 10 nM all-trans-Retinoic Acid (RA) (FUJIFILM Wako) from day 4 to 5.

To investigate the effect of BMP4 treatment duration on NCC induction, 1231A3 iPSCs were aggregated in a medium with 10 µM Y-27632 in 96-well V-bottom ultra-low-cell-adhesion plates (10000 cells/well). After 24 h (day 1), to assess whether BMP4 treatment influences NCC induction efficiency, the culture medium was replaced with StemFit Basic03 containing 10 µM SB431542 and 1 µM CHIR99021 with or without BMP4. After 24 h, the aggregates were transferred into 48-well plates and cultured in a medium containing 10 µM SB and 1 µM CHIR on an orbital shaker at 120 rpm from day 2 to 5. To test whether extended BMP4 treatment affects NCC induction efficiency, day 1 aggregates were cultured in a medium containing 10 µM SB and 10 ng/ml BMP4 for 2 days, then transferred into 48-well plates and cultured in a medium containing 10 µM SB and 1 µM CHIR on an orbital shaker at 120 rpm from day 3 to 5.

### <NCC induction from iPSCs in two-dimensional (2D) conditions>

In 2D conditions, NCCs were induced from 1231A3 iPSCs following previously established protocols (Non-Patent Literature 3). Briefly, iPSCs were seeded onto iMatrix-511-coated culture plates at a density of 3.6 × 10³ cells/cm² in StemFit AK03N medium and maintained in culture for 4 days. For NCC induction, the cells were cultured in StemFit Basic03 supplemented with 10 µM SB431542 and 1 µM CHIR99021 for 10 days, with medium changes every 2 days.

### <Differentiation of NCC aggregates>

NCC aggregates at day 5 were transferred into 96-well U-bottom ultra-low-cell-adhesion plates (Sumitomo Bakelite).

For peripheral neuron differentiation, NCC aggregates were cultured in Neurobasal medium (Thermo Fisher Scientific) supplemented with B27 supplement (Thermo Fisher Scientific), N-2 supplement (Thermo Fisher Scientific), 2 mM L-glutamine (FUJIFILM Wako), and 10 ng/ml each of BDNF, GDNF, NT-3, and NGF (FUJIFILM Wako) for 14 days, with medium changes every 3 days. Differentiation was confirmed by immunostaining with anti-Peripherin and anti-class III β-Tubulin antibodies.

For melanoblast differentiation, NCC aggregates were cultured in Stemfit Basic03 supplemented with 1 µM CHIR99021, 25 ng/ml BMP4, and 100 nM Endothelin-3 (TOCRIS, Bristol, UK) for 6 days, with medium changes every 3 days. Differentiation was confirmed by immunostaining with anti-MITF antibody.

For mesenchymal stem/stromal cell (MSC) differentiation, NCC aggregates were first dissociated into single cells with Accutase and seeded onto fibronectin-coated (Millipore, Darmstadt, Germany) plates at a density of 1.0 × 10⁴ cells/cm². The cells were cultured with StemFit Basic03 supplemented with 10 µM SB431542, 20 ng/ml each of EGF (FUJIFILM Wako) and FGF2 (FUJIFILM Wako). Medium was changed every 3 days, and cells were passaged before reaching subconfluency. Expanded cells (passage number 2 (PN2)) were seeded onto fibronectin-coated plates at a density of 1.0 × 10⁴ cells/cm² in StemFit Basic03 supplemented with 10 µM SB431542, 20 ng/ml each of EGF and FGF2. After 24 h, the culture medium was replaced with PRIME-XV MSC Expansion XSFM culture medium (FUJIFILM Irvine Scientific). Passages were performed every 3 or 4 days. At PN2, MSC differentiation was confirmed by FACS analysis using Human MSC markers (positive markers: CD105, CD90, CD73, CD44, CD29; negative markers: CD45, CD34, HLA-DR). To confirm the differentiation potencies of the induced MSC-like cells, osteogenic induction, chondrogenic induction, and adipogenic induction were performed, and the cells were stained with Alizarin Red, Alcian Blue, and Oil Red O, respectively, as previously described (Non-Patent Literature 3).

### <Induction of mdEM from HOX-negative NCC aggregates>

To initiate the induction of mandibular prominence ectomesenchyme (mdEM), HOX-negative NCC aggregates (day 5) were cultured in 350 µl of StemFit Basic03 supplemented with 100 ng/ml FGF8b (FUJIFILM Wako), 50 nM Endothelin-1 (PEPTIDE INSTITUTE, Osaka, Japan), and 2.5 ng/ml BMP4 on an orbital shaker at 120 rpm from day 5 to 9.

To assess the impact of EDN1 signaling on the bifurcation of maxillary prominence ectomesenchyme (mxEM) and mdEM, NCC aggregates were cultured in 350 µl of StemFit Basic03 supplemented with 100 ng/ml FGF8b and 10 µM BQ-123 (Endothelin A receptor antagonist; Selleck, Tokyo, Japan) on an orbital shaker at 120 rpm from day 5 to 12, with a medium change on day 9.

To investigate the significance of the initial anterior-posterior (A-P) identity of NCCs in mdEM differentiation, RA-treated NCCs were cultured with 350 µl of StemFit Basic03 supplemented with 100 ng/ml FGF8b, 50 nM Endothelin-1, and 2.5 ng/ml BMP4 on an orbital shaker at 120 rpm from day 5 to 9.

To evaluate the effects of FGF8, EDN1, and BMP4 on 2D-induced NCCs under 2D conditions, CD271^{high+} sorted NCCs were seeded onto fibronectin-coated plates at a density of 1.0 × 10⁴ cells/cm² and cultured with StemFit Basic03 supplemented with 100 ng/ml FGF8b, 50 nM Endothelin-1, and 2.5 ng/ml BMP4 for 4 days.

To examine the effects of FGF8, EDN1, and BMP4 on 2D NCCs in a 3D setting, CD271^{high+} sorted NCCs were rapidly aggregated in 100 µl of StemFit Basic03 supplemented with 100 ng/ml FGF8b, 50 nM Endothelin-1, and 2.5 ng/ml BMP4 at a density of 1.0×10⁴ cells/well in 96-well V-bottom ultra-low-cell-adhesion plates and cultured for 4 days.

### <Induction of mdEM derivatives>

mdEM aggregates (day 9) were transferred into 96-well U-bottom ultra-low-cell-adhesion plates (Sumitomo Bakelite).

For the induction of the distal cap domain, mdEM aggregates were cultured in 200 µl of StemFit Basic03 supplemented with 50 nM Endothelin-1, 20 ng/ml FGF2, and 25 ng/ml BMP4 from day 9 to 13, with a medium change on day 11.

For the induction of the proximal-oral domain, mdEM were cultured in 200 µl of StemFit Basic03 supplemented with 100 ng/ml FGF8b, 400 nM SAG (Smoothened Agonist, Selleck), 10 µM BQ-123, and 500 nM LDN193189 (ALK2/3 inhibitor, TOCRIS) from day 9 to 13, with a medium change on day 11. Subsequently, for dental mesenchyme induction, the proximal-oral domain induction medium was replaced with 200 µl of StemFit Basic03 supplemented with 100 ng/ml FGF8b, 400 nM SAG, 3 µM CHIR99021, and 30 ng/ml Activin A (R&D). Dental mesenchyme induction was performed from day 13 to 17, with a medium change on day 15.

### <Generation of jawbone-like organoids from mdEM>

mdEM aggregates (day 9) were transferred into 96-well U-bottom ultra-low-cell-adhesion plates. The mdEM were cultured in 200 µl of StemFit Basic03 supplemented with 20 ng/ml EGF and FGF2, 50 ng/ml BMP2 (R&D), 3 µM CHIR99021, and 400 nM SAG for 3 days. On day 12, the differentiation medium was replaced with 200 µl MSCgo Osteogenic Differentiation Medium (SARTORIUS, Göttingen, Germany) and the clusters were cultured for 7 days. On day 19, the differentiation medium was replaced with 200 µl of StemFit Basic03 supplemented with 10 mM β-glycerophosphate disodium salt hydrate (Sigma, St. Louis, MO, USA), 250 µM L-Ascorbic acid 2-phosphate sesquimagnesium salt hydrate (Sigma), 100 nM Dexamethasone (Sigma), 50 ng/ml BMP2 (R&D), 3 µM CHIR99021, and 400 nM SAG and the clusters were cultured for 7 days. From day 26 to 38, the clusters were cultured in MSCgo Osteogenic Differentiation Medium, with medium changes every 2 days.

### <Chondrogenic induction of mdEM>

To induce chondrogenic differentiation in 3D conditions, mdEM aggregates (day 9) were transferred into 96-well U-bottom ultra-low-cell-adhesion plates and cultured in MSCgo Chondrogenic XF (SARTORIUS) for 21 days. Chondrogenic differentiation was confirmed by Safranin O/Fast green staining and immunofluorescence analysis with anti-COLX antibody.

### < Induction of NCCs into Smooth Muscle Cells >

The outline of this induction method is shown in Fig. 69. Specifically, the method was conducted in accordance with the following procedure. The NCC aggregates obtained in Example 1 were dispersed into single cells using Accutase, and cells strongly positive for CD271 were sorted through flow cytometry. The sorted cells were suspended in a culture solution prepared by adding 10 µM SB431542, 20 ng/mL Egf (manufactured by FUJIFILM Wako Pure Chemical Corporation), and Fgf2 (manufactured by FUJIFILM Wako Pure Chemical Corporation) to the StemFit Basic03 culture medium, and were seeded on a fibronectin-coated culture plate at a cell concentration of 1×10⁴ cells/cm². Then, the cells were cultured for 5 days. Following this, the NCCs were again dispersed into single cells using Accutase, suspended in culture medium containing StemFit Basic03 supplemented with 2 ng/mL TGF-β1 and 50 nM Endothelin-1, and seeded back on a fibronectin-coated culture plate at a cell density of 1 × 10⁴ cells/cm² to initiate smooth muscle cell differentiation. Subsequent passage was conducted every 4 days using Accutase, and the cells were seeded on a culture plate at 1×10⁴ cells/cm² and were cultured. On day 10 of smooth muscle cell induction, smooth muscle marker expression was confirmed through immunostaining and RT-qPCR analysis.

### <Flow cytometry analysis and cell sorting>

Flow cytometry was performed using a BD FACSAria II_v1.87 (BD Biosciences, NJ, USA) following the manufacturer's instructions. Cell aggregates or 2D cultured cells were dissociated using Accutase, washed with FACS buffer (1% human serum albumin in PBS), and filtered through a 35 µm filter (Corning, NY, USA). The cells were then pelleted by centrifugation (1200 rpm, 3 min, 4°C) and resuspended in FACS buffer with appropriate antibodies, followed by a 60-minute incubation at 4°C. An isotype control was included in all experiments to eliminate nonspecific background signals. After the antibody reaction, cells were washed with FACS buffer, pelleted, and washed again. Finally, the cells were resuspended in FACS buffer and collected through a filter. Flow cytometry and sorting were performed on a BD FACSAria II. Resultant data were collected using FACSDiva_v9.0.1 and analyzed using FlowJo_v10.8.1 software (BD Biosciences). Details of the antibodies used are provided in Table 2.

**[Table 2]**

| **Antibody for flow cytometry** | | | | |
|---|---|---|---|---|
| **Antibody** | **Host** | **Company** | **Catalog number** | **Dilution ratio** |
| **CD271 Alexa647** | **Mouse** | BD | 560326 | 1:200 |
| **CD105 APC** | **Mouse** | invitrogen | 17-1057-42 | 1:200 |
| **CD90 APC** | **Mouse** | BD | 555596 | 1:200 |
| **CD73 PE** | **Mouse** | BD | 550257 | 1:200 |
| **CD44 PE** | **Mouse** | BD | 550989 | 1:200 |
| **CD29 PE** | **Mouse** | BD | 557332 | 1:200 |
| **CD45 APC** | **Mouse** | Biolegend | 340943 | 1:200 |
| **CD34 PE** | **Mouse** | BD | 560941 | 1:200 |
| **HLA-DR APC-H7** | **Mouse** | BD | 641393 | 1:100 |
| **Alexa647 Isotype** | **Mouse** | BD | 557714 | 1:200 |
| **APC Isotype** | **Mouse** | BD | 555751 | 1:200 |
| **PE Isotype** | **Mouse** | BD | 551436 | 1:200 |
| **APC-H7 Isotype** | **Mouse** | BD | 560897 | 1:100 |

### <Real-time quantitative PCR analysis>

RNA was extracted from cell aggregates or 2D cultured cells by lysing them with RLT Plus Buffer. Total RNA was purified using the RNeasy Micro Kit (QIAGEN, Venlo, Netherlands) and treated with the RNase-Free DNase Set (QIAGEN) to eliminate genomic DNA. Subsequently, 0.5 µg of total RNA was reverse transcribed into single-stranded cDNA using SuperScript III Reverse Transcriptase (Invitrogen, Massachusetts, USA) according to the manufacturer's instructions. Real-time quantitative PCR (RT-qPCR) was performed using the THUNDERBIRD Next SYBR qPCR Mix (TOYOBO, Osaka, Japan) and gene-specific primers on the QuantStudio 12K Flex Real-Time PCR System and StepOnePlus Real-Time PCR System_v2.3 (Applied Biosystems, Massachusetts, USA). The cycling parameters were as follows: initial denaturation (95°C, 1 min), followed by 45 cycles of amplification and SYBR signal detection (95°C denaturation, 3 s; 60°C annealing, 30 s; 72°C extension, 30 s), with a final step to generate a dissociation curve at the end of each qPCR run. Data were analyzed using the 2^{-ΔΔCt} method and visualized using GraphPad Prism 9_v9.5.1 software. As examples of the primer sequences used, those listed in Table 1 above and those described in Supplementary Table 2 of Non-Patent Literature 3 can be mentioned. Primer sequences other than these were taken from known sources.

### <RNA-seq analysis>

To perform RNA-seq analysis, total RNA was extracted using the RNeasy Micro Kit and treated with the RNase-Free DNase Set to eliminate genomic DNA contamination. Subsequently, 10 ng of total RNA underwent reverse transcription to generate single-stranded cDNA, employing the SuperScript VILO cDNA Synthesis Kit (Thermo Fisher Scientific). For NGS library construction targeting the Ion AmpliSeq Transcriptome, we utilized the Ion AmpliSeq Transcriptome Human Gene Expression Panel, Chef-Ready Kit (Thermo Fisher Scientific), and the Ion Chef (Thermo Fisher Scientific) instruments, following the manufacturer's protocol. Briefly, a reverse transcription master mix was prepared by combining 5X VILO Reaction Mix and 10X SuperScript III Enzyme Mix. The mix was then aliquoted into reaction plates, with 10 ng of total RNA added to each. The plates were loaded into a thermal cycler and subjected to incubation at 42°C for 30 min, followed by 85°C for 5 min, and then held at 10°C.

During library preparation on the Ion Chef instruments, the following consumables and cartridges were loaded: Ion AmpliSeq Chef Solutions DL8 cartridge, Ion AmpliSeq Chef Reagents DL8 cartridge, Ion AmpliSeq Tip Cartridge L8, PCR Frame Seal, IonCode 96 Well PCR Plate, PCR Plate Frame, Empty Tip Cartridge L8, and Enrichment Cartridge. Notably, IonCode 96 Well PCR Plates used for reverse transcription reactions contained an IonCode barcode adapter. Additionally, the tube containing the Ion AmpliSeq Transcriptome Human Gene Expression Panel was inserted into an Ion AmpliSeq Chef Reagents DL8 cartridge. Multiplex PCR was performed in the thermal cycler within the Ion Chef, with 13 cycles of 16-minute annealing/extension reactions. Subsequent steps involved primer digestion with FUPA reagents, ligation of IonCode barcode adapters, library purification using magnetic beads, equalization of library concentrations, and mixing in a single tube.

Following library preparation, the Ion Chef facilitated template preparation and ISP beads loading onto the Ion 540 Chip Kit. NGS sequencing was conducted using the Ion GeneStudio S5 Prime sequencer (Thermo Fisher Scientific). Read counts were computed utilizing the AmpliSeq RNA Plugin, and data analysis was performed using R studio_v2023.06.1+524 software with the "DESeq2" package for normalization to detect significantly (p ≤ 0.05) differentially expressed genes (Love, M. I., Huber, W. & Anders, S. Genome Biol. 15, 1-21 (2014)). Differentially expressed genes (DEGs) were identified at a false discovery rate (FDR) < 0.05 with pairwise comparison. Gene-Set Enrichment Analysis (GSEA) based on Gene Ontology (GO) terms was performed using integrated Differential Expression and Pathway analysis (iDEP) software (Ge, S. X., Son, E. W. & Yao, R. BMC Bioinformatics 19, 1-24 (2018)) (version 9.6).

### <Immunocytochemistry>

Cells cultured on dishes were fixed with 4% paraformaldehyde (PFA) at room temperature (RT) for 30 min and permeabilized with 0.3% Triton-X-100 (Sigma) in PBS at RT for 15 min. Subsequently, the cells were treated with Blocking One solution (Nacalai Tesque, Kyoto, Japan) at RT for 1 h and then incubated with primary antibodies diluted in 10% (v/v) Blocking One in PBS-T (PBS containing 0.1% Tween-20 (Sigma)) at 4°C overnight. Following this, the cells were incubated with secondary antibodies diluted in 10% Blocking One in PBS-T at RT for 2 h. Afterward, the nuclei were counterstained with 4',6-diamidino-2-phenylindole (DAPI) (1:5000; DOJINDO, Kumamoto, Japan) at RT for 15 min. Following a PBS wash, fluorescence signals were observed using a fluorescence microscope (BZ-X800; Keyence, Osaka, Japan). The primary and secondary antibodies used are listed in Table 3.

**[Table 3]**

| **Antibody for immunofluorescence** | | | | | |
|---|---|---|---|---|---|
| | **Antibody** | **Host** | **Company** | **Catalog number** | **Dilution ratio** |
| **Primary antibody** | **ALPL** | **Rat** | R&D Systems | MAB29091 | 1:200 |
| | **Calponin 1** | **Rabbit** | Abcam | ab46794 | 1:200 |
| | **CD271** | **Mouse** | ATS | AB-N07 | 1:500 |
| | **COL I** | **Rabbit** | Abcam | ab138492 | 1:500 |
| | **COL X** | **Mouse** | Invitrogen | 14-9771-82 | 1:500 |
| | **DLX2** | **Mouse** | Abnova | H00001746-M07 | 1:200 |
| | **DLX5** | **Rabbit** | Novus | NBP1-85793 | 1:200 |
| | **ECAD** | **Rabbit** | Cell Signaling TECHNOLOGY | #3195 | 1:200 |
| | **FOXF1** | **Rabbit** | R&D Systems | AF4798 | 1:200 |
| | **GATA3** | **Rabbit** | Cell Signaling TECHNOLOGY | #5852 | 1:200 |
| | **HAND1** | **Goat** | R&D Systems | AF3168 | 1:200 |
| | **HAND2** | **Goat** | R&D Systems | AF3876 | 1:200 |
| | **LHX6** | **Mouse** | Santa Cruz | sc-271433 | 1:200 |
| | **MITF** | **Rabbit** | Sigma | SAB4501879 | 1:200 |
| | **OCN** | **Mouse** | R&D Systems | MAB1419 | 1:200 |
| | **OCT3/4** | **Mouse** | Santa Cruz | sc-5279 | 1:200 |
| | **OPN** | **Goat** | R&D Systems | AF1433 | 1:200 |
| | **PAX6** | **Rabbit** | Abcam | Ab195045 | 1:200 |
| | **PAX9** | **Rabbit** | GeneTex | GTX104454 | 1:200 |
| | **PDPN** | **Rat** | Biolegend | 337001 | 1:200 |
| | **PHEX** | **Rabbit** | Bioss antibodies | bs-12313R | 1:200 |
| | **PITX1** | **Rabbit** | Novus | NBP1-88644 | 1:200 |
| | **PRPH** | **Mouse** | Santa Cruz | sc-377093 | 1:200 |
| | **SOST** | **Rabbit** | Sigma | SAB1300753 | 1:200 |
| | **SOX10** | **Goat** | R&D Systems | AF2864 | 1:200 |
| | **SP7** | **Rabbit** | Abcam | ab209484 | 1:200 |
| | **TAGLN** | **Goat** | Abcam | ab10135 | 1:200 |
| | **TFAP2A** | **Mouse** | DSHB | 3B5 | 1:200 |
| | **TFAP2B** | **Rabbit** | Cell Signaling TECHNOLOGY | #2509 | 1:200 |
| | **TUBB3** | **Rabbit** | Abcam | ab18207 | 1:200 |
| | **TWIST1** | **Rabbit** | Cell Signaling TECHNOLOGY | #31174 | 1:200 |
| | **Vimentin** | **Rabbit** | Invitrogen | MA5-16409 | 1:200 |
| | **αSMA** | **Rabbit** | Abcam | ab32575 | 1:200 |
| | **Alexa Fluor 594 Phalloidin** | | Thermo Fisher Scientific | A12381 | 1:200 |
| **Secondary antibody** | **Alexa Fluor (registered trademark) 488 Goat anti-Rabbit IgG (H+L)** | | Invitrogen | A11008 | 1:500 |
| | **Alexa Fluor (registered trademark) 555** | | Invitrogen | A21422 | 1:500 |
| | | **Goat anti-Mouse IgG(H+L)** | | | |
| | | **Alexa Fluor (registered trademark) 647 Goat anti-Rat IgG(H+L)** | Invitrogen | A21247 | 1:500 |
| | | **Alexa Fluor (registered trademark) 488 Donkey anti-Rabbit IgG(H+L)** | Invitrogen | A21206 | 1:500 |
| | | **Alexa Fluor (registered trademark) 555 Donkey anti-Mouse IgG(H+L)** | Invitrogen | A31570 | 1:500 |
| | | **Alexa Fluor (registered trademark) 555 Donkey anti-Goat IgG(H+L)** | Invitrogen | A21432 | 1:500 |
| | | **Alexa Fluor (registered trademark) 647 Donkey anti-Goat IgG(H+L)** | Invitrogen | A21447 | 1:500 |

### <Alizarin Red and von Kossa staining>

Fixed samples were embedded in paraffin and sectioned into 8-µm-thick slices. Alizarin Red staining was performed by incubating sections with 1% Alizarin Red (MUTO PURE CHEMICALS CO., LTD, Tokyo, Japan) at RT for 3 min. Von Kossa staining was conducted using the Calcium stain Kit (Modified Von Kossa; ScyTek Laboratory, Inc., Utah, USA) following the provided manual. The osteogenic potential was assessed using light microscopy.

### <Histological analysis of in vitro samples>

Cell aggregates and clusters were fixed with 4% PFA at RT for 3 h (samples from day 26 and 38 were fixed for 6 h). Samples undergoing osteogenic induction (day 19-38) were decalcified with Decalcifying Solution B (FUJIFILM Wako) at RT overnight, except in the case of Alizarin Red and von Kossa staining. For haematoxylin-eosin (HE), Azan, and Safranin O/Fast green staining, samples were dehydrated in graded ethanol, cleared with Histo-clear (National diagnostics, Georgia, USA), and embedded in paraffin. Sections of 8-µm thickness were then prepared and stained using standard protocols, followed by observation under light microscopy.

For immunofluorescence analysis, decalcified samples were embedded in Tissue-Tek O.C.T compound (Sakura Finetek, Tokyo, Japan), and 20-µm-thick sections were obtained using a cryostat (CM1950; Leica, Tokyo, Japan). These sections were washed with PBS, permeabilized with 0.3% Triton-X-100 in PBS at RT for 20 min, and then treated with Blocking One Histo solution (Nacalai Tesque) at RT for 1 h. Primary antibodies, diluted in antibody working solution (Can Get Signal Immunostain solution B, TOYOBO), were incubated with the sections at 4°C overnight. After washing, the sections were incubated with secondary antibodies diluted in antibody working solution at RT for 4 h. Following DAPI counterstaining for 15 min and PBS washing, the sections were mounted with VECTASHIELD Mounting Medium (Vector, California, USA) and subjected to confocal microscopy (FV3000; Olympus, Tokyo, Japan). Confocal imaging data were analyzed using FV31S-SW software (Olympus). The primary and secondary antibodies used are listed in Table 3.

To visualize denatured COLI, 5-FAM-conjugated Collagen Hybridizing Peptide (F-CHP) (3Helix, Inc., Utah, USA) was employed according to the manufacturer's instructions. Cryosections were treated with blocking solution 2, incubated with 15 µM F-CHP in PBS solution overnight at 4°C, and then incubated with secondary antibodies and DAPI as described above. Fluorescence signals were detected using a confocal microscope.

### <Cell viability>

To assess cell viability, cell aggregates were evaluated using a Cyto3D Live-Dead Assay Kit (TheWell BIOSCIENCE, NJ, USA) following the manufacturer's instructions. Briefly, 2 µL of Cyto3D reagent was added to each well, and the aggregates were then incubated at 37°C for 15 min. Fluorescence signals were observed using a fluorescence microscope. To identify apoptotic cells, sectioned samples were subjected to Terminal deoxynucleotidyl transferase dUTP nick end labelling (TUNEL) staining (DeadEnd Fluorometric TUNEL System; Promega, Wisconsin, USA) according to the manufacturer's instructions. Nuclei were counterstained with DAPI, and fluorescence signals were visualized using a confocal microscope. TUNEL-positive cells were quantified using ImageJ_v1.54f software (NIH, Maryland, USA).

### <Transplantation>

For jawbone-like organoid transplantation under the renal capsule, 8-week-old male NOD/Scid/IL2Rγ (NSG) mice were used as recipients. Mice were anesthetized with an intraperitoneal injection of a combination of medetomidine hydrochloride (0.3 mg/kg), midazolam (4 mg/kg), and butorphanol tartrate (5 mg/kg). Following skin shaving and disinfection at the surgical site, a jawbone-like organoid (day 38: derived from 1231A3, OI-iPSC, resOI-iPSC) was transplanted under the renal capsule of each mouse without artificial materials (n = 6/group). The incisions were closed with 5-0 monofilament sutures (Bearmedic, Ibaragi, Japan). Mice were euthanized with CO₂ four weeks post-transplantation, and transplanted tissues were collected after micro-computed tomography (micro-CT) scanning.

For jawbone-like organoid transplantation into jawbone defects, 12-week-old male NSG mice were employed as recipients. A 10-mm skin incision was made to expose the right hemimandible, followed by the creation of a 2-mm-diameter circular defect at the mandible angle using a micro engine with round steel burs (VOLVER i7, NAKANISHI, Tochigi, Japan). Three jawbone-like organoids (day 38: derived from 1231A3) were transplanted into each defect without artificial materials (n = 8/group), as illustrated in Fig. 64. Muscle and skin were sutured with 5-0 monofilament sutures. Mice were fed a soft diet for two weeks post-surgery before transitioning to a regular diet. Mandibular bones were harvested and scanned by micro-CT four weeks post-transplantation.

### <Micro-CT analysis>

Before euthanasia, renal regions and jawbones were scanned using a micro-CT system (inspeXio SMX-100CT; Shimadzu, Kyoto, Japan) with the following parameters: voxel size of 30 µm, tube voltage of 60 kV, tube current of 0.2 mA, and exposure time of 480 ms. CT slice resolution was 1024 × 1024 pixels. Bone morphometrics were analyzed using 3D imaging software (TRI/3D-BON-FCS; RATOC system engineering, Osaka, Japan) according to the manufacturer's instructions. Using a calibration curve based on known density phantoms scanned under the same conditions, the CT value of each tissue was converted to the tissue mineral density (TMD).

### <Histological analysis of in vivo samples>

Four weeks following the transplantation of jawbone-like organoids, mice were euthanized subsequent to micro-CT image scanning, and both kidneys and jawbones were collected. The specimens were fixed in 4% PFA at 4°C for 24 h, followed by a decalcification period of 7 days (except for von Kossa staining). Subsequently, the specimens underwent dehydration using graded ethanol, were cleared with Histo-clear, and were embedded in paraffin. The specimens were sectioned into 8-µm-thick serial slices, with jawbone samples cut in the frontal plane.

HE staining, Azan staining, and TRAP staining (TRAP/ALP Stain Kit, FUJIFILM Wako) were performed according to standard protocols and observed via light microscopy. Quantitative assessment of osteocytes and the Azan red-stained area was conducted utilizing ImageJ software.

For immunofluorescence analysis, deparaffinized sections were permeabilized with 0.3% Triton-X-100 in PBS at RT for 20 min, followed by treatment with LAB solution (Polyscience, Illinois, USA) for antigen activation at RT for 15 min. Subsequently, the sections were blocked with blocking solution 2 at RT for 1 h and then incubated with primary antibodies diluted in antibody working solution at 4°C overnight. For mouse antibodies against human OCN, the sections were blocked using the Mouse on Mouse (MOM) kit (ReadyProbes Mouse-on-Mouse IgG Blocking Solution (30X), Invitrogen). Afterward, the sections were incubated with secondary antibodies diluted in antibody working solution at RT for 4 h. To eliminate tissue autofluorescence, sections were treated with the Vector True VIEW Autofluorescence Quenching kit (Vector) at RT for 5 min. Following counterstaining with DAPI, the sections were mounted, and fluorescence signals were detected using a confocal microscope. Details of the primary and secondary antibodies used are provided in Table 3.

To visualize F-actin filaments, phalloidin staining was performed on cryosections. Fixed and decalcified specimens were embedded in Tissue-Tek O.C.T compound, and 20-µm-thick sections were cut. The sections were washed with PBS, permeabilized with 0.3% Triton-X-100 in PBS at RT for 20 min, blocked with blocking solution 2 at RT for 1 h, and then incubated with Alexa Fluor 594 phalloidin (Thermo Fisher Scientific) and primary antibodies diluted in antibody working solution at 4°C overnight. After incubation, the sections were exposed to Alexa Fluor 594 phalloidin and secondary antibodies diluted in antibody working solution at RT for 4 h. Nuclei were counterstained with DAPI.

### <3D analysis of osteocytes>

To visualize the 3D structure of phalloidin-stained osteocyte dendritic processes, forty optical serial z-axis sections (0.5 µm step size) were acquired using a confocal microscope and reconstructed using IMARIS_v9.7.2 software (Oxford Instruments, Oxfordshire, UK). The number of dendritic processes per osteocyte were counted in rotating the 3D image using the Filament Tracer function of IMARIS. The diameter for spots was set to 1 µm. Measurements were performed on randomly selected ten osteocytes per specimen.

### <Statistics>

Statistical analyses were performed using GraphPad Prism 9 software, and the results are presented as mean ± standard error of the mean (s.e.m.). One-way analysis of variance (ANOVA) with the Tukey-Kramer post-hoc test was employed to compare multiple groups, while the two-sided Student's t-test was used for comparisons between two groups. The number of biological replicates is indicated in the figure legends. P-values < 0.05 were considered statistically significant.

### <Study approval>

The experimental protocols involving human subjects received approval from the Ethics Committee of the Department of Medicine and Graduate School of Medicine, Kyoto University, and the Ethics Committee of Shiga Medical Center for Children. Written informed consent was obtained from all donors. All procedures involving mice were conducted in strict accordance with the Regulations on Animal Experimentation at Kyoto University (protocol number: 16-73, 22-155, 24-225).

### Example 6: Induction of HOX-negative NCCs from human iPSCs in 3D conditions

To establish a stepwise induction protocol for generating jawbone organoids from iPSCs, the inventors' initial focus was on developing a 3D culture system conducive to the targeted induction of HOX-negative NCCs (Fig. 46a). Thus, the inventors sought to adapt these induction protocols to 3D-cultured iPSCs. Initially, dissociated human 1231A3 iPSCs (a feeder-free and xeno-free iPSC line) were aggregated on 96-well V-bottom ultra-low-cell-adhesion plates in medium containing the ROCK inhibitor, Y-27632 (Figs. 46b, c), and cultured with SB/CHIR under shaking conditions (to enhance nutrient absorption) for 4 days (Fig. 47a). The induction of CD271^{high+} NCCs within these aggregates was observed, alongside undesired PAX6⁺ neuroectoderm (Figs. 47b, c). To mitigate neuroectoderm derivation, the aggregates to BMP4 ((Marchant, L., Linker, C., Ruiz, P., Guerrero, N. & Mayor, R. Dev. Biol. 198, 319-329 (1998)) treatment before SB/CHIR culture were subjected (Fig. 47d). Although excessive BMP4 signaling induced E-Cadherin (ECAD)⁺ cells on the aggregate surface (Figs. 47e, f), a 1-day BMP4 treatment effectively induced SOX10⁺CD271⁺TFAP2A⁺ NCCs (Figs. 46b-d). Flow cytometry analysis confirmed the enrichment of CD271^{high+} NCCs (94.9 ± 1.9%) (Fig. 46e), with TFAP2A⁺ECAD⁺ non-neural ectoderm, PAX6⁺ neural ectoderm, and OCT3/4⁺ undifferentiated iPSCs observed at minimal levels (Fig. 46f). The high efficiency of NCC induction was consistent across different iPSC clones, including Ff-I 14s04, HLA-KO Ff-I 14s04, Ff-I 01s04, osteogenesis imperfecta (OI) patient-derived iPSCs (OI-iPSCs; clone OI08-12) (Kawai, S. et al. Nat. Biomed. Eng. 3, 558-570 (2019)), and mutation-rescued OI-iPSCs, achieved by adjusting initial cell density and BMP4 concentration for each cell line (Fig. 48). qPCR analysis demonstrated that CD271^{high+} sorted cells from day 5 aggregates (d5 271H) expressed NCC markers (SOX10, NGFR, TFAP2A, RHOB, PAX3) at levels comparable to those from previously reported 2D protocols (Non-Patent Literature 3) (2D 271H) (Fig. 46g), with downregulation of the pluripotent cell marker POU5F1 in both cell populations. RNA sequencing (RNA-seq) analysis revealed a gradual transition in cell population from iPSCs (day 0) to NCCs (day 5), passing through the neural plate border cell state (Fig. 48a). Consistent with these findings, SOX10 and CD271 proteins were detected after 4-day induction (Fig. 48b). Furthermore, the capacity of the aggregates to differentiate into NCC derivatives was confirmed by the induction of class III β-Tubulin (TUBB3)⁺ and Peripherin (PRPH)⁺ peripheral neurons (Fig. 46h), MITF⁺ melanoblasts (Fig. 46i), and mesenchymal stem/stromal cells (MSCs) (Fig. 50).

To evaluate the A-P axial identity of the NCC aggregates, aggregates treated with retinoic acid (RA) were prepared to induce posterior fates (Mica, Y., Lee, G., Chambers, S. M., Tomishima, M. J. & Studer, L. Cell Rep. 3, 1140-1152 (2013); Fattahi, F. et al. Nature 531, 105-109 (2016)) as a positive control (Figs. 51a-c) and conducted qPCR analysis. Regular NCC aggregates exhibited minimal expression of HOX genes (HOXA1, HOXA2, HOXB2, HOXA3) (Rothstein, M., Bhattacharya, D. & Simoes-Costa, M. Dev. Biol. 444, S170-S180 (2018)) compared to RA-treated NCC aggregates (Figs. 46j, k), with RNA-seq analysis confirming these results (Fig. 51d). Additionally, regular NCC aggregates expressed midbrain and midbrain-hindbrain border markers but lacked expression of forebrain and spinal cord region markers. These findings demonstrate the efficiency of our 3D culture system in inducing HOX-negative midbrain-anterior hindbrain NCCs from human iPSCs.

### Example 7: Generation of mdEM from HOX-negative NCC aggregates

The intra-arch polarity of PA1 ectomesenchyme along the proximal-distal axis is characterized by Dlx and Hand genes (Minoux, M. & Rijli, F. M. Development vol. 137 2605-2621 (2010); Depew, M. J., Lufkin, T. & Rubenstein, J. L. R. Science (80-. ). 298, 381-385 (2002); Selleri, L. & Rijli, F. M. Nature Reviews Genetics vol. 24 610-626 (2023)) (Fig. 52a). While Dlx2 is expressed in both maxillary ectomesenchyme (mxEM) and mdEM, Dlx5 expression is exclusive to mdEM, with further restriction of Hand2 towards the distal end. To selectively induce HOX-negative NCCs into mdEM rather than other NCC derivatives, the epithelial signals in developing PA1 were investigated. Fgf8, crucial for the survival and proliferation of PA1 ectomesenchyme, is detected throughout the nascent PA1 ectoderm (Creuzet, S., Schuler, B., Couly, G. & Le Douarin, N. M. Proc. Natl. Acad. Sci. U. S. A. 101, 4843-4847 (2004); Trumpp, A., Depew, M. J., Rubenstein, J. L. R., Bishop, J. M. & Martin, G. R. Genes Dev. 13, 3136-3148 (1999)). Endothelin-1 (Edn1), exclusively secreted from the mandibular prominence, drives PA1 ectomesenchyme towards mdEM fate through the activation of Dlx5 and subsequent Hand2 (Sato, T. et al. Proc. Natl. Acad. Sci. U. S. A. 105, 18806-18811 (2008)). Additionally, Bmp4 promotes distal mandibular development (Zuniga, E., Rippen, M., Alexander, C., Schilling, T. F. & Crump, J. G. Development 138, 5147-5156 (2011); Alexander, C. et al. Development 138, 5135-5146 (2011); Tucker, A. S., Khamis, A. Al & Sharpe, P. T. Dev. Dyn. 212, 533-539 (1998)). To determine the optimal condition, the combinatorial effects of FGF8 and EDN1 were tested. The inventors observed that FGF8 was essential for downregulating the NCC marker SOX10 and upregulating the early mesenchyme markers TWIST1 and PRRX1 (Soo, K. et al. Dev. Biol. 247, 251-270 (2002); Doufexi, A. El & Mina, M. Dev. Dyn. 237, 3115-3127 (2008)) (Fig. 53). The combination of FGF8 with EDN1 induced common PA1 ectomesenchyme markers (DLX1, DLX2) and mdEM markers (DLX5, DLX3, GSC), with additional BMP4 further upregulating these genes and activating the distal marker HAND2 (Fig. 52b). RNA-seq analysis confirmed these results and revealed minimal induction of melanocyte, sensory neuron, autonomic neuron, and glia cell markers (Fig. 54). Low expression of HOX genes indicated the absence of induction in other arches. Therefore, the triple activation of these pathways, referred to as FEDB, was employed for further experiments (Fig. 52c).

Immunostaining demonstrated that the FEDB condition effectively drove SOX10⁺ NCCs into TWIST1⁺ ectomesenchyme with a mesenchymal migratory morphology characterized by actin stress fiber formation (mesenchymal migratory morphology) (Lamouille, S., Xu, J. & Derynck, R. Nat. Rev. Mol. Cell Biol. 15, 178-196 (2014)) (Figs. 52d, e). Notably, the aggregates contained DLX2 single-positive cells in the central zone, DLX2⁺DLX5⁺ cells in the intermediate zone, and DLX2⁺DLX5⁺HAND2⁺ cells in the surface zone, suggesting that FEDB generated mdEM recapitulating the proximal-distal patterning of embryonic mdEM (Figs. 52f, g).

To further elucidate the effect of the EDN1 signal on the bifurcation of mxEM and mdEM, NCC aggregates were treated with a combination of FGF8 with the Endothelin Receptor Type A (EDNRA) (Minoux, M. & Rijli, F. M. Development vol. 137 2605-2621 (2010)) antagonist, BQ-123 (referred to as FQ) (Fig. 52h). Immunostaining revealed differentiation into ectomesenchyme, albeit relatively less than with FEDB (Fig. 52i). EDN1 inhibition notably increased the expression of maxillary ectomesenchyme markers (POU3F3, CYP26A1) (Jeong, J. et al. Development 135, 2905-2916 (2008)), but not mdEM markers (Figs. 52j, k). These findings suggest that Edn-1 signaling is critical for maxillary-mandibular selection.

To examine the importance of initial A-P identity in mdEM differentiation, RA-treated posteriorized NCCs were cultured with FEDB (Fig. 521). RA-treated NCCs differentiated into ectomesenchyme, but exhibited minimal expression of DLX5 and significantly increased HOX genes (HOXB2, HOXB4) (Figs. 52m, n), confirming that initial positional information in NCCs affects the patterning pathways.

### Example 8: Recapitulation of later stage regional patterning using mdEM

To ascertain the induction of developmentally accurate mdEM, the inventors investigated whether mdEM exhibited detailed regional patterning characteristic of later stages of development. In the later stage, epithelial domains undergo further subdivision along proximal, distal, aboral, and oral axes, characterized by the expression of Fgf8, Bmp4, Edn-1, and Shh, respectively (Xu, J. et al. Elife 8, 1-26 (2019); Vincentz, J. W. et al. Proc. Natl. Acad. Sci. U. S. A. 113, 7563-7568 (2016); Neubuser, A., Peters, H., Balling, R. & Martin, G. R. Cell 90, 247-255 (1997)) (Fig. 55a). Particularly, Bmp4 signaling directs mdEM towards the distal cap domain (Vincentz, J. W. et al. Proc. Natl. Acad. Sci. U. S. A. 113, 7563-7568 (2016); Barron, F. et al. Development 138, 2249-2259 (2011) (Figs. 55a, b). Conversely, Shh signaling specifies the oral domain, which is responsible for tooth development (Cobourne, M. T. & Sharpe, P. T. Arch. Oral Biol. 48, 1-14 (2003)). Moreover, the antagonistic interactions between Fgf8 and Bmp4 along the proximal-distal axis are involved in the specification of molars and incisors, respectively (Neubuser, A., Peters, H., Balling, R. & Martin, G. R. Cell 90, 247-255 (1997)). Therefore, the inventors aimed to segregate mdEM into two distinct domains: the distal cap and the proximal-oral molar domain.

The mdEM aggregates were transferred to a 96-well U-bottom ultra-low-cell-adhesion plates. For distal cap induction, mdEM aggregates were cultured with various induction media containing BMP4 (Fig. 56a). Notably, a pronounced upregulation of HAND1, the principal distal cap marker, alongside other distal markers (HAND2, GATA3, DKK1) in response to elevated BMP4 concentrations, was observed (25 ng/ml). These genes were further activated with decreased levels of the proximal signal, FGF8, albeit with an increase in apoptotic cells (Fig. 54b). To enhance cell viability, FGF8 was replaced with FGF2, leading to the adoption of a condition termed BMP4^{high} EDN1, FGF2^{low} for distal cap induction (Fig. 55c). Immunostaining revealed decreased expression of DLX5 (Fig. 54d) and clear detection of HAND1 in the outer zone (Fig. 54e), akin to the mouse distal cap (Vincentz, J. W. et al. Proc. Natl. Acad. Sci. U. S. A. 113, 7563-7568 (2016)). Expression of HAND2 and GATA3 was also distinctly observed.

For proximal-oral domain induction, mdEM aggregates were cultured with various induction media containing FGF8 and the Hedgehog signaling agonist, SAG (Fig. 56c). This combination effectively induced proximal-oral-related genes (FOXF1, GLI1, PITX1, GBX2, BARX1, PAX9, LHX6). Furthermore, these genes were upregulated when opposite regional signals, EDN1 and BMP4, were inhibited by BQ-123 and LDN193189 (LDN). Hence, the condition FGF8, SAG, BQ-123, LDN was selected for proximal-oral domain induction (Fig. 55c). Immunostaining revealed maintained expression of DLX5 and clear detection of FOXF1 and PITX1. Importantly, these marker expression patterns were mutually exclusive between distal cap and molar domain induction (Fig. 55d-f). qPCR and RNA-seq analyses confirmed these findings (Fig. 55g, Fig. 57a).

Lastly, the odontogenic differentiation capacity of proximal-oral domain mdEM. Dental mesenchyme differentiation requires signaling from dental epithelium was assessed (Neubuser, A., Peters, H., Balling, R. & Martin, G. R. Cell 90, 247-255 (1997); Prochazka, J. et al. Dev. Cell 35, 713-724 (2015); Yu, T. & Klein, O. D. Dev. 147, (2020); Zhang, Y. D., Chen, Z., Song, Y. Q., Liu, C. & Chen, Y. P. Cell Res. 15, 301-316 (2005)), including Shh, Fgf8, Wnt, and Activin. Treatment of proximal-oral domain mdEM with FGF8, SHH, CHIR, and ACTIVIN-A (referred to as FSCA) resulted in the expression of dental mesenchyme markers represented by TFAP2B, TFAP2A, LHX6, and PAX9 (Jing, J. et al. Nat. Commun. 13, (2022)) (Figs. 57b, 58a). Gene ontology (GO) analysis revealed a significant enrichment of genes related to "Odontogenesis" and "Odontogenesis of dentin-containing tooth" in the induction from NCCs (Fig. 58b). In summary, mdEM successfully exhibited regional patterning similar to the embryonic mandible, demonstrating developmentally faithful mdEM induction.

### Example 9: Generation of jawbone-like organoids from mdEM

Unlike mesoderm-derived bones, which primarily form through endochondral ossification, mandibular bone predominantly undergoes an intramembranous process (Salhotra, A., Shah, H. N., Levi, B. & Longaker, M. T. Nat. Rev. Mol. Cell Biol. 21, 696-711 (2020); Parada, C. & Chai, Y. Curr. Top. Dev. Biol. 115, 31-58 (2015)). In this process, mdEM condenses and directly differentiates into osteoblasts (Salhotra, A., Shah, H. N., Levi, B. & Longaker, M. T. Nat. Rev. Mol. Cell Biol. 21, 696-711 (2020)). To mimic this process, induced mdEM aggregates with condensed cell structures were cultured directly in xeno-free osteogenic induction medium, optimized for 3D culture (Fig. 59a). By day 38, white clusters with diameters of 1.0-1.5 mm were obtained (Fig. 59b). There was a significant increase in ALP activity and calcium content (Fig. 59c). Additionally, mineralized tissue formation was evidenced by Alizarin Red and von Kossa staining (Fig. 59d). qPCR analysis indicated peak expression of osteoblast markers at day 26, with continued increase in bone matrix protein and osteocyte markers until day 38 (Fig. 60a). Notably, the mature osteocyte marker, SOST, showed clear upregulation after day 19 (Fig. 59e), which was corroborated by RNA-seq analysis (Fig. 60b). Conversely, chondrogenic markers were not significantly upregulated (Fig. 60c), and HOX gene expression remained marginal (Fig. 60d), confirming maintenance of positional information in derivatives.

Histological analysis of decalcified specimens revealed bone-like tissues with eosinophilic affinity and dense matrices stained with Azan Red (Fig. 59f). Moreover, major bone matrix protein, COLI, was abundantly expressed in the matrices, and non-collagenous bone proteins, OCN and OPN, were also detected (Fig. 59g, Fig. 61a). Safranin O staining and immunostaining against COLX demonstrated that a little chondrogenic differentiation occurred (Fig. 61b).

Subsequently, cellular characteristics in the bone-like tissues were examined. SP7⁺ osteogenic lineages appeared at day 12 and increased during differentiation (Fig. 61c). The cell morphology was gradually changed from fibroblastic to dendritic and PDPN⁺PHEX⁺ early osteocytes were detected around the surface area after 26 days of induction (Fig. 61d). By day 38, ALP⁺SP7⁺ osteoblasts with cuboidal shapes lined the surface layer of the tissues (Fig. 59h), and a notable number of cells differentiated into PDPN⁺PHEX⁺ early osteocytes with dendritic processes extending from the surface to the inner area (Figs. 59i, j). Notably, within the bone matrices, SOST⁺ mature osteocytes formed 3D dendritic networks (Fig. 59k). In summary, our approach generated jawbone-like organoids from mdEM aggregates, recapitulating 3D network-forming osteocytes embedded in mineralized bone matrices (Fig. 591). The generation of jawbone-like organoids via mdEM were reproduced using other iPSC clones (Fig. 62).

In vivo, mandibular bone mineralization and maturation progress following vascularisation (Vesela, B., Svandova, E., Bobek, J., Lesot, H. & Matalova, E. Front. Physiol. 10, 1-8 (2019)). The inventors hypothesized that our jawbone-like organoids represent an early stage of jawbone development before vascularisation. To assess the potential of these organoids to give rise to more mature bone tissues, an organoid under the capillary-rich renal capsule of NOD/Scid/IL2Rγ (NSG) mice was transplanted. Four weeks post-transplantation, white tissue was observed under the renal capsule (Fig. 59m). Micro-computed tomographic (micro-CT) analysis revealed highly mineralized tissue (Fig. 59n). Histological analysis confirmed the formation of vasculature and mature bone tissues (Fig. 59o, Fig. 63a). Cartilage formations were hardly observed. In addition, the tissues consisted of human bone matrices and human Vimentin-positive donor cells with dense dendritic processes (Fig. 59p, Fig. 63b). These findings demonstrate that jawbone-like organoids have the capacity to develop into mature human bone tissues in vivo.

### Example 10: Transplantation of jawbone-like organoids induces bone regeneration in an immunodeficient mouse jawbone defect model

To evaluate the regenerative potential of jawbone-like organoids, three clusters of these organoids were directly transplanted into mandibular jawbone defects measuring 2.0 mm in diameter in NSG mice (Figs. 64a, b). Four weeks post-transplantation, micro-CT analysis revealed a significant increase in the amount of mineralized tissue in the transplanted group compared to the group without grafts (Figs. 64c, d, Fig. 65). Histological examination using HE and Azan staining demonstrated that while some new bone formation was observed in the group without grafts, bone tissues in the transplanted group closely resembled the mature structure of the host's original bone (Fig. 64e). Immunostaining with antibodies specific to human COLI, OCN, and OPN confirmed the presence of bone matrix proteins secreted by donor human cells derived from the jawbone-like organoids, further validating their contribution to bone regeneration (Fig. 64f). Moreover, within the bone matrices, both Vimentin-positive donor human osteocytes and -negative host mouse osteocytes formed dendritic cellular networks (Figs. 64g, h), and tartrate-resistant acid phosphatase (TRAP)-positive osteoclasts were observed along the newly formed bone, indicating active bone remodelling (Fig. 64i). These findings collectively suggest that transplanted jawbone-like organoids promote bone regeneration through successful osteogenesis, involving both direct contribution from donor cells and indirect stimulation of host osteogenesis.

### Example 11: In vitro disease modelling using OI-iPSC-derived jawbone-like organoids

OI is a heritable bone disease characterized by bone matrix fragility, typically resulting from abnormal synthesis and processing of COLI (Van Dijk, F. S. & Sillence, D. O. Am. J. Med. Genet. Part A 164, 1470-1481 (2014)). Currently, OI diagnosis primarily focuses on mesoderm-derived long bones, with minimal attention given to the jawbone. Furthermore, although the severity of bone fragility likely involves osteocyte-to-bone matrix interactions, understanding the role of osteocytes in OI pathogenesis has been limited (Pathak, J. L., Bravenboer, N. & Klein-Nulend, J. Front. Endocrinol. (Lausanne). 11, 1-14 (2020)). Therefore, the utility of jawbone-like organoids for histological analysis of OI was investigated.

First, mdEM was induced from OI-iPSCs obtained from a patient with autosomal dominant mutations of COLIA1, along with resOI-iPSCs (Kawai, S. et al. Nat. Biomed. Eng. 3, 558-570 (2019)) (Fig. 66a). After 3 days of osteogenic induction, initial COLI production was observed in both OI- and resOI-iPSC-derived aggregates (Fig. 67a). Notably, collagen secreted from OI-iPSC-derived aggregates stained positive for collagen hybridizing peptide (CHP), indicating the production of misfolded triple-helical chains. By day 38, Azan blue-stained immature bone matrices were evident in OI-iPSC-derived jawbone-like organoids (OI-organoids) (Fig. 67b), with disrupted formation of osteocyte dendritic processes (Fig. 67c). These abnormal features were rectified in resOI-iPSC-derived organoids (Figs. 67a-c).

Subsequently, OI- and resOI-organoids were transplanted under the renal capsule of NSG mice. Micro-CT analysis after four weeks revealed that OI-organoids generated less and lower mineralized tissues compared to resOI (Figs. 67d, f). Histological analysis demonstrated that OI-organoids formed Azan blue-stained immature bone tissue, with a large number of osteocytes exhibiting characteristics typical of OI bone features (Mahr, M. et al. Int. J. Mol. Sci. 22, (2021)) (Figs. 67e, g, Figs. 66b). These osteocytes were partially apoptotic (Figs. 67h, j). Conversely, resOI-organoids exhibited recovery from these abnormal features. Furthermore, osteocyte 3D network formations were disoriented in OI, with significantly lower dendrite number compared to resOI (Figs. 67i, k), suggesting aberrant osteocyte-to-bone matrix interactions. These findings indicate that the combination of jawbone-like organoids and OI-iPSCs successfully recapitulated the phenotypes of OI.

### Example 12: Induction of smooth muscle cells from NCC clusters

The inventors hypothesized that scaffold stiffness could influence the direction of NCC differentiation. Indeed, mechanical forces have been identified as crucial for NCC migration and differentiation (Canales Coutino, B. & Mayor, R. Dev. Cell 57, 1792-1801 (2022); Zhu, Y. et al. J. Cell. Physiol. 234, 7569-7578 (2019)). Additionally, in mouse embryos, shaping the mandibular prominence requires 3D mesenchymal intercalations guided by actomyosin polarity (Tao, H. et al. Nat. Commun. 10, 1-18 (2019)). Interestingly, NCCs treated with FEDB in 2D culture expressed smooth muscle cell markers without upregulation of mdEM markers (Figs. 68a-d). Moreover, inhibition of actin-stress fiber formation by Y-27632 treatment substantially rescued the expression of EDNRA and mdEM markers in 2D cultured cells, albeit not to the extent observed in 3D culture (Fig. 68e). Conversely, spheroid culture of NCCs under FEDB induction led to significant cell apoptosis (Fig. 68f). These preliminary findings suggest that our aggregate culture may provide NCCs with appropriate stiffness for mdEM differentiation.

Next, differentiation induction from NCC aggregates to smooth muscle cells was carried out. The induced cell aggregates showed high expression of Calponin, TAGLN, ACTA2, CNN1, and TAGLN, which are smooth muscle markers, and also showed high expression of MYH11, which is a mature smooth muscle marker. Therefore, it was shown that the NCC aggregates of the present invention have a capacity to differentiate into smooth muscle cells.

### Industrial Applicability

With the present invention, since neural crest cells can be produced in a short time with high efficiency, cell sorting becomes unessential, which contributes to a reduction in the development expense and thus a reduction in the cost for clinical applications. The neural crest cells produced through the present invention are very useful as starting cells for producing differentiated cells such as mesenchymal stem cells and melanocytes, and organoids such as bone organoids. The differentiated cells and organoids obtained through the present invention can be used to regenerate damaged tissues, and are useful particularly for regenerative medicine. Furthermore, it is possible to produce first pharyngeal arch ectomesenchyme cells, which have not been previously reported, from the neural crest cells obtained through the present invention, which contributes significantly to particularly regenerative medicine in the field of oral surgery.

The present application claims the benefit of priority based on Japanese Patent Application No. 2023-082964 filed in Japan (filing date: May 19, 2023), which is incorporated herein by reference in its entirety.

## Claims

1. A method for producing a neural crest cell from a pluripotent stem cell, comprising:
(1) a step of suspension-culturing the pluripotent stem cell in a culture medium containing an ALK inhibitor and a bone morphogenetic protein; and
(2) a step of suspension-culturing the cell in a culture medium containing the ALK inhibitor and a GSK-3β inhibitor.

2. The method according to claim 1, wherein step (2) comprises a step of conducting shaking culture and/or stirred culture.

3. The method according to claim 1 or 2, wherein a culture period in step (1) is 6 hours to 5 days.

4. The method according to any one of claims 1 to 3, wherein a culture period in step (2) is 1 day to 6 days.

5. The method according to any one of claims 1 to 4, comprising a step of suspension-culturing the pluripotent stem cell in a culture medium containing a ROCK inhibitor prior to step (1).

6. The method according to any one of claims 1 to 5, wherein at least one of the bone morphogenetic proteins used in step (1) is selected from the group consisting of BMP4, BMP2, and BMP7.

7. The method according to any one of claims 1 to 6, wherein at least one of steps (1) and (2) is a step of culturing the cell under xeno-free conditions and/or feeder-free conditions.

8. The method according to any one of claims 1 to 7, wherein the pluripotent stem cell is an induced pluripotent stem cell.

9. A neural crest cell obtainable by the method according to any one of claims 1 to 8.

10. A neural crest cell having all of features (A) to (C) below:
(A) derived from a pluripotent stem cell,
(B) expressing one or more genes selected from CD271, SOX10, TFAP2A, and PAX3, and
(C) having a capacity to differentiate into a first pharyngeal arch ectomesenchyme cell.

11. The neural crest cell according to claim 10, having at least one of features (D) to (F) below:
(D) having a capacity to differentiate into a melanoblast,
(E) having a capacity to differentiate into a nerve cell, and
(F) having a capacity to differentiate into a mesenchymal stem cell.

12. A method for producing a differentiated cell or an organoid, comprising a step of inducing differentiation of the neural crest cell according to any one of claims 9 to 11.

13. The method according to claim 12, wherein the differentiated cell is selected from the group consisting of a mesenchymal cell, a nerve cell, and a melanoblast.

14. The method according to claim 13, wherein the mesenchymal cell is selected from the group consisting of a mesenchymal stem cell, a first pharyngeal arch ectomesenchyme cell, an osteoprogenitor cell, an osteoblast, an osteocyte, a chondrocyte, a myoblast, an odontogenic mesenchymal cell, and a smooth muscle cell.

15. The method according to claim 12, wherein the organoid is a bone organoid.

16. The method according to claim 15, wherein the bone organoid is a jawbone organoid.

17. The method according to claim 12, wherein the step of inducing differentiation is a step of culturing the neural crest cell in a culture medium containing endothelin and a fibroblast growth factor, and the differentiated cell obtainable through this culture is a first pharyngeal arch ectomesenchyme cell.

18. The method according to claim 17, wherein the culture medium further contains a bone morphogenetic protein.

19. A differentiated cell or an organoid obtainable by the method according to any one of claims 12 to 18.

20. A first pharyngeal arch ectomesenchyme cell having all of features (a) to (c) below:
(a) derived from a neural crest cell that does not express HOX,
(b) expressing DLX5, PRRX1 and TWIST1, and
(c) responding to the epithelial signal and enabling induction of patterning.

21. An agent for transplantation therapy comprising the differentiated cell or the organoid according to claim 19 or 20.

22. A method for screening a therapeutic or preventive medicament for a tissue damage or a disease, comprising a step of culturing the differentiated cell or the organoid according to claim 19 or 20 in the presence or absence of a test substance.
